# EUROPEAN PATENT SPECIFICATION

(11) **EP 3 939 662 B1**
(45) Date of publication and mention of the grant of the patent: **11.11.2026**
(21) Application number: 20769948.9
(22) Date of filing: 13.03.2020
(51) Int. Cl.: A61P 27/02, C12N 5/0793, C12Q 1/06, C12Q 1/6851, A61K 35/30, A61L 27/38, A61L 27/40, C12Q 1/6881

(54) **METHOD FOR EVALUATING QUALITY OF TRANSPLANT NEURAL RETINA, AND TRANSPLANT NEURAL RETINA SHEET**
VERFAHREN ZUR BEWERTUNG DER QUALITÄT EINES NEURONALEN RETINATRANSPLANTATS UND NEURONALE RETINATRANSPLANTATSCHICHT
PROCÉDÉ D'ÉVALUATION DE LA QUALITÉ D'UNE RÉTINE NEURALE DE TRANSPLANTATION, ET FEUILLE DE RÉTINE NEURALE DE TRANSPLANTATION

(30) Priority: 13.03.2019 JP 2019046505
(43) Date of publication of application: 19.01.2022
(73) Proprietor: RACTHERA Co., Ltd., Tokyo 103-6012 (JP); RIKEN, Wako-shi Saitama 351-0198 (JP)
(72) Inventor: KUWAHARA Atsushi, Kobe-shi, Hyogo 650-0047 (JP); WATARI Kenji, Kobe-shi, Hyogo 650-0047 (JP); MATSUSHITA Keizo, Osaka-shi, Osaka 554-0022 (JP); YAMASAKI Suguru, Kobe-shi, Hyogo 650-0047 (JP); MANDAI Michiko, Wako-shi, Saitama 351-0198 (JP); TAKAHASHI Masayo, Wako-shi, Saitama 351-0198 (JP)
(74) Representative: Hoffmann Eitle
(86) International application number: PCT/JP2020/011254
(87) International publication number: WO 2020/184720

(56) References cited:
- EP-A1- 2 989 200
- EP-A1- 3 081 637
- EP-A1- 3 447 129
- EP-A2- 2 486 126
- EP-B1- 2 486 126
- EP-B1- 2 496 688
- EP-B1- 2 989 200
- WO-A1-2013/183774
- WO-A1-2017/176810
- WO-A1-2019/017491
- JP-A- 2016 189 789
- JUTHAPORN ASSAWACHANANONT ET AL: "Transplantation of Embryonic and Induced Pluripotent Stem Cell-Derived 3D Retinal Sheets into Retinal Degenerative Mice", STEM CELL REPORTS, vol. 2, no. 5, 1 May 2014 (2014-05-01), United States, pages 662 - 674, XP055617220, ISSN: 2213-6711, DOI: 10.1016/j.stemcr.2014.03.011
- ASSAWACHANANONT JUTHAPORN ET AL: "Supplemental Information Transplantation of Embryonic and Induced Pluripotent Stem Cell-Derived 3D Retinal Sheets into Retinal Degenerative Mice", 1 May 2014 (2014-05-01), XP093019793, Retrieved from the Internet <URL:https://ars.els-cdn.com/content/image/1-s2.0-S2213671114000903-mmc3.pdf> [retrieved on 20230201]
- KUWAHARA, A. ET AL.: "Generation of a ciliary margin-like stem cell niche from self-organizing human retinal tissue", NAT. COMMUN., vol. 6, no. 6286, 2015, pages 1 - 15, XP055371363, DOI: 10.1038/ncomms7286
- SHIRAI, H. ET AL.: "Transplantation of human embryonic stem cell -derived retinal tissue in two primate models of retinal degeneration", PROC. NATL. ACAD. SCI., vol. 113, no. 1, 2015, U. S. A., pages E81 - E90, XP055396646, DOI: 10.1073/pnas.1512590113
- NAKANO, T. ET AL.: "Self-formation of optic cups and storable stratified neural retina from human ESCs", CELL STEM CELL, vol. 10, no. 6, 2012, pages 771 - 785, XP028492663, DOI: 10.1016/j.stem.2012.05.009
- ASSAWACHANANONT, J. ET AL.: "Transplantation of embryonic and induced pluripotent stem cell-derived 3D retinal sheets into retinal degenerative mice", STEM CELL REPORTS, vol. 2, no. 5, 2014, pages 662 - 674, XP055617220, DOI: 10.1016/j.stemcr.2014.03.011
- IRAHA, S. ET AL.: "Establishment of Immunodeficient Retinal Degeneration Model Mice and Functional Maturation of Human ESC-Derived Retinal Sheets after Transplantation", STEM CELL REPORTS, vol. 10, no. 3, 2018, pages 1059 - 1074, XP055721922, DOI: 10.1016/j.stemcr.2018.01.032
- KUWAHARA, ATSUSHI, YAMASAKI SUGURU, MANDAI MICHIKO, WATARI KENJI, MATSUSHITA KEIZO, FUJIWARA MASAYO, HORI YORIKO, HIRAMINE YASUSHI: "Preconditioning the Initial State of Feeder-free Human Pluripotent Stem Cells Promotes Self-formation of Three-dimensional Retinal Tissue", SCI. REP ., vol. 9, no. 1, 12 December 2019 (2019-12-12), pages 1 - 16, XP055738603, DOI: 10.1038/s41598-019-55130-w

## Description

### Technical Field

The present invention relates to a method for evaluating the quality of a transplant neural retina and a transplant neural retina sheet and, particularly, relates to a method for evaluating the quality of a transplant neural retina derived from a pluripotent stem cell and a transplant neural retina sheet derived from a pluripotent stem cell.

### Background Art

In neural tissues *in vivo,* a single or a plurality of types of neural cells form a layer structure. One of the neural tissues, retinal tissue, is mainly constituted of 5 types of neuronal cells including photoreceptor cells, bipolar cells, horizontal cells, amacrine cells and ganglion cells, and glial cells, and forms a three-dimensional layer structure. For treating a neurological disease, for example, a retinal degenerative disease, it has been suggested that a transplantation therapy using neural tissue is effective. However, it was difficult to obtain a tissue maintaining a layer structure and a function thereof reflecting the neural tissue *in vivo* of a human. Because of this, transplantation therapy was rarely used as a common therapy. Recently, the production of neural tissue (e.g., retinal tissue) has been made possible by differentiation from pluripotent stem cells (Non Patent Literatures 1, 2, 3 and 4).

Retinal tissue derived from pluripotent stem cells contains a variety of retinal layer-specific neuronal cells and, besides, is constituted by assuming a layer structure, and therefore has a very complicated structure. For using the retinal tissue having such a very complicated structure as a transplant cell medicine, it is particularly required to strictly control its quality. As a method for evaluating the quality of a neural retina, there exists an approach such as an image analysis method of analyzing the presence or absence of a continuous epithelium structure in a cell aggregate (Patent Literature 1-3).

### Citation List

### Patent Literature

Patent Literature 1: WO2017/090741
Patent Literature 2: EP 3 447 129 A1
Patent Literature 3: WO 2017/176810 A1

### Non Patent Literature

Non Patent Literature 1: Eiraku M. et al., "Self-organized Formation of Polarized Cortical Tissues From ESCs and Its Active Manipulation by Extrinsic Signals", Cell Stem Cell, 3 (5), 519-32 (2008)
Non Patent Literature 2: Eiraku M. et al., "Self-organizing optic-cup morphogenesis in three-dimensional culture", Nature, 472, 51-56 (2011)
Non Patent Literature 3: Nakano T. et al., "Self-formation of Optic Cups and Storable Stratified Neural Retina From Human ESCs" Cell Stem Cell, 10 (6), 771-775 (2012)
Non Patent Literature 4: Kawahara A. et al., "Generation of a ciliary margin-like stem cell niche from self-organizing human retinal tissue" Nature Communications, 6, 6286 (2015)

### Summary of Invention

### Technical Problem

Accordingly, in light of the circumstances, an object of the present invention is to provide a method for evaluating the quality of a transplant neural retina and a transplant neural retina sheet selected by the method.

### Solution to Problem

The present inventors have conducted diligent studies and consequently found the possibility that in the production process of cell aggregates containing a neural retina derived from pluripotent stem cells, cells other than retinal layer-specific neuronal cells (non-target cells; non-neural retina-related cells) are produced as by-products, in addition to the retinal layer-specific neuronal cells (target cells; neural retina-related cells), in some cell aggregates. Furthermore, as a result of comprehensively analyzing gene expression, etc. as to a plurality of samples, it has been revealed that the non-target cells that might be produced as by-products are brain and spinal cord tissue and eyeball-related tissue. Moreover, as a result of analyzing the brain and spinal cord tissue and the eyeball-related tissue in detail, it has been revealed that: the telencephalon (cerebrum), the diencephalon (including the hypothalamus), the midbrain, and the spinal cord may be produced as by-products as the brain and spinal cord tissue; and retinal pigment epithelium (RPE), ciliary body, lens and optic stalk (optic stalk and optic nerve tissue) may be produced as by-products as the eyeball-related tissue.

From these novel findings, the present inventors have found that: whether to be a neural retina suitable for transplantation can be evaluated by analyzing the expression of genes related to target cells and the expression of genes related to non-target cells; and a transplant neural retina can thereby be selected, reaching the completion of the present invention.

Specifically, the present invention is defined in the appended claims.

### Advantageous Effects of Invention

According to the present invention, it becomes possible to provide a method for evaluating the quality of a transplant neural retina and a transplant neural retina sheet selected by the method, and a method for producing the transplant neural retina sheet.

### Brief Description of Drawings

[Figure 1] Figure 1 is fluorescence microscope images showing results of performing immunostaining on cell aggregates containing a transplant neural retina with Crx and Chx10 in Example 1.
[Figure 2] Figure 2 is fluorescence microscope images showing results of performing immunostaining on cell aggregates containing a transplant neural retina with Rx and Recoverin in Example 1.
[Figure 3] Figure 3 shows microarray analysis results of RNA extracted from a neural retina and by-products A, B, C, D, E and F in Example 2.
[Figure 4] Figure 4 is a conceptual view of preparing a Cap and a Ring from a typical cell aggregate.
[Figure 5] Figure 5 is a conceptual view of preparing a Cap and a Ring from cell aggregates having various shapes. Portions indicated in black color and gray color mean non-target tissue.
[Figure 6] Figure 6 shows images of typical grafts and a schematic view of a graft as well as the heights, major axes and minor axes of grafts in Example 4.
[Figure 7] Figure 7 is confocal fluorescence microscope images showing results of performing immunostaining on grafts with Crx and Chx10 in Example 5.
[Figure 8] Figure 8 shows results of analyzing gene expression for RNA extracted from a Cap and a Ring by quantitative PCR in Example 6.
[Figure 9] Figure 9 shows results of analyzing gene expression for RNA extracted from a Cap and a Ring by quantitative PCR in Example 7.
[Figure 10] Figure 10 is images showing results of analyzing RNA extracted from a Ring by quantitative PCR, then subretinally transplanting a graft (cap) to a rat, and observing an image of post-transplant engraftment under a fluorescence microscope in Example 8.
[Figure 11] Figure 11 is images showing results of analyzing RNA extracted from a Ring by quantitative PCR, then subretinally transplanting a graft (cap) to a rat, and observing an image of post-transplant engraftment under a fluorescence microscope in Example 9.
[Figure 12] Figure 12 is images in which a Ring was observed under an inverted microscope, and images showing results of subretinally transplanting a Ring to a rat and observing an image of post-transplant engraftment under a fluorescence microscope in Example 10.
[Figure 13] Figure 13 is fluorescence microscope images showing results of performing immunostaining on a Cap and a Ring prepared from one cell aggregate in Example 11.
[Figure 14] Figure 14 shows results of analyzing gene expression for RNA extracted from a Cap and a Ring prepared from a neural retina and non-neural retinas (telencephalon tissue, spinal cord tissue, RPE, and optic stalk) by quantitative PCR in Example 12.
[Figure 15] Figure 15 shows immunostained images in which a stained section was observed using a fluorescence microscope (manufactured by Keyence Corp.) in Example 14.

### Description of Embodiments

### [Definition]

The "stem cells" refer to undifferentiated cells having differentiation potency and proliferation potency (particularly, self-renewal ability). In the stem cells, subgroups of pluripotent stem cells, multipotent stem cells and unipotent stem cells, are included according to the differentiation potency. The pluripotent stem cells refer to stem cells that can be cultured *in vitro* and has an ability (pluripotency) to be able to differentiate into three germ layers (ectoderm, mesoderm, endoderm) and/or all cell lineages belonging to the extraembryonic tissue. The multipotent stem cells refer to stem cells having an ability to differentiate into a plurality of tissues or cells, although the definition is not applied to all of them. The unipotent stem cells refer to stem cells having an ability to be able to differentiate into a predetermined tissue or cells.

The "pluripotent stem cells" can be induced from, e.g., a fertilized egg, a cloned embryo, germline stem cells, tissue stem cells and somatic cells. Examples of the pluripotent stem cells can include embryonic stem cells (ES cells), embryonic germ cells (EG cells) and induced pluripotent stem cells (iPS cells). Muse cells (Multi-lineage differentiating stress enduring cells) obtained from the mesenchymal stem cells (MSC) and GS cells prepared from germ cells (for example, testis) are included in the pluripotent stem cells.

Human embryonic stem cells were established in 1998 and have been used also for regenerative medicine. The embryonic stem cells can be produced by culturing inner cell aggregate on feeder cells or a culture medium containing bFGF. The method for producing embryonic stem cells is described, for example, in WO96/22362, WO02/101057, US5,843,780, US6,200,806, US6,280,718. The embryonic stem cells are available from a predetermined institution and also, commercially available. For example, human embryonic stem cells such as KhES-1, KhES-2 and KhES-3 are available from the Institute for Frontier Life and Medical Sciences, Kyoto University. Human embryonic stem cells such as Crx::Venus strain (derived from KhES-1) are available from RIKEN.

The "induced pluripotent stem cells" refers to cells having pluripotency, which is induced by reprogramming somatic cells by a method known in the art.

The induced pluripotent stem cells were established in mouse cells by Yamanaka et al., in 2006 (Cell, 2006, 126 (4), pp. 663-676). The induced pluripotent stem cells were also established in human fibroblasts in 2007. The induced pluripotent stem cells have pluripotency and self-renewal ability similarly to embryonic stem cells (Cell, 2007, 131 (5), pp. 861-872; Science, 2007, 318 (5858), pp. 1917-1920; Nat. Biotechnol., 2008, 26 (1), pp. 101-106).

The induced pluripotent stem cells more specifically refer to cells which are induced to be pluripotent by reprogramming somatic cells differentiated into, for example, fibroblasts and peripheral blood mononuclear cells, by allowing any one of sets of a plurality of genes selected from a reprogramming gene group containing Oct3/4, Sox2, Klf4, Myc (c-Myc, N-Myc, L-Myc), Glis1, Nanog, Sall4, lin28 and Esrrb to express. Examples of a preferable set of reprogramming factors may include (1) Oct3/4, Sox2, Klf4, and Myc (c-Myc or L-Myc) and (2) Oct3/4, Sox2, Klf4, Lin28 and L-Myc (Stem Cells, 2013; 31: 458-466).

Other than producing induced pluripotent stem cells through direct reprogramming by gene expression, the pluripotent stem cells can be artificially induced from somatic cells, for example, by adding a chemical compound (Science, 2013, 341, pp. 651-654).

Alternatively, an induced pluripotent stem cell strain is available. For example, human induced pluripotent cell strains established by Kyoto University, such as 201B7 cell, 201B7-Ff cell, 253G1 cell, 253G4 cell, 1201C1 cell, 1205D1 cell, 1210B2 cell and 1231A3 cell, are available form Kyoto University and iPS Academia Japan, Inc. As the induced pluripotent stem cells, for example, Ff-I01 cell, Ff-I14 cell and QHJI01s04 cell established by Kyoto University, are available from Kyoto University.

In the specification, the pluripotent stem cells are preferably embryonic stem cells or induced pluripotent stem cells, more preferably induced pluripotent stem cells.

In the specification, the pluripotent stem cells are human pluripotent stem cells, preferably human induced pluripotent stem cells (iPS cells) or human embryonic stem cells (ES cells).

Pluripotent stem cells such as human iPS cells can be subjected to maintenance culture and expansion culture performed by methods known to those skilled in the art.

The "retinal tissue" means a tissue in which a single type or a plurality of types of retinal cells constituting each retinal layer in a retina *in vivo* are present according to a predetermined order. The "neural retina" is a retinal tissue and means a tissue containing an inside neural retinal layer that does not contain a retinal pigment epithelial layer among retinal layers mentioned later.

The "retinal cells" mean cells constituting each retinal layer in a retina *in vivo* or precursor cells thereof. In the retinal cells, cells such as photoreceptor cells (rod photoreceptor cell, cone photoreceptor cell), horizontal cells, amacrine cells, intermediate neuronal cells, retinal ganglion cells (ganglion cell), bipolar cells (rod bipolar cell, cone bipolar cell), Muller glial cells, retinal pigment epithelial (RPE) cells, ciliary body, their precursor cells (e.g., photoreceptor precursor cell, bipolar precursor cell), and retinal precursor cells are included, though not limited thereto. Among the retinal cells, examples of cells constituting a neural retinal layer (also referred to as neural retina cells or neural retina-related cells) specifically include cells such as photoreceptor cells (rod photoreceptor cell, cone photoreceptor cell), horizontal cells, amacrine cells, intermediate neuronal cells, retinal ganglion cells (ganglion cell), bipolar cells (rod bipolar cell, cone bipolar cell), Muller glial cells, and their precursor cells (e.g., photoreceptor precursor cell, bipolar precursor cell). In other words, in the neural retina-related cells, neither retinal pigment epithelial cells nor ciliary body cells are included.

The "matured retinal cells" mean cells that may be contained in the retinal tissue of a human adult, and specifically mean differentiated cells such as photoreceptor cells (rod photoreceptor cell, cone photoreceptor cell), horizontal cells, amacrine cells, intermediate neuronal cells, retinal ganglion cells (ganglion cell), bipolar cells (rod bipolar cell, cone bipolar cell), Muller glial cells, retinal pigment epithelial (RPE) cells, and ciliary body cells. The "immature retinal cells" mean precursor cells (e.g., photoreceptor precursor cell, bipolar precursor cell, retinal precursor cell) destined for differentiation into matured retinal cells.

The photoreceptor precursor cells, the horizontal precursor cells, the bipolar precursor cells, the amacrine precursor cells, the retinal ganglion precursor cells, the Muller glial precursor cells, and the retinal pigment epithelial precursor cells refer to precursor cells destined for differentiation into photoreceptor cells, horizontal cells, bipolar cells, amacrine cells, retinal ganglion cells, Muller glial cells, and retinal pigment epithelial cells, respectively.

The "retinal precursor cells" are precursor cells capable of differentiating into any one of the immature retinal cells such as photoreceptor precursor cells, horizontal precursor cells, bipolar precursor cells, amacrine precursor cells, retinal ganglion precursor cells, Muller glial cells, and retinal pigment epithelial precursor cells, and refer to precursor cells also capable of eventually differentiating into any one of the matured retinal cells such as photoreceptor cells, rod photoreceptor cells, cone photoreceptor cells, horizontal cells, bipolar cells, amacrine cells, retinal ganglion cells, and retinal pigment epithelial cells.

The "photoreceptor cells" are present in the photoreceptor layer of a retina *in vivo* and plays a role in absorbing light stimuli and converting them to electrical signals. The photoreceptor cells have two types, cones which function in the light and rods which function in the dark (referred to as cone photoreceptor cells and rod photoreceptor cells, respectively). Examples of the cone photoreceptor cells can include S cone photoreceptor cells which express S-opsin and receive blue light, L cone photoreceptor cells which express L-opsin and receive red light, and M cone photoreceptor cells which express M-opsin and receive green light. The photoreceptor cells are matured after differentiation from photoreceptor precursor cells. Whether or not cells are photoreceptor cells or photoreceptor precursor cells can be readily confirmed by those skilled in the art, for example, through the expression of cell markers (Crx and Blimp1 expressed in photoreceptor precursor cells, recoverin expressed in photoreceptor cells, rhodopsin, S-opsin and M/L-opsin expressed in mature photoreceptor cells, etc.) mentioned later or the formation of an outer segment structure. In an embodiment, the photoreceptor precursor cells are Crx-positive cells, and the photoreceptor cells are rhodopsin-, S-opsin- and M/L-opsin-positive cells. In an embodiment, the rod photoreceptor cells are NRL- and rhodopsin-positive cells. In an embodiment, the S cone photoreceptor cells are S-opsin-positive cells, the L cone photoreceptor cells are L-opsin-positive cells, and the M cone photoreceptor cells are M-opsin-positive cells.

The presence of neural retina-related cells can be confirmed from the presence or absence of expression of a neural retina-related cell-related gene (hereinafter, also referred to as "neural retina-related cell marker" or "neural retina marker"). The presence or absence of expression of the neural retina-related cell marker, or the ratio of neural retina-related cell marker-positive cells in a cell population or a tissue can be readily confirmed by those skilled in the art. Examples thereof include an approach using an antibody, an approach using nucleic acid primers, and an approach using sequencing reaction. As the approach using an antibody, the expression of a protein of the neural retina-related cell marker can be confirmed, for example, by dividing the number of predetermined neural retina-related cell marker-positive cells by the total number of cells in accordance with an approach such as flow cytometry or immunostaining using a commercially available antibody. As the approach using nucleic acid primers, the expression of RNA of the neural retina-related cell marker can be confirmed by, for example, PCR, semiquantitative PCR, or quantitative PCR (e.g., real-time PCR). As the approach using sequencing reaction, the expression of RNA of the neural retina-related cell marker can be confirmed using, for example, a nucleic acid sequencer (e.g., next-generation sequencer).

Examples of the neural retina-related cell marker include Rx (also referred to as Rax) and PAX6 expressed in retinal precursor cells, Rx, PAX6 and Chx10 (also referred to as Vsx2) expressed in neural retinal precursor cells, and Crx and Blimp1 expressed in photoreceptor precursor cells. Examples thereof also include Chx10 strongly expressed in bipolar cells, PKCα, Goα, VSX1 and L7 expressed in bipolar cells, TuJ1 and Brn3 expressed in retinal ganglion cells, calretinin and HPC-1 expressed in amacrine cells, calbindin expressed in horizontal cells, recoverin expressed in photoreceptor cells and photoreceptor precursor cells, rhodopsin expressed in rod cells, Nrl expressed in rod photoreceptor cells and rod photoreceptor precursor cells, S-opsin and LM-opsin expressed in cone photoreceptor cells, RXR-y expressed in cone cells, cone photoreceptor precursor cells and ganglion cells, TRβ2, OTX2 and OC2 expressed in cone photoreceptor cells that appear at the early phase of differentiation among cone photoreceptor cells, or precursor cells thereof, and Pax6 commonly expressed in horizontal cells, amacrine cells and ganglion cells.

The "positive cells" mean cells expressing a predetermined marker on the cell surfaces or within the cells. For example, the "Chx10-positive cells" mean cells expressing Chx10 protein.

The "retinal pigment epithelial cells" mean epithelial cells present outside the neural retina in a retina *in vivo.* Whether or not cells are retinal pigment epithelial cells can be readily confirmed by those skilled in the art, for example, through the expression of cell markers (RPE65, MITF, CRALBP, MERTK, BEST1, TTR, etc.), the presence of melanin granules (brown-black), intercellular tight junctions, or polygonal/flagstone-like characteristic cell morphology. Whether or not cells have a function of retinal pigment epithelial cells can be readily confirmed from the ability to secrete cytokines such as VEGF and PEDF. In an embodiment, the retinal pigment epithelial cells are RPE65-positive cells, MITF-positive cells, or RPE65-positive and MITF-positive cells.

The "retinal layer" means individual layers constituting the retina, and examples thereof can specifically include retinal pigment epithelial layer, photoreceptor layer, outer limiting membrane, outer nuclear layer, outer plexiform layer, inner nuclear layer, inner plexiform layer, ganglion cell layer, nerve fiber layer and inner limiting membrane.

The "neural retinal layer" means individual layers constituting the neural retina, and examples thereof can specifically include photoreceptor layer, outer limiting membrane, outer nuclear layer, outer plexiform layer, inner nuclear layer, inner plexiform layer, ganglion cell layer, nerve fiber layer and inner limiting membrane. The "photoreceptor layer" means a retinal layer that is formed in the outermost of the neural retina and is rich in photoreceptor cells (rod photoreceptor cell, cone photoreceptor cell), photoreceptor precursor cells and retinal precursor cells. Each layer other than the photoreceptor layer is referred to as an inner layer. Which retinal layer the individual cells constitute can be confirmed by a known method, for example, by determining the presence or absence of expression or expression level of a cell marker.

In the case of retinal tissue at a stage where the appearance ratio of photoreceptor cells or photoreceptor precursor cells is low, a layer containing proliferating neural retinal precursor cells is referred to as "neuroblastic layer" and includes inner neuroblastic layer and outer neuroblastic layer. Those skilled in the art can make a judgment from the shade of color (the outer neuroblastic layer is light, and the inner neuroblastic layer is dark) by a known method, for example, under a bright field microscope.

The "ciliary body" includes "ciliary body" and "ciliary marginal zone" in the process of development and of an adult. Examples of a marker of the "ciliary body" include Zic1, MAL, HNF1beta, FoxQ1, CLDN2, CLDN1, GPR177, AQP1 and AQP4. Examples of the "ciliary marginal zone (CMZ)" can include a tissue that is present in a boundary region between the neural retina and the retinal pigment epithelium in a retina *in vivo,* and is a region containing tissue stem cells of the retina (retinal stem cells). The ciliary marginal zone is also called ciliary margin or retinal margin, and the ciliary marginal zone, the ciliary margin and the retinal margin are equivalent tissues. The ciliary marginal zone is known to play an important role in the supply of retinal precursor cells or differentiated cells to retinal tissue, the maintenance of a retinal tissue structure, etc. Examples of a marker gene of the ciliary marginal zone can include Rdh10 gene (positive), Otx1 gene (positive) and Zic1 (positive). The "ciliary marginal zone-like structure" is a structure similar to the ciliary marginal zone.

The "cell aggregate" is not particularly limited as long as a plurality of cells mutually adhere to form a three-dimensional structure, and refers to, for example, a mass formed by the aggregation of cells dispersed in a vehicle such as a culture medium, or a mass of cells formed through cell division. In the cell aggregate, the case of forming a predetermined tissue is also included.

The "sphere-like cell aggregate" means a cell aggregate having a stereoscopic shape close to a spherical shape. The stereoscopic shape close to a spherical shape is a shape having a three-dimensional structure, and examples thereof include a spherical shape that exhibits a circle or an ellipse when projected onto a two-dimensional surface, and a shape formed by fusing a plurality of spherical shapes (e.g., which exhibits a shape formed by 2 to 4 circles or ellipses overlapping when two-dimensionally projected). In an embodiment, the core part of the aggregate has a vesicular lamellar structure and is characterized in that the central part is observed to be dark and the outer edge portion is observed to be bright under a bright field microscope.

In an embodiment, epithelial tissue is polarized so that "apical surface" and "basal membrane" are formed. The "basal membrane" refers to the basal membrane in which a basal side layer (basal membrane) rich in laminin and IV-type collagen, being 50-100 nm and produced by epithelial cells, is present. The "apical surface" refers to the surface (upper surface layer) formed on the opposite side to the "basal membrane". In an embodiment, in the retinal tissue developed to the extent that photoreceptor cells or photoreceptor precursor cells are observed, the "apical surface" refers to a surface in contact with photoreceptor layer (outer nuclear layer) in which outer limiting membrane is formed and photoreceptor cells and photoreceptor precursor cells are present. Such an apical surface can be identified by, for example, immunostaining (known to those skilled in the art) using an antibody against an apical surface marker (e.g., atypical PKC (hereinafter, abbreviated to "aPKC"), E-cadherin, N-cadherin).

The "epithelial tissue" is a tissue formed by covering the body surface or the surface of a lumen (digestive tract, etc.), body cavity (pericardial cavity, etc.) or the like with cells without any space. The cells forming the epithelial tissue are referred to as epithelial cells. The epithelial cells have a polarity in the apical-basal direction. The epithelial cells can mutually and firmly join via adherence junction and/or tight junction to form a layer of the cells. A tissue formed from a single layer or dozen layers overlapping of this layer of the cells is the epithelial tissue. In a tissue capable of forming the epithelial tissue, retinal tissue, brain and spinal cord tissue, eyeball tissue, neural tissue or the like of a fetal stage and/or an adult is also included. In the specification, the neural retina is also the epithelial tissue. The "epithelial structure" means a structure characteristic of the epithelial tissue, such as apical surface or basal membrane.

The "continuous epithelial tissue" is a tissue having a continuous epithelium structure. The continuous epithelium structure is a structure where the epithelial tissue is continuously formed. The epithelium tissue continuously formed is a state in which 10 cells to 10⁷ cells, for example, in the tangent direction of the epithelial tissue, preferably 30 cells to 10⁷ cells, further preferably 10² cells to 10⁷ cells, in the tangent direction, are aligned.

For example, in the continuous epithelium structure formed in retinal tissue, the retinal tissue has an apical surface intrinsic to the epithelial tissue. The apical surface is formed almost in parallel to, for example, at least photoreceptor layer (outer nuclear layer) among the layers forming a neural retinal layer and continuously on the surface of the retinal tissue. For example, in the case of a cell aggregate containing retinal tissue prepared from pluripotent stem cells, the apical surface is formed on the surface of the aggregate by regularly and continuously aligning 10 cells or more, preferably 30 cells or more, more preferably 100 cells or more, further preferably 400 cells or more of photoreceptor cells or photoreceptor precursor cells in the tangent direction of the surface.

### [Method for evaluating quality of transplant neural retina]

An aspect of the present invention is a method for evaluating the quality of a transplant neural retina.

The method according to the present invention comprises: sampling a part or the whole of a cell aggregate containing a neural retina having an epithelial structure derived from a pluripotent stem cell as a sample for quality evaluation; detecting the expression of a neural retina-related cell (target cell)-related gene and a non-neural retina-related cell (non-target cell)-related gene in the sample for quality evaluation; and when the expression of the neural retina-related cell-related gene (target cell-related gene) is found and the expression of the non-neural retina-related cell-related gene (non-target cell-related gene) is not found, determining that (1) the neural retina (transplant neural retina) in the same cell aggregate as the cell aggregate containing the sample for quality evaluation being the part, (2) the neural retina (transplant neural retina) in a cell aggregate of the same lot as the cell aggregate containing the sample for quality evaluation being the part, or (3) the neural retina (transplant neural retina) in a cell aggregate of the same lot as the cell aggregate of the sample for quality evaluation being the whole, is applicable as the transplant neural retina. When the expression of the neural retina-related cell-related gene (target cell-related gene) is found and the expression of the non-neural retina-related cell-related gene (non-target cell-related gene) is not found in the sample for quality evaluation, it is determined that the sample for quality evaluation is also applicable as the transplant neural retina. However, the sample for quality evaluation which is the transplant neural retina is destroyed for evaluation and as such, cannot actually be used in transplantation.

### <Cell aggregate containing neural retina>

### (Method for producing cell aggregate)

In the specification, the cell aggregate containing a neural retina has an epithelial structure and can be obtained by differentiating pluripotent stem cells. An embodiment includes a method for producing the cell aggregate containing a neural retina using a differentiation factor. Examples of the differentiation factor include basal membrane preparations, BMP signaling pathway agonists, Wnt signaling pathway inhibitors, and IGF signaling pathway agonists. An embodiment includes a method for producing the cell aggregate containing a neural retina by self-organization. The self-organization refers to a mechanism under which a population of cells autonomically yields a complicated structure. The self-organization can be performed by, for example, SFEB (serum-free floating culture of embryoid bodies-like aggregates) (WO2005/12390) or SFEBq (WO2009/148170).

Examples of a specific differentiation method include, but are not particularly limited to, methods disclosed in WO2011/055855, WO2013/077425, WO2015/025967, WO2016/063985, WO2016/063986, WO2017/183732, PLoS One. 2010 Jan 20; 5 (1): e8763, Stem Cells. 2011 Aug; 29 (8): 1206-18, Proc Natl Acad Sci USA. 2014 Jun 10; 111 (23): 8518-23, and Nat Commun. 2014 Jun 10; 5: 4047.

In a specific embodiment, the cell aggregate containing a neural retina can be prepared by a method comprising the following steps (A), (B) and (C):
(A) culturing pluripotent stem cells in a culture medium containing a factor for maintaining undifferentiated state in the absence of feeder cells;
(B) forming a cell aggregate by suspension-culturing the cells obtained in the step (A); and
(C) further suspension-culturing the cell aggregate obtained in the step (B) in a culture medium containing a BMP signaling pathway agonist.

The step (A) may further involve a TGFβ family signaling pathway inhibitor and/or a sonic hedgehog signaling pathway agonist.

Also, the step (B) may involve a sonic hedgehog signaling pathway agonist and/or a Wnt signaling pathway inhibitor, as mentioned later.

This method is also disclosed in, for example, WO2015/025967, WO2016/063985, and WO2017/183732. For more details, see WO2015/025967, WO2016/063985, and WO2017/183732.

The culture medium that is used in the preparation of the cell aggregate containing a neural retina can employ a basal medium for cell proliferation (also referred to as a basal medium), unless otherwise specified. The basal medium for cell proliferation is not particularly limited as long as the culture of cells is possible. A basal medium commercially available as a culture medium for cell proliferation can be appropriately used. Specifically, examples thereof can include culture media that can be used in the culture of animal cells, such as BME medium, BGJb medium, CMRL 1066 medium, Glasgow MEM(GMEM) medium, Improved MEM Zinc Option medium, IMDM medium, Medium 199 medium, MEM medium, Eagle MEM medium, αMEM medium, DMEM medium, F-12 medium, DMEM/F12 medium, IMDM/F12 medium, Ham's medium, RPMI 1640 medium, Fischer's medium, Leibovitz's L-15 medium and mixtures of these media. Alternatively, a culture medium supplemented with N2 medium which is an assisted culture medium may be used.

The TGFβ family signaling pathway inhibitor refers to a substance inhibiting the TGFβ family signaling pathway, i.e., the signaling pathway transduced by the Smad family. Specifically, examples thereof can include TGFβ signaling pathway inhibitors (e.g., SB431542, LY-364947, SB505124, A-83-01), Nodal/activin signaling pathway inhibitors (e.g., SB431542, A-83-01) and BMP signaling pathway inhibitors (e.g., LDN193189, dorsomorphin). These substances are commercially available and can be obtained.

The sonic hedgehog (hereinafter, also referred to as "Shh") signaling pathway agonist is a substance capable of enhancing signal transduction mediated by Shh. Examples of the Shh signaling pathway agonist include SHH, partial peptides of SHH, PMA (purmorphamine), and SAG (smoothened agonist).

The concentrations of the TGFβ family signaling pathway inhibitor and the sonic hedgehog signaling pathway agonist can be concentrations capable of inducting differentiation into retinal cells. For example, SB431542 is used at a concentration of usually 0.1 to 200 µM, preferably 2 to 50 µM. A-83-01 is used at a concentration of usually 0.05 to 50 µM, preferably 0.5 to 5 µM. LDN193189 is used at a concentration of usually 1 to 2000 nM, preferably 10 to 300 nM. SAG is used at a concentration of usually 1 to 2000 nM, preferably 10 to 700 nM. PMA is used at a concentration of usually 0.002 to 20 µM, preferably 0.02 to 2 µM.

The factor for maintaining undifferentiated state is not particularly limited as long as it is a substance having an action of suppressing the differentiation of pluripotent stem cells. Examples of the factor for maintaining undifferentiated state that is generally used by those skilled in the art can include FGF signaling pathway agonists, TGFβ family signaling pathway agonists, and insulin. Examples of the FGF signaling pathway agonist specifically include fibroblast growth factors (e.g., bFGF, FGF4, FGF8). Examples of the TGFβ family signaling pathway agonist include TGFβ signaling pathway agonists and Nodal/activin signaling pathway agonists. Examples of the TGFβ signaling pathway agonist include TGFβ1 and TGFβ2. Examples of the Nodal/activin signaling pathway agonist include Nodal, activin A, and activin B. In the case of culturing human pluripotent stem cells (human ES cells, human iPS cells), the culture medium in the first step preferably contains bFGF as the factor for maintaining undifferentiated state.

The concentration of the factor for maintaining undifferentiated state in the culture medium that is used in the first step is a concentration capable of maintaining the undifferentiated state of the pluripotent stem cells to be cultured, and can be appropriately set by those skilled in the art. For example, specifically, in the case of using bFGF as the factor for maintaining undifferentiated state in the absence of feeder cells, its concentration is usually on the order of 4 ng to 500 ng/mL, preferably on the order of 10 ng to 200 ng/mL, more preferably on the order of 30 ng to 150 ng/mL.

Many synthetic media have been developed or are commercially available as feeder-free media containing the factor for maintaining undifferentiated state and applicable for culturing pluripotent stem cells. Examples thereof include Essential 8 medium (manufactured by Life Technologies Corp.). The Essential 8 medium contains L-ascorbic acid-2-phosphate magnesium (64 mg/L), sodium selenium (14 µg/L), insulin (19.4 mg/L), NaHCO₃ (543 mg/L), transferrin (10.7 mg/L), bFGF (100 ng/mL), and the TGFβ family signaling pathway agonist (TGFβ1 (2 ng/mL) or Nodal (100 ng/mL)) as additives in DMEM/F12 medium (Nature Methods, 8, 424-429 (2011)). Examples of other commercially available feeder-free media include S-medium (manufactured by DS Pharma Biomedical Co., Ltd.), StemPro (manufactured by Life Technologies Corp.), hESF9 (Proc. Natl. Acad. Sci. USA. 2008 Sep 9; 105 (36): 13409-14), mTeSR1 (manufactured by STEMCELL Technologies Inc.), mTeSR2 (manufactured by STEMCELL Technologies Inc.), TeSR-E8 (manufactured by STEMCELL Technologies Inc.), and StemFit (manufactured by Ajinomoto Co., Inc.). In the first step, the present invention can be conveniently carried out by using these. By using these culture media, it is possible to perform the culture of pluripotent stem cells under feeder-free conditions. The culture medium that is used in the step (A) is, as one example, a serum-free medium that is not supplemented with any of the BMP signaling pathway agonist, the Wnt signaling pathway agonist and the Wnt signaling pathway inhibitor.

In the culture of pluripotent stem cells under feeder-free conditions in the step (A), a suitable matrix may be used as a scaffold in order to provide a scaffold as a replacement for feeder cells to the pluripotent stem cells. Examples of the matrix that can be used as a scaffold include laminin (Nat Biotechnol 28, 611-615, (2010)), laminin fragments (Nat Commun 3, 1236, (2012)), basal membrane preparations (Nat Biotechnol 19, 971-974, (2001)), gelatin, collagen, heparan sulfate proteoglycan, entactin, and vitronectin.

The culture time of the pluripotent stem cells in the step (A) is not particularly limited within a range in which an effect of improving the quality of the cell aggregate to be formed in the step (B) can be achieved in the case of culture in the presence of the TGFβ family signaling pathway inhibitor and/or the sonic hedgehog signaling pathway agonist (e.g., from 100 nM to 700 nM), and is usually from 0.5 to 144 hours. In an embodiment, it is preferably from 2 to 96 hours, more preferably from 6 to 48 hours, further preferably from 12 to 48 hours, still further preferably from 18 to 28 hours (e.g., 24 hours).

The culture medium that is used in the step (B) may be a serum-containing medium or a serum-free medium. A serum-free medium is suitably used from the viewpoint of circumventing contamination with chemically undetermined components. In order to circumvent the complication of preparation, examples thereof include serum-free media supplemented with an appropriate amount of a serum replacement such as commercially available KSR. The amount of KSR added to the serum-free medium is usually from about 1% to about 30%, preferably from about 2% to about 20%.

For the formation of the aggregate, first, dispersed cells are prepared by the dispersion operation of the cells obtained in the step (A). The "dispersed cells" obtained by dispersion operation include a state in which 70% (preferably 80% or more) or more are single cells and 30% or less (preferably 20% or less) of 2- to 50-cell masses are present. The dispersed cells include a state in which the mutual adhesion (e.g., surface adhesion) of cells has been mostly lost.

A suspension of the dispersed cells is seeded into an incubator, and the dispersed cells are cultured under conditions of non-adhesive to the incubator, thereby causing the aggregation of a plurality of cells to form an aggregate. In an embodiment, when a predetermined number of dispersed stem cells is placed in each well of a multi-well plate (U-bottom, V-bottom) such as a 96-well plate and this is statically cultured, the cells aggregate rapidly, thereby forming one aggregate in each well (SFEBq). In the case of suspension-culturing cells using a 96-well plate, a liquid prepared so as to attain about 1 × 10³ to about 1 × 10⁵ cells (preferably about 3 × 10³ to about 5 × 10⁴ cells or about 4 × 10³ to about 2 × 10⁴ cells) per well is added to the wells, and the plate is left standing to form aggregates.

In an embodiment, the culture medium that is used in the step (B) contains a sonic hedgehog signaling pathway agonist.

In other words, in a specific embodiment, the cell aggregate containing a neural retina can be prepared by a method comprising the following steps (A), (B) and (C):
(A) culturing pluripotent stem cells in a culture medium containing a factor for maintaining undifferentiated state and optionally containing a TGFβ family signaling pathway inhibitor and/or a sonic hedgehog signaling pathway agonist in the absence of feeder cells;
(B) forming a cell aggregate by suspension-culturing the cells obtained in the step (A) in a culture medium containing a sonic hedgehog signaling pathway agonist; and
(C) further suspension-culturing the cell aggregate obtained in the step (B) in a culture medium containing a BMP signaling pathway agonist.

As the sonic hedgehog signaling pathway agonist in the step (B), the one mentioned above can be used at the concentration mentioned above (e.g., from 10 nM to 300 nM). The sonic hedgehog signaling pathway agonist is preferably contained in the culture medium from the start of suspension culture. A ROCK inhibitor (e.g., Y-27632) may be added to the culture medium. The culture time is, for example, from 12 hours to 6 days. The culture medium that is used in the step (B) is, as one example, a culture medium that is not supplemented with one or more (preferably all) selected from the group consisting of a BMP signaling pathway agonist, a Wnt signaling pathway agonist, a TGFβ family signaling pathway inhibitor and a TGFβ family signaling pathway agonist.

The BMP signaling pathway agonist is a substance capable of enhancing the signaling pathway mediated by BMP. Examples of the BMP signaling pathway agonist include BMP protein such as BMP2, BMP4 and BMP7, GDF protein such as GDF7, anti-BMP receptor antibodies, and BMP partial peptides. The BMP2 protein, the BMP4 protein and the BMP7 protein are available from, for example, R&D Systems, Inc., and the GDF7 protein is available from, for example, Wako Pure Chemical Industries, Ltd.

Examples of the culture medium that is used in the step (C) include serum-free media and serum media (preferably serum-free media) supplemented with a BMP signaling pathway agonist. The serum-free medium and the serum medium can be provided as mentioned above. The culture medium that is used in the step (C) is, as one example, a culture medium that is not supplemented with one or more (preferably all) selected from the group consisting of a Wnt signaling pathway agonist, a TGFβ family signaling pathway inhibitor and a TGFβ family signaling pathway agonist. Alternatively, the culture medium that is used in the step (C) is, as one example, a culture medium that is not supplemented with a sonic hedgehog signaling pathway agonist. Alternatively, the culture medium that is used in the step (C) is a culture medium that may be supplemented with a Wnt signaling pathway agonist.

The concentration of the BMP signaling pathway agonist can be a concentration capable of inducing differentiation into retinal cells. For example, human BMP4 protein is added to the culture medium so as to attain a concentration of about 0.01 nM to about 1 µM, preferably about 0.1 nM to about 100 nM, more preferably about 1 nM to about 10 nM, further preferably about 1.5 nM (55 ng/mL).

The BMP signaling pathway agonist can be added about 24 hours or later after the start of suspension culture in the step (A), and may be added to the culture medium within several days (e.g., within 15 days) after the start of suspension culture. Preferably, the BMP signaling pathway agonist is added to the culture medium between Day 1 and Day 15, more preferably between Day 1 and Day 9, most preferably on Day 3, after the start of suspension culture.

In a specific embodiment, a part or the whole of the culture medium is exchanged with a culture medium containing BMP4, for example, on Days 1 to 9, preferably Days 1 to 3, after the start of suspension culture in the step (B) to adjust the final concentration of BMP4 to about 1 to 10 nM. Culture can be performed for, for example, 1 to 12 days, preferably 2 to 9 days, further preferably 2 to 5 days, in the presence of BMP4. In this context, in order to maintain the concentration of BMP4 at the same concentration, a part or the whole of the culture medium can be exchanged with a culture medium containing BMP4 once or about twice. Alternatively, the concentration of BMP4 may be decreased in stages. For example, the concentration of the BMP signaling pathway agonist (BMP4) is maintained from Days 2 to 10 after the start of suspension culture in the step (B), and then, the concentration of the BMP signaling pathway agonist (BMP4) may be decreased in stages from Days 6 to 20 after the start of suspension culture in the step (B).

Culture conditions such as culture temperature and CO₂ concentration in the step (A) to the step (C) can be appropriately set. The culture temperature is, for example, from about 30°C to about 40°C, preferably about 37°C. The CO₂ concentration is, for example, from about 1% to about 10%, preferably about 5%.

Retinal cells at various stages of differentiation can be produced as retinal cells contained in the cell aggregate by varying the culture period in the step (C). In other words, retinal cells in the cell aggregate containing immature retinal cells (e.g., retinal precursor cell, photoreceptor precursor cell) and matured retinal cells (e.g., photoreceptor cell) at various ratios can be produced. The ratio of matured retinal cells can be increased by extending the culture period in the step (C).

The step (B) and/or the step (C) may employ a method disclosed in WO2017/183732. Specifically, in the step (B) and/or the step (C), the cell aggregate can be formed by suspension culture in a culture medium further containing a Wnt signaling pathway inhibitor.

The Wnt signaling pathway inhibitor that is used in the step (B) and/or the step (C) is not particularly limited as long as it is capable of suppressing signal transduction mediated by Wnt, and may be any of a protein, a nucleic acid, a low-molecular compound, and the like. Signals mediated by Wnt are transduced via Wnt receptor present as a heterodimer of frizzled (Fz) and LRP5/6 (low-density lipoprotein receptor-related protein 5/6). Examples of the Wnt signaling pathway inhibitor include, but are not limited to, substances acting directly on Wnt or Wnt receptor (anti-Wnt neutralizing antibody, anti-Wnt receptor neutralizing antibody, etc.), substances suppressing the expression of a gene encoding Wnt or Wnt receptor (e.g., antisense oligonucleotide, siRNA), substances inhibiting the binding of Wnt to Wnt receptor (soluble Wnt receptor, dominant negative Wnt receptor, etc., Wnt antagonist, Dkk1, Cerberus protein, etc.), and substances inhibiting bioactivity caused by signal transduction ascribable to Wnt receptor [e.g., low-molecular compounds such as CKI-7 (N-(2-aminoethyl)-5-chloroisoquinoline-8-sulfonamide), D4476 (4-[4-(2,3-dihydro-1,4-benzodioxin-6-yl)-5-(2-pyridinyl)-1H-imidazol-2-yl]benzamide), IWR-1-endo (IWR1e) (4-[(3aR,4S,7R,7aS)-1,3,3a,4,7,7a-hexahydro-1,3-dioxo-4,7-methano-2H-isoindol-2-yl]-N-8-quinolinyl-benzamide), and IWP-2 (N-(6-methyl-2-benzothiazolyl)-2-[(3,4,6,7-tetrahydro-4-oxo-3-phenylthieno[3,2-d]pyrimidin-2-yl)thio]acetamide)]. One or two or more of these may be contained as the Wnt signaling pathway inhibitor. CKI-7, D4476, IWR-1-endo (IWR1e), IWP-2, and the like are known Wnt signaling pathway inhibitors, and commercially available products, etc. can be appropriately obtained. IWR1e is preferably used as the Wnt signaling pathway inhibitor.

The concentration of the Wnt signaling pathway inhibitor in the step (B) can be a concentration capable of inducing the favorable formation of the cell aggregate. For example, IWR-1-endo is added to the culture medium so as to attain a concentration of about 0.1 µM to about 100 µM, preferably about 0.3 µM to about 30 µM, more preferably about 1 µM to about 10 µM, further preferably about 3 µM. In the case of using a Wnt signaling pathway inhibitor other than IWR-1-endo, it is desirable to be used at a concentration that exhibits Wnt signaling pathway inhibitory activity equivalent to the concentration of IWR-1-endo.

In the step (B), the timing of adding the Wnt signaling pathway inhibitor to the culture medium is preferably earlier. The Wnt signaling pathway inhibitor is added to the culture medium usually within 6 days, preferably within 3 days, more preferably within 1 day, more preferably within 12 hours, from the start of suspension culture in the step (B), further preferably at the start of suspension culture in the step (B). Specifically, for example, the addition of a basal medium supplemented with the Wnt signaling pathway inhibitor, or the exchange of a part or the whole of the culture medium with the basal medium can be performed. Although a period for which the Wnt signaling pathway inhibitor is allowed to act on the cells obtained in the step (A) in the step (B) is not particularly limited, preferably, it is added to the culture medium at the start of suspension culture in the step (B) and then allowed to act until the completion of the step (B) (immediately before addition of a BMP signaling pathway agonist). Further preferably, as mentioned later, exposure to the Wnt signaling pathway inhibitor is continued even after the completion of the step (B) (i.e., during the period of the step (C)). In an embodiment, as mentioned later, the action of the Wnt signaling pathway inhibitor is continued even after the completion of the step (B) (i.e., during the period of the step (C)), and the action may be performed until retinal tissue is formed.

In the step (C), as the Wnt signaling pathway inhibitor, any of the Wnt signaling pathway inhibitors mentioned above can be used. Preferably, the same type as the Wnt signaling pathway inhibitor used in the step (B) is used in the step (C).

The concentration of the Wnt signaling pathway inhibitor in the step (C) can be a concentration capable of inducing retinal precursor cells and retinal tissue. For example, IWR-1-endo is added to the culture medium so as to attain a concentration of about 0.1 µM to about 100 µM, preferably about 0.3 µM to about 30 µM, more preferably about 1 µM to about 10 µM, further preferably about 3 µM. In the case of using a Wnt signaling pathway inhibitor other than IWR-1-endo, it is desirable to be used at a concentration that exhibits Wnt signaling pathway inhibitory activity equivalent to the concentration of IWR-1-endo. The concentration of the Wnt signaling pathway inhibitor in the culture medium in the step (C) is preferably 50 to 150, more preferably 80 to 120, further preferably 90 to 110, when the concentration of the Wnt signaling pathway inhibitor in the culture medium in the step (B) is defined as 100. It is more preferable to be equivalent to the concentration of the Wnt signaling pathway inhibitor in the culture medium in the second step.

The timing of addition of the Wnt signaling pathway inhibitor to the culture medium is not particularly limited within a range that can achieve the formation of an aggregate containing retinal cells or retinal tissue, and is preferably earlier. Preferably, the Wnt signaling pathway inhibitor is added to the culture medium at the start of the step (C). More preferably, the Wnt signaling pathway inhibitor is added in the step (B) and then also continuously (i.e., from the start of the step (B)) contained in the culture medium in the step (C). Further preferably, the Wnt signaling pathway inhibitor is added at the start of suspension culture in the step (B) and then also continuously contained in the culture medium in the step (C). For example, a BMP signaling pathway agonist (e.g., BMP4) can be added to the cultures (suspension of aggregates in a culture medium containing a Wnt signaling pathway inhibitor) obtained in the step (B).

A period for which the Wnt signaling pathway inhibitor is allowed to act is not particularly limited, but is preferably from 2 days to 30 days, more preferably from 6 days to 20 days, from 8 days to 18 days, from 10 days to 18 days, or from 10 days to 17 days (e.g., 10 days), with the start of suspension culture in the step (B) as a commencement when the Wnt signaling pathway inhibitor is added at the start of suspension culture in the step (B). In another embodiment, the period for which the Wnt signaling pathway inhibitor is allowed to act is preferably from 3 days to 15 days (e.g., 5 days, 6 days, 7 days), more preferably from 6 days to 10 days (e.g., 6 days), with the start of suspension culture in the step (B) as a commencement when the Wnt signaling pathway inhibitor is added at the start of suspension culture in the step (B).

A neural retina having a ciliary marginal zone-like structure can also be produced by culturing the cell aggregate obtained by the method mentioned above in a serum-free medium or a serum medium containing a Wnt signaling pathway agonist and/or a FGF signaling pathway inhibitor for a period on the order of 2 days to 4 days (step (D)), followed by culture in a serum-free medium or a serum medium containing neither a Wnt signaling pathway agonist nor a FGF signaling pathway inhibitor for about 30 days to about 200 days (from 30 days to 150 days, from 50 days to 120 days, from 60 days to 90 days) (step (E)).

In an embodiment, a neural retina having a ciliary marginal zone-like structure can be produced by the step (D) and the step (E) from the cell aggregate obtained in the steps (A) to (C), the cell aggregate being of Days 6 to 30 or Days 10 to 20 (Day 10, Day 11, Day 12, Day 13, Day 14, Day 15, Day 16, Day 17, Day 18, Day 19 or Day 20) after the start of suspension culture in the step (B).

The Wnt signaling pathway agonist is not particularly limited as long as it is capable of enhancing signal transduction mediated by Wnt. Examples of a specific Wnt signaling pathway agonist can include GSK3β inhibitors (e.g., 6-bromoindirubin-3'-oxime (BIO), CHIR99021, kenpaullone). For example, in the case of CHIR99021, the range of about 0.1 µM to about 100 µM, preferably about 1 µM to about 30 µM, can be included.

The FGF signaling pathway inhibitor is not particularly limited as long as it can inhibit signal transduction mediated by FGF. Examples of the FGF signaling pathway inhibitor include SU-5402, AZD4547, and BGJ398. For example, SU-5402 is added at a concentration of about 0.1 µM to about 100 µM, preferably about 1 µM to about 30 µM, more preferably about 5 µM.

The culture medium that is used in the step (D) is, as one example, a culture medium that is not supplemented with one or more (preferably all) selected from the group consisting of a BMP signaling pathway agonist, a Wnt signaling pathway inhibitor, a SHH signaling pathway agonist, a TGFβ family signaling pathway inhibitor and a TGFβ family signaling pathway agonist.

A part of the step (E) or the whole step can perform culture using a culture medium for continuous epithelial tissue maintenance disclosed in WO2019/017492. Specifically, the continuous epithelium structure of the neural retina can be maintained by culture using a culture medium for continuous epithelial tissue maintenance. One example of the culture medium for continuous epithelial tissue maintenance can include a medium in which Neurobasal medium (e.g., manufactured by Thermo Fisher Scientific Inc., 21103049) is blended with B27 supplement (e.g., Thermo Fisher Scientific Inc., 12587010).

For the culture in the step (E), exchange with the culture medium for continuous epithelial tissue maintenance in stages is preferable for achieving both the differentiation and/or maturation of retinal cells (particularly, photoreceptor cell) and the maintenance of the continuous epithelium structure. For example, culture can be performed using a basal medium for cell proliferation (e.g., a culture medium in which DMEM/F12 medium is supplemented with 10% fetal bovine serum, 1% N2 supplement, and 100 µM taurine) for first 10 days to 30 days, a mixture of a basal medium for cell proliferation and a culture medium for continuous epithelial tissue maintenance (culture medium in which a medium in which DMEM/F12 medium is supplemented with 10% fetal bovine serum, 1% N2 supplement, and 100 µM taurine, and a medium in which Neurobasal medium is supplemented with 10% fetal bovine serum, 2% B27 supplement, 2 mM glutamine, and 100 µM taurine, are mixed at a ratio of 1:3) for next 10 days to 40 days, and a culture medium for continuous epithelial tissue maintenance (e.g., a culture medium in which Neurobasal medium is supplemented with 10% fetal bovine serum, 2% B27 supplement, 2 mM glutamine, and 100 µM taurine) for next 20 days to 140 days.

In a part of the step (E) or the whole step, in the case of using any medium of the basal medium for cell proliferation, the culture medium for continuous epithelial tissue maintenance or a mixture of these media, a thyroid hormone signaling pathway agonist may be further contained. By culture in a culture medium containing a thyroid hormone signaling pathway agonist, the production of a cell aggregate containing a neural retina becomes possible in which the ratio of bipolar cells, amacrine cells, ganglion cells or horizontal cells, etc. contained in the neural retina is low and the ratio of photoreceptor precursor cells has been increased.

In the specification, the thyroid hormone signaling pathway agonist is a substance capable of enhancing signal transduction mediated by thyroid hormone, and is not particularly limited as long as it is capable of enhancing the thyroid hormone signaling pathway. Examples of the thyroid hormone signaling pathway agonist include triiodothyronine (hereinafter, also abbreviated to T3), thyroxin (hereinafter, also abbreviated to T4), and thyroid hormone receptor (preferably TRβ receptor) agonists.

Examples of the thyroid hormone receptor agonist known to those skilled in the art can include compounds such as diphenylmethane derivatives, diaryl ether derivatives, pyridazine derivatives, pyridine derivatives and indole derivatives described in International Publication No. WO 97/21993, International Publication No. WO 2004/066929, International Publication No. WO 2004/093799, International Publication No. WO 2000/039077, International Publication No. WO 2001/098256, International Publication No. WO 2003/018515, International Publication No. WO 2003/084915, International Publication No. WO 2002/094319, International Publication No. WO 2003/064369, Japanese Unexamined Patent Publication No. 2002-053564, Japanese Unexamined Patent Publication No. 2002-370978, Japanese Unexamined Patent Publication No. 2000-256190, International Publication No. WO 2007/132475, International Publication No. WO 2007/009913, International Publication No. WO 2003/094845, International Publication No. WO 2002/051805 or International Publication No. WO 2010/122980.

In the case of using T3 as the thyroid hormone signaling pathway agonist, it can be added to the culture medium so as to attain, for example, the range of 0.1 to 1000 nM. Preferably, examples thereof include concentrations having thyroid hormone signaling enhancing activity that corresponds to T3 with a concentration of 1 to 500 nM; more preferably 10 to 100 nM; further preferably 30 to 90 nM; still more preferably around 60 nM. In the case of using T4 as the thyroid hormone signaling pathway agonist, it can be added to the culture medium so as to attain, for example, the range of 1 nM to 500 µM. Preferably, it is the range of 50 nM to 50 µM; more preferably 500 nM to 5 µM. In the case of using other thyroid hormone receptor agonists, the concentration can exhibit activity equivalent to the agonist activity exhibited by T3 or T4 with the concentration mentioned above.

The culture medium that is used in the step (E) may appropriately contain L-glutamine, taurine, serum, or the like. The culture medium that is used in the step (E) is, as one example, a culture medium that is not supplemented with one or more (preferably all) selected from the group consisting of a BMP signaling pathway agonist, a FGF signaling pathway inhibitor, a Wnt signaling pathway agonist, a Wnt signaling pathway inhibitor, a SHH signaling pathway agonist, a TGFβ family signaling pathway inhibitor and a TGFβ family signaling pathway agonist.

In a specific embodiment, the cell aggregate containing a neural retina can be prepared by a method comprising the following steps (A) to (E):
(A) culturing pluripotent stem cells in a culture medium containing a factor for maintaining undifferentiated state and optionally containing a TGFβ family signaling pathway inhibitor and/or a sonic hedgehog signaling pathway agonist in the absence of feeder cells;
(B) forming a cell aggregate by suspension-culturing the cells obtained in the step (A) in a culture medium optionally containing a Wnt signaling pathway inhibitor and/or a sonic hedgehog signaling pathway agonist;
(C) further suspension-culturing the cell aggregate obtained in the step (B) in a culture medium containing a BMP signaling pathway agonist;
(D) culturing the cell aggregate obtained in the step (C) in a serum-free medium or a serum medium containing a Wnt signaling pathway agonist and/or a FGF signaling pathway inhibitor for a period on the order of 2 days to 4 days; and
(E) culturing the cell aggregate obtained in the step (D) in a serum-free medium or a serum medium containing neither a Wnt signaling pathway agonist nor a FGF signaling pathway inhibitor and optionally containing a thyroid hormone signaling pathway agonist for about 30 days to about 200 days.

In a specific embodiment, the cell aggregate containing a neural retina can be prepared by a method comprising the following steps (A) to (E):
(A) culturing pluripotent stem cells in a culture medium containing a factor for maintaining undifferentiated state and containing a TGFβ family signaling pathway inhibitor and/or a sonic hedgehog signaling pathway agonist in the absence of feeder cells for 12 hours to 48 hours;
(B) forming a cell aggregate by suspension-culturing the cells obtained in the step (A) in a culture medium containing a Wnt signaling pathway inhibitor and/or a sonic hedgehog signaling pathway agonist for 12 hours to 72 days (24 hours to 48 hours);
(C) further suspension-culturing the cell aggregate obtained in the step (B) in a culture medium containing a BMP signaling pathway agonist for 8 days to 15 days (10 days to 13 days);
(D) culturing the cell aggregate obtained in the step (C) in a serum-free medium or a serum medium containing a Wnt signaling pathway agonist and/or a FGF signaling pathway inhibitor for 2 days to 4 days; and
(E) culturing the cell aggregate obtained in the step (D) in a serum-free medium or a serum medium containing neither a Wnt signaling pathway agonist nor a FGF signaling pathway inhibitor and optionally containing a thyroid hormone signaling pathway agonist for about 10 days to about 200 days.

In this context, the step (E) may comprise the step of performing culture in a basal medium for cell proliferation for 10 days to 30 days, subsequently performing culture in a mixture of a basal medium for cell proliferation and a culture medium for continuous epithelial tissue maintenance containing a thyroid hormone signaling pathway agonist for 10 days to 40 days, and further performing culture in a culture medium for continuous epithelial tissue maintenance containing a thyroid hormone signaling pathway agonist for 20 days to 140 days.

In an embodiment, the step (E) comprises performing culture in the presence of a thyroid hormone signaling pathway agonist for 20 days to 60 days (30 days to 50 days).

In an embodiment, the culture period from the step (B) to the step (E) is from 70 days to 100 days (from 80 days to 90 days).

The cell aggregate containing a neural retina can be produced by the method mentioned above, though not limited thereto. In an embodiment, the cell aggregate containing a neural retina can also be obtained as a mixture of cell aggregates. In another embodiment, for example, one cell aggregate may be produced per well of a 96-well plate, and cell aggregates containing a neural retina may be obtained one by one. In any of the cases, cell aggregates produced under the same conditions are regarded as cell aggregates of the same lot. The cell aggregates of the same lot can be set to an arbitrary range by those skilled in the art. For example, cell aggregates contained in the mixture of cell aggregates mentioned above, or cell aggregates contained in the same cell culture container (e.g., 96-well plate) may be set as the cell aggregates of the same lot. As another example, a range using materials such as the same stem cells or culture media prepared at the same time may be set as the cell aggregates of the same lot. The cell aggregates of the same lot have diversity as to the composition, purity, or morphology of cells. On the other hand, in the case of evaluating only predetermined tissues (e.g., neural retina) from the cell aggregates of the same lot, equivalent gene expression profiles are usually exhibited.

### (Cell aggregate containing neural retina)

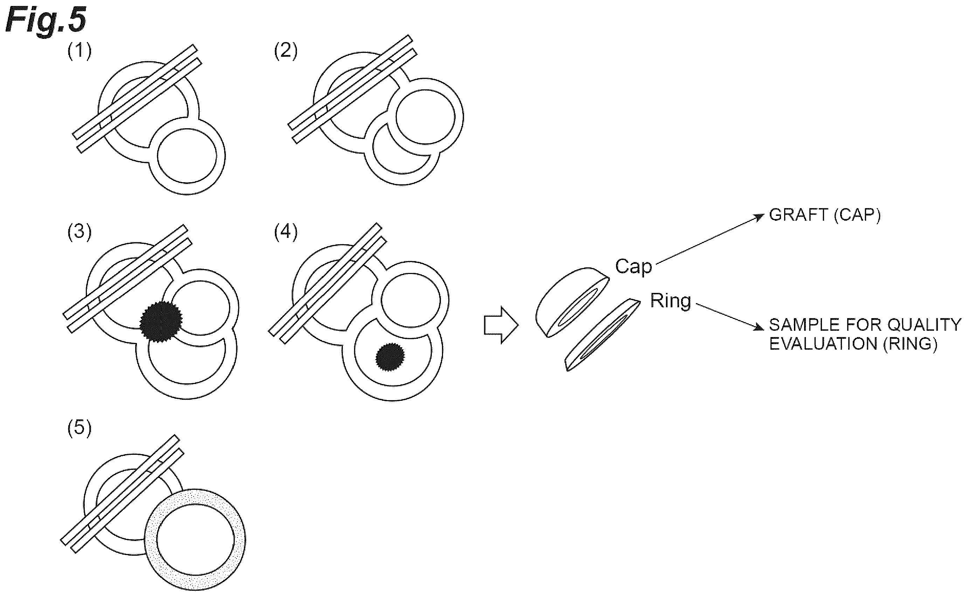

The cell aggregate containing a neural retina can contain the neural retina, and the structure of the cell aggregate is not limited. In an embodiment, the cell aggregate containing a neural retina is a sphere-like cell aggregate. In an embodiment, in the cell aggregate containing a neural retina, a plurality of neural retinas may be present with an overlap (e.g., see conceptual views (1) and (2) in Figure 5). In an embodiment, the cell aggregate containing a neural retina contains first epithelial tissue (target epithelial tissue) containing the transplant neural retina, and second epithelial tissue (non-target epithelial tissue) having the continuity of the slope of a tangent line to a surface different from the continuity of the slope of a tangent line to the surface of the first epithelial tissue, and containing a non-neural retina-related cell. In this context, the first epithelial tissue refers to epithelial tissue that does not substantially contain a non-neural retina-related cell (non-target cell) and allows the transplant neural retina to be dissected. On the other hand, the second epithelial tissue is epithelial tissue that may contain a neural retina, but is ineligible for dissecting the transplant neural retina because of containing non-target cells. In another embodiment, the cell aggregate containing a neural retina contains only the first epithelial tissue (target epithelial tissue) containing the transplant neural retina and does not contain non-target epithelial tissue.

The transplant neural retina is a human neural retina suitable for transplantation in humans and preferably consists of only the neural retina. The transplant neural retina contains at least a photoreceptor layer. The photoreceptor layer is formed at least in the outmost of the cell aggregate. Also, photoreceptor cells or photoreceptor precursor cells may be present in the inside. Alternatively, the photoreceptor layer may be formed in the inside. Photoreceptor cells, etc. are present continuously, i.e., by mutual adhesion, in the tangent direction of the surface of the cell aggregate. The photoreceptor cells, etc. are present continuously in the tangent direction of the surface of the cell aggregate, thereby forming a photoreceptor layer containing the photoreceptor cells, etc. The tangent direction refers to a direction tangent to the surface of the cell aggregate, i.e., a direction along which the photoreceptor cells, etc. in the photoreceptor layer are arranged, and is the direction in parallel to the neural retina or the lateral direction. The slope of a tangent line to the surface of epithelial tissue refers to a direction along which cells are arranged when individual cells in the epithelial tissue are arranged in a predetermined direction, and refers to the direction in parallel to the epithelial tissue (or epithelial sheet) or the lateral direction.

The second epithelial tissue contained in the cell aggregate is epithelial tissue containing epithelial tissue other than the neural retina, i.e., non-target epithelial tissue. Examples of the second epithelial tissue include eyeball-related tissue and brain and spinal cord tissue. The eyeball-related tissue means a non-retinal tissue surrounding eyeball tissue, and examples thereof include retinal pigment epithelial cells, ciliary body (e.g., ciliary marginal zone), and lens. The brain and spinal cord tissue means neural tissue of the brain and the spinal cord, and examples thereof include the forebrain, the telencephalon, the cerebrum, the diencephalon, the hypothalamus, the midbrain, the hindbrain, the cerebellum, and the spinal cord. Cells and expressed genes contained in the second epithelial tissue are as mentioned later.

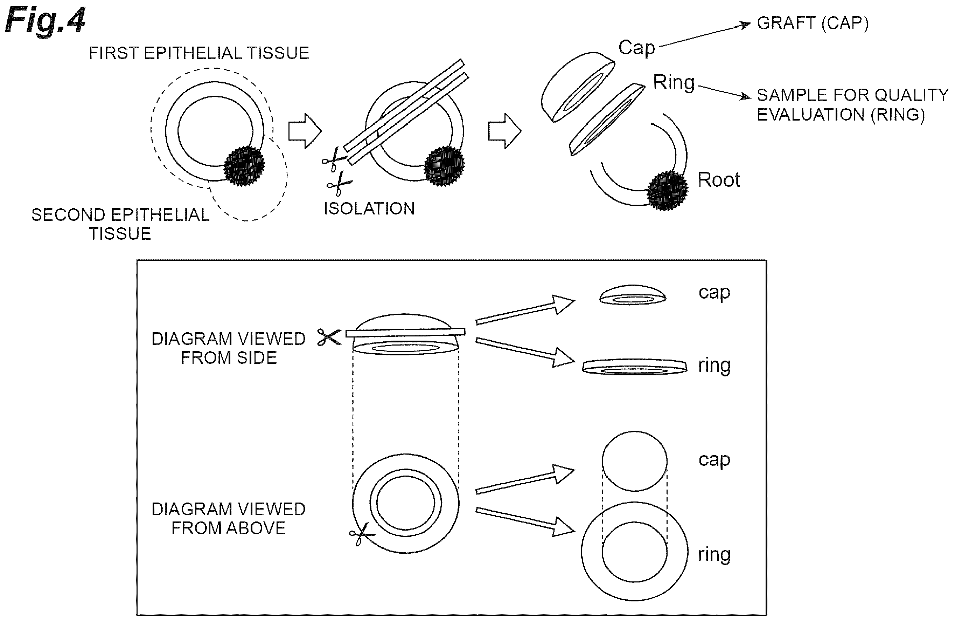

One example of the cell aggregate containing the first epithelial tissue and the second epithelial tissue includes cell aggregates shown in a conceptual view of Figure 4 and conceptual views (3) and (5) of Figure 5. The conceptual view of Figure 4 shows one example of a cell aggregate in which eyeball-related tissue (retinal pigment epithelial cells, ciliary body) (black portion of Figure 4) is present as the second epithelial tissue in a part of a neural retina which is the first epithelial tissue. The conceptual view (3) of Figure 5 shows one example of a cell aggregate in which eyeball-related tissue (retinal pigment epithelial cells, ciliary body) (black portion of Figure 5(3)) is further present as the second epithelial tissue when a plurality of neural retinas are present with an overlap (e.g., conceptual views (1) and (2) of Figure 5). The conceptual view (5) of Figure 5 shows one example of a cell aggregate in which brain and spinal cord tissue (cerebrum, etc.) (gray portion of Figure 5(5)) is present as the second epithelial tissue. As shown in the conceptual view (4) of Figure 5, non-target tissue may be contained inside the cell aggregate containing a transplant neural retina. This case does not apply to the definition "having the continuity of the slope of a tangent line to a surface different from the continuity of the slope of a tangent line to the surface of the first epithelial tissue", and therefore does not apply to the second epithelial tissue. It is preferable that the transplant neural retina and the sample for quality evaluation should be selected from a cell aggregate that does not contain non-target tissue in the inside.

### <Sampling step>

The method for evaluating the quality of a transplant neural retina according to the present invention comprises sampling a part or the whole of a cell aggregate containing a neural retina having an epithelial structure derived from a pluripotent stem cell as a sample for quality evaluation (hereinafter, referred to as " sampling step"). The sampling of a part of the cell aggregate as the sample for quality evaluation means selecting some (one or more) cell aggregates, or all cell aggregates from among a plurality of cell aggregates, and isolating (e.g., dissecting) a portion of the selected cell aggregates as the sample for evaluation using tweezers, scissors and/or a knife, etc. The sampling of the whole of the cell aggregate as the sample for quality evaluation means selecting some (one or more) cell aggregates from among a plurality of cell aggregates, and separately picking up the whole of the selected one or more cell aggregates as the sample for quality evaluation. In the case of selecting one or more cell aggregates from among a plurality of cell aggregate, random sampling is preferable. In the specification, the cell aggregate in the case of sampling a part of the cell aggregate as the sample for quality evaluation is referred to as "cell aggregate containing the sample for quality evaluation", and the cell aggregate in the case of sampling the whole of the cell aggregate as the sample for quality evaluation is referred to as "cell aggregate of the sample for quality evaluation".

### (Sampling of whole of cell aggregate)

In an embodiment, the sample for quality evaluation is the whole of a cell aggregate containing a neural retina having an epithelial structure derived from a pluripotent stem cell. In order to perform the quality evaluation of cell aggregates containing a neural retina in the same lot, the sample for quality evaluation may be the whole of one or more cell aggregates among the cell aggregates of the same lot. In this context, a mixture of cell aggregates of the same lot contains, for example, 2 or more and 10000 or less cell aggregates.

Specifically, in the quality evaluation of cell aggregates contained in the same lot, one or more cell aggregates are selected from the same lot, and the whole of the selected one or more cell aggregates is sampled as the sample for quality evaluation. In using the whole of the selected one or more cell aggregates as the sample for quality evaluation, all of a plurality of cell aggregates may be unified and sampled, or all of a plurality of cell aggregates may be separately sampled. The cell aggregate of the sample for quality evaluation used in the quality evaluation can no longer be used in transplantation.

As a result of performing determination using a quality evaluation sample, when the whole of the one or more cell aggregates as the sample for quality evaluation are determined as being applicable as the transplant neural retina, it can be determined that neural retinas in cell aggregates other than the sample for quality evaluation of the same lot produced under a condition exhibiting a gene expression profile equivalent to that of the transplant neural retina contained in the cell aggregates are applicable as the transplant neural retina. Epithelial tissue containing the neural retinas contained in these other cell aggregates can be used in transplantation (also referred to as evaluation within the same lot). Thus, this sampling method is useful for efficient quality evaluation.

As a result of performing determination using a quality evaluation sample, even if only one of the plurality of cell aggregates as the sample for quality evaluation is determined as being inapplicable as the transplant neural retina, it is determined that neural retinas in the other cell aggregates of the same lot are inapplicable as the transplant neural retina. Thus, for example, when the number of samples in the same lot is large and the possibility of being contaminated with ineligible samples is low, it is usually preferable to use this method (evaluation within the same lot). However, since the whole of the cell aggregate is sampled as the sample for evaluation, it is preferable that the method should not be used for a lot containing cell aggregates containing the second epithelial tissue. In this case, the sampling and evaluation of a part (neural retina) of the cell aggregate mentioned later, not the whole of the cell aggregate, is more suitable. Also, total evaluation mentioned later rather than the evaluation within the same lot is preferably used for a lot containing cell aggregates containing the second epithelial tissue.

### (Sampling of part of cell aggregate)

In an embodiment, the sample for quality evaluation is a part of a cell aggregate containing a neural retina having an epithelial structure derived from a pluripotent stem cell. By sampling a part of the cell aggregate as the sample for quality evaluation, there is an advantage that a neural retina contained in the remaining portion can be used in transplantation without completely destroying the cell aggregate. Specifically, provided that the sample for quality evaluation which is a part of the cell aggregate is determined as being accepted by a determination step mentioned later, a neural retina having an epithelial structure in the cell aggregate containing the sample for quality evaluation is regarded as being applicable as the transplant neural retina and can be used in transplantation.

In an embodiment, this sampling method can be used for individually evaluating the quality of a cell aggregate containing a neural retina (also referred to as individual evaluation). In this case, there is also an advantage that all the cell aggregates containing a neural retina to be evaluated can be selected and subjected to quality evaluation (total evaluation). This evaluation method (individual evaluation/total evaluation) can individually evaluate the quality of transplant neural retinas and is therefore the most accurate quality evaluation method. On the other hand, if the number of samples is large, a great deal of labor and cost are required. The evaluation method is based on the precondition that: sites except for the transplant neural retina in one cell aggregate include a site that exhibits a gene expression profile equivalent to that of the neural retina (sample for quality evaluation); and this sample for quality evaluation can be dissected. Particularly, whether or not the precondition is satisfied by a cell aggregate containing the second epithelial tissue becomes a major issue. The present inventors have evaluated large amounts of samples and thereby found for the first time that: a cell aggregate containing a neural retina satisfies the precondition; and the evaluation method can be used.

For exploiting the advantage, it is preferable that a part of the cell aggregate to be sampled as the sample for quality evaluation should be a part that does not contain the transplant neural retina or a candidate of the transplant neural retina. It is also preferable that the sample for quality evaluation should be a part that exhibits a gene expression profile equivalent to that of the transplant neural retina or a candidate of the transplant neural retina. For the sample for quality evaluation exhibiting the gene expression profile equivalent to that of the transplant neural retina, it is preferable that the sample for quality evaluation should be adjacent or continuous at least partially to the transplant neural retina, and it is preferable to be contained in the same epithelial tissue as the transplant neural retina. Furthermore, it is preferable that the transplant neural retina should contain the center and/or its neighborhood of the same epithelial tissue as the most favorable (e.g., containing a neural retina, not containing a non-neural retina, and having a continuous epithelium structure) portion in the cell aggregate, and it is preferable to be the center and/or its neighborhood of the same epithelial tissue and to have a size described in [Transplant neural retina sheet] mentioned later. Thus, it is preferable that the sample for quality evaluation should not contain the center and/or its neighborhood of the same epithelial tissue. In this context, the same epithelial tissue means continuous epithelial tissue having the continuity of the slope of a tangent line to the surface of the epithelial tissue, and the same epithelial tissue is preferably continuous epithelial tissue. In this context, the center and/or its neighborhood of epithelial tissue (continuous epithelial tissue) is a site at which distances from both ends are equal on the surface of the same epithelial tissue, and those skilled in the art can make estimation by observation under a microscope. It is also preferable that the sample for quality evaluation should be a portion continuous or adjacent to a site that is used as the transplant neural retina (e.g., a site that contains the center and/or its neighborhood of one epithelial tissue and is used in transplantation), and should be a portion as narrow as possible within a range that permits quality evaluation. For example, the transplant neural retina is a site of the center and/or its neighborhood of the same epithelial tissue, and the sample for quality evaluation is a portion continuous or adjacent at least partially to the transplant neural retina in the same epithelial tissue.

In an embodiment of the individual evaluation and/or the total evaluation, when the cell aggregate containing a transplant neural retina contains first epithelial tissue containing the transplant neural retina, and non-target epithelial tissue (second epithelial tissue) having the continuity of the slope of a tangent line to a surface different from the continuity of the slope of a tangent line to the surface of the first epithelial tissue, and containing a non-target cell, it is preferable that the transplant neural retina should contain a region on the first epithelial tissue most distant from the non-target epithelial tissue. In this respect, the sample for quality evaluation may be a part present between the non-target epithelial tissue and the transplant neural retina. The region on the first epithelial tissue most distant from the non-target epithelial tissue is a region containing, for example, when a straight line is drawn from the center of the non-target epithelial tissue toward the outer periphery of the first epithelial tissue, a point at which the length is the largest on the outer periphery of the first epithelial tissue. Furthermore, it is preferable that the sample for quality evaluation should be a portion adjacent or continuous to the transplant neural retina (e.g., a site that contains the center and/or its neighborhood of one epithelial tissue and is used in transplantation) and should be a portion as narrow as possible within a range that permits quality evaluation. One example of a graft and the sample for quality evaluation is shown in the conceptual view of Figure 4.

In another embodiment, in order to perform the quality evaluation (evaluation within the same lot) of cell aggregates containing a neural retina in the same lot, a part of one or more cell aggregates among the cell aggregates of the same lot may be sampled as the sample for quality evaluation, instead of the whole of the one or more cell aggregates among the cell aggregates of the same lot. Specifically, one or more cell aggregates are selected from the cell aggregates of the same lot, and only a part dissected from the selected cell aggregates may be used as the sample for quality evaluation. Particularly, when the whole cannot be used in quality evaluation, as in a cell aggregate containing the second epithelial tissue, and when it is desired that the quality evaluation should be efficiently carried out, this sampling method is useful. In this case, it is preferable for the sample for quality evaluation to use the transplant neural retina. In this context, the transplant neural retina that is used as the sample for quality evaluation refers to a neural retina in a cell aggregate produced under a condition exhibiting a gene expression profile equivalent to that of the transplant retina. By using, in quality evaluation, a site supposed to be used as the transplant neural retina unless sampled as the sample for quality evaluation, it is possible to more accurately perform the quality evaluation of the other cell aggregates containing a transplant neural retina in the same lot.

In the cell aggregate, it can be determined that a site having a continuous epithelium structure where an outer neuroblastic layer and an inner neuroblastic layer appear to be divided as two layers is the neural retina. On the other hand, eyeball-related tissue as the second epithelial tissue, particularly, retinal pigment epithelial cells, assume black color visually or under a microscope and therefore, can readily be distinguished from the neural retina by those skilled in the art. Also, brain and spinal cord tissue as the second epithelial tissue, visually or under a microscope, cannot be confirmed to have a continuous epithelium structure, which is a morphological feature, on the surface of the cell aggregate, cannot be confirmed to have morphological features intrinsic to the neural retina, and/or appears to have a dull color, and thus, can readily be distinguished from the neural retina by those skilled in the art by focusing thereon. Thus, those skilled in the art can isolate the transplant neural retina and the sample for quality evaluation from the first epithelial tissue containing the neural retina even in a cell aggregate containing the second epithelial tissue.

As mentioned above, in an embodiment, the sample for quality evaluation is set and sampled depending on a predetermined positional relationship with the transplant neural retina or a candidate of the transplant neural retina. In other words, a region to be dissected as the sample for quality evaluation can be fixed by the setting of the transplant neural retina or its candidate. In this context, in an embodiment, the transplant neural retina (also referred to as a graft or a cap) and its candidate can be defined by the position in the cell aggregate mentioned above (e.g., being the center and/or its neighborhood of the epithelial tissue (continuous epithelial tissue), and in the case of having the second epithelial tissue, being a region on the first epithelial tissue most distant from the second epithelial tissue), and a size described in [Transplant neural retina sheet] mentioned later, etc. Thus, those skilled in the art can set a neural retina having these features as the transplant neural retina or its candidate.

In the case of sampling the transplant neural retina and the sample for quality evaluation from the same cell aggregate, the sample for quality evaluation (also referred to as a ring) can be set as a region continuous or adjacent at least partially to the transplant neural retina set as mentioned above, and a region as narrow as possible within a range that permits quality evaluation, by those skilled in the art. In the case of sampling a part of one or more cell aggregates among the cell aggregates of the same lot as the sample for quality evaluation, the sample for quality evaluation can be sampled as the transplant neural retina mentioned above or its candidate portion in the cell aggregates by those skilled in the art. In this case, a size to be dissected as the sample for quality evaluation may be a size described in [Transplant neural retina sheet] mentioned later, or may be smaller. Thus, the sample for quality evaluation can be set and sampled depending on the positional relationship with the transplant neural retina or its candidate and the size.

### <Detection step>

The method for evaluating the quality of a transplant neural retina according to the present invention comprises detecting the expression of a neural retina-related cell-related gene and a non-neural retina-related cell-related gene (non-target cell-related gene) in the sample for quality evaluation (detection step). It is preferable for the detection step to quantitatively detect the expression levels of the genes. The non-target cell-related gene comprises one or more genes selected from the group consisting of brain and spinal cord tissue marker gene and eyeball-related tissue marker gene.

### (Neural retina-related cell-related gene)

The neural retina-related cell-related gene (target cell-related gene) means a gene expressed by neural retina-related cells. As the neural retina-related cell-related gene, a gene highly expressed in photoreceptor cells (rod photoreceptor cell, cone photoreceptor cell), horizontal cells, amacrine cells, intermediate neuronal cells, retinal ganglion cells (ganglion cell), bipolar cells (rod bipolar cell, cone bipolar cell), Muller glial cells, or precursor cells of these cells, neural retinal precursor cells, or the like as compared with non-target cells is preferable. Examples of the neural retina-related cell-related gene include the neural retina-related cell markers described above, and RAX, Chx10, SIX3, SIX6, RCVRN, CRX, NRL and NESTIN are preferable. GenBank IDs of the neural retina-related cell markers are shown in Table 1 below.

**[Table 1]**

| Gene name | GenBank ID |
|---|---|
| RAX | NM_013435.2 |
| Chx10 | NM_182894.2 |
| SIX3 | NM_005413.4 |
| SIX6 | NM_007374.2 |
| RCVRN | NM_002903.2 |
| CRX | NM_000554.6 |
| NRL | NM_006177.4 |
| NESTIN | NM_006617.2 |

The neural retina-related cell-related gene is preferably the gene described in Table 1, though not limited thereto. Other examples of the neural retina-related cell-related gene include Rax2, Vsx1, Blimp1, RXRG, S-opsin, M/L-opsin, rhodopsin, Brn3, and L7.

### (Non-neural retina-related cell-related gene)

The method for the quality evaluation of a medicine to detect the non-target cells induced as by-products in the process of producing the cell aggregate containing a neural retina as a medicine raw material has not been known at all. In the course of conducting diligent studies on a method for continuously producing a neural retina having quality that satisfies standards as medicine starting materials, the present inventors have found cells or tissue that might be produced as by-products, efficiently and effectively identified them, and found a non-neural retina-related cell-related gene (non-target cell-related gene) as a gene that can be used for carrying out the quality evaluation of a neural retina.

In an embodiment, examples of the non-neural retina-related cell-related gene (non-target cell-related gene) include brain and spinal cord tissue marker gene and eyeball-related tissue marker gene. In an embodiment, as the non-neural retina-related cell-related gene, undifferentiated iPS cell marker gene may be contained.

In an embodiment, the brain and spinal cord tissue marker gene may be one or more genes selected from the group consisting of telencephalon marker gene, diencephalon/midbrain marker gene and spinal cord marker gene. The diencephalon/midbrain marker gene may be one or more genes selected from the group consisting of diencephalon marker gene, midbrain marker gene, and hypothalamus marker gene regarding the hypothalamus which is a part of the diencephalon.

In an embodiment, the eyeball-related tissue marker gene may be one or more genes selected from the group consisting of optic stalk marker gene, ciliary body marker gene, lens marker gene and retinal pigment epithelium marker gene.

The telencephalon marker gene means a gene expressed in the telencephalon. The telencephalon marker gene may comprise one or more genes selected from the group consisting of FoxG1 (also called Bf1), Emx2, Dlx2, Dlx1 and Dlx5. GenBank IDs of the telencephalon marker genes are shown in Table 2 below.

**[Table 2]**

| Gene name | GenBank ID |
|---|---|
| FOXG1 | NM_005249.4 |
| Emx2 | NM_004098.4 |
| | NM_001165924.1 |
| DLX2 | NM_004405.4 |
| DLX1 | NM_178120.5 |
| | NM_001038493.1 |
| DLX5 | NM_005221.6 |
| | XM_005250185.3 |
| | XM_017011803.1 |

The telencephalon marker gene is preferably the gene described in Table 2, though not limited thereto. Other examples of the telencephalon marker gene include Emx1, LHX2, LHX6, LHX7, and Gsh2.

The diencephalon/midbrain marker gene means a gene expressed in the diencephalon and/or the midbrain. The diencephalon/midbrain marker gene may comprise one or more genes selected from the group consisting of OTX1, OTX2 and DMBX1. GenBank IDs of the diencephalon/midbrain marker genes are shown in Table 3 below. The diencephalon/midbrain marker gene may comprise a hypothalamus marker mentioned later regarding the hypothalamus which is a region of the diencephalon. In other words, the diencephalon/midbrain marker gene may comprise one or more genes selected from the group consisting of OTX1, OTX2, OTX2, DMBX1, Rx, Nkx2.1, OTP, FGFR2, EFNA5 and GAD1.

**[Table 3]**

| Gene name | GenBank ID |
|---|---|
| OTX1 | NM_001199770.1 |
| | NM_014562.4 |
| OTX2 | NM_001270523.1 |
| | NM_001270524.1 |
| | NM_001270525.1 |
| | NM_021728.3 |
| | NM_172337.2 |
| DMBX1 | NM_172225.1 |
| | NM_147192.2 |
| | XM_011540668.2 |
| | XM_017000289.1 |

The hypothalamus marker gene means a gene expressed in the hypothalamus. The hypothalamus marker gene may comprise one or more genes selected from the group consisting of Rx, Nkx2.1, Dmbx1, OTP, gad1, FGFR2 and EFNA5. GenBank IDs of the hypothalamus marker gene are shown in Table 4 below.

**[Table 4]**

| Gene name | GenBank ID |
|---|---|
| Rx | NM_013435.2 |
| Nkx2.1 | NM_003317.3, NM_001079668.2 |
| OTP | NM_032109.2 |
| gad1 | NM_000817.3, NM_013445.3 |
| | XM_005246444.3, XM_011510922.1 |
| | XM_017003756.1, XM_017003758.2 |
| | XM_017003757.2, XM_024452783.1 |
| FGFR2 | NM_022970.3, NM_000141.4 |
| | NM_023029.2, NM_001144913.1 |
| | NM_001144914.1, NM_001144915.1 |
| | NM_001144916.1, NM_001144917.1 |
| | NM_001144918.1, NM_001144919.1 |
| | NM_001320654.1, NM_001320658.1 |
| | NR_073009.1, XM_006717708.3 |
| | XM_006717710.4, XM_017015920.2 |
| | XM_017015921.2, XM_017015924.2 |
| | XM_017015925.2, XM_024447888.1 |
| | XM_024447887.1, XM_024447890.1 |
| | XM_024447889.1, XM_024447892.1 |
| | XM_024447891.1 |
| EFNA5 | NM_001962.3, XM_006714565.3 |
| | XM_011543250.3, XM_011543251.2 |
| | XM_017009205.1 |

The spinal cord marker gene means a gene expressed in the spinal cord. The spinal cord marker gene may comprise one or more genes selected from the group consisting of HoxB2, HoxA5, HOXC5, HOXD1, HOXD3 and HOXD4. GenBank IDs of the spinal cord marker gene are shown in Table 5 below.

**[Table 5]**

| Gene name | GenBank ID |
|---|---|
| HOXB2 | NM_002145.3 |
| | XM_005257275.4 |
| HOXA5 | NM_019102.4 |
| HOXC5 | NM_018953.3 |
| | NR_003084.2 |
| HOXD1 | NM_024501.3 |
| HOXD3 | NM_006898.4 |
| | XM_005246509.4 |
| | XM_005246511.4 |
| | XM_005246513.5 |
| | XM_011511065.3 |
| | XM_011511066.3 |
| HOXD4 | NM_014621.3 |
| | XM_005246514.4 |

The spinal cord marker gene is preferably the gene described in Table 5, though not limited thereto. Other examples of the spinal cord marker gene include a gene group forming the Hox cluster.

Meanwhile, in an embodiment, in the case of using retinoic acid in a production step, the expression of HOX gene (e.g., HOXC5, HOXA5 and HOXB2) may be found even if a good product of retinal tissue is produced. It is considered that the expression of the HOX gene is regulated by retinoic acid signals, and the HOX gene expression increases to an extent that does not influence differentiation into retinal tissue. This effect of the retinoic acid signals is considered to be ascribable to the promotion of posterior shift along the anteroposterior axis. Thus, in the case of using retinoic acid in a production step (particularly, in the case of using retinoic acid at the time or later when differentiation into the retina has started), the HOX gene (e.g., HOXC5, HOXA5 and HOXB2) can be excluded from subject genes of quality evaluation, or the quality of a transplant neural retina can be determined as being good even if the expression of these genes is found.

The optic stalk marker gene means a gene expressed in the optic stalk. The optic stalk marker gene may comprise one or more genes selected from the group consisting of GREM1, GPR17, ACVR1C, CDH6, Pax2, Pax8, GAD2 and SEMA5A. GenBank IDs of the optic stalk marker genes are shown in Table 6 below.

**[Table 6]**

| Gene name | GenBank ID |
|---|---|
| GREM1 | NM_013372.7, NM_001191322.1 |
| | NM_001191323.1, NM_001368719.1 |
| | XM_017022077.1 |
| GPR17 | NM_001161417.1, NM_005291.2 |
| | NM_001161415.1, NM_001161416.1 |
| | XM_017003833.2 |
| ACVR1C | NM_145259.3, NM_001111031.1 |
| | NM_001111032.1, NM_001111033.1 |
| CDH6 | NM_004932.4, NM_001362435.1 |
| | XM_011513921.3, XM_017008910.2 |
| | XR_001741972.2 |
| Pax2 | NM_000278.4, NM_003987.4 |
| | NM_003988.4, NM_003989.4 |
| | NM_003990.4, NM_001304569.1 |
| Pax8 | NM_003466.4, NM_013952.3 |
| | NM_013953.3, NM_013992.3 |
| GAD2 | NM_001134366.2, NM_000818.2 |
| SEMA5A | NM_003966.3, XM_006714506.3 |
| | XM_006714507.3, XM_011514155.2 |
| | XM_011514156.2, XM_011514157.2 |
| | XM_011514158.2, XM_011514159.2 |
| | XM_017010016.2 |

The lens marker gene means a gene expressed in the lens. The lens marker gene may comprise one or more genes selected from the group consisting of CRYAA and CRYBA1. GenBank IDs of the lens marker genes are shown in Table 7 below.

**[Table 7]**

| Gene name | GenBank ID |
|---|---|
| CRYAA | NM_000394.3 |
| | NM_001363766.1 |
| CRYBA1 | NM_005208.4 |
| | XM_017024198.1 |

The ciliary body marker gene means a gene expressed in the ciliary body and/or the ciliary marginal zone. The ciliary body marker gene may comprise one or more genes selected from the group consisting of Zic1, MAL, HNF1beta, FoxQ1, CLDN2, CLDN1, GPR177, AQP1 and AQP4. GenBank IDs of the ciliary body marker genes are shown in Table 8 below.

**[Table 8]**

| Gene name | GenBank ID |
|---|---|
| Zic1 | NM_003412.4 |
| MAL | NM_002371.4, NM_022438.2 |
| | NM_022439.2, NM_022440.2 |
| HNF1beta | NM_000458.4, NM_001165923.3 |
| | NM_001304286.1, XM_011525160.1 |
| | XM_011525161.1, XM_011525162.2 |
| | XM_011525163.2, XM_011525164.1 |
| FoxQ1 | NM_033260.4 |
| CLDN2 | NM_001171092.1, NM_020384.3 |
| | NM_001171095.1 |
| CLDN1 | NM_021101.5 |
| GPR177 | NM_024911.7, NM_001002292.3 |
| | NM_001193334.1, XM_011542191.2 |
| | XM_011542192.3, XM_017002390.2 |
| AQP1 | NM_198098.3, NM_001329872.1 |
| AQP4 | NM_001650.7, NM_004028.4 |
| | NM_001317384.2, NM_001317387.2 |
| | NM_001364286.1, NM_001364287.1 |
| | NM_001364289.1, XM_011525942.3 |

The retinal pigment epithelium marker gene means a gene expressed in retinal pigment epithelial cells. Examples of the retinal pigment epithelium marker gene include the retinal pigment epithelium markers described above, and may comprise one or more genes selected from the group consisting of MITF, TTR and BEST1. GenBank IDs of the retinal pigment epithelium marker genes are shown in Table 9 below.

**[Table 9]**

| Gene name | GenBank ID |
|---|---|
| MITF | NM_000248.3, NM_006722.2 |
| | NM_198158.2, NM_198159.2 |
| | NM_198177.2, NM_198178.2 |
| | NM_001184967.1, NM_001184968.1 |
| | NM_001354604.1, NM_001354605.1 |
| | NM_001354606.1, NM_001354607.1 |
| | NM_001354608.1 |
| | NM_004183.4, NM_001139443.1 |
| | NM_001300786.1, NM_001300787.1 |
| | NM_001363591.1, NM_001363592.1 |
| | NM_001363593.1, NR_134580.1 |
| BEST1 | XM_005274210.4, XM_005274215.4 |
| | XM_005274216.4, XM_005274219.4 |
| | XM_005274221.4, XM_011545229.3 |
| | XM_011545230.3, XM_011545233.3 |
| | XM_017018230.2, XR_001747952.2 |
| | XR_001747953.2, XR_001747954.2 |
| TTR | NM_000371.3 |

In an embodiment, the non-target cell-related gene may further comprise undifferentiated pluripotent stem cell marker gene.

The undifferentiated pluripotent stem cell marker gene may comprise one or more genes selected from the group consisting of Oct3/4, Nanog and lin28. Preferably, the undifferentiated pluripotent stem cell marker gene is one or more genes selected from the group consisting of Oct3/4, Nanog and lin28. GenBank IDs of the undifferentiated pluripotent stem cell marker genes are shown in Table 10 below.

**[Table 10]**

| Gene name | GenBank ID |
|---|---|
| Oct3/4 (POU5F1) | NM_002701.6, NM_203289.5 |
| | NM_001173531.2, NM_001285986.1 |
| | NM_001285987.1 |
| Nanog | NM_024865.4, NM_001297698.1 |
| lin28 | NM_024674.6, XM_011542148.2 |
| SOX2 | NM_003106.4 |
| KLF4 | NM_001314052.1, NM_004235.6 |
| c-Myc | NM_001354870.1, NM_002467.6 |
| Glis1 | NM_001367484.1, NM_147193.2 |
| | XM_017000409.1, XM_017000411.1 |
| | XM_017000408.1, XM_017000410.1 |
| | XM_017000412.1 |
| Sall4 | NM_001318031.1, NM_020436.5 |
| | XM_011528921.2, XM_011528922.2 |
| | XM_005260467.4 |
| Esrrb | NM_004452.3, XM_011536553.2 |
| | XM_024449508.1, XM_011536547.2 |
| | XM_011536554.2, XM_011536550.2 |
| | XM_024449509.1, XM_017021085.1 |

### (Detection approach)

In an embodiment, although the detection of the expression of the neural retina-related cell-related gene and the non-neural retina-related non-target cell-related gene is not particularly limited, examples thereof include approaches such as Western blotting, immunostaining, flow cytometry analysis/flow cytometers (FACS(R) manufactured by Becton, Dickinson and Company), etc.), Northern blotting, electrophoresis, PCR (preferably, quantitative PCR (qPCR) and/or real-time PCR), gene chip analysis, and next-generation sequencers. Among them, quantitative PCR is useful from the viewpoint of quantitativeness, detection sensitivity, stability of results and rapidness. Furthermore, by applying an apparatus that is used for performing single-cell quantitative PCR (e.g., Biomark HD (manufactured by Fluidigm Corp.)) to usual quantitative PCR, it is possible to evaluate a plurality of samples for quality evaluation in a short time. Particularly, in the case of carrying out total evaluation, particularly, when the number of samples to be used in quality evaluation and the number of genes to be evaluated are large, rapid quality evaluation is possible by using this approach.

In an embodiment, the respective expression levels of the neural retina-related cell-related gene and the non-neural retina-related cell-related gene in two or more samples for quality evaluation may be simultaneously detected by quantitative PCR. The quantitative PCR may be performed by, for example, a method comprising the following steps (1) to (5):
(1) providing a flow channel plate having one sample well group consisting of 8 or more and 800 or less independent sample wells, one or more primer well groups consisting of 8 or more and 800 or less independent primer wells, and flow channels connecting the independent sample wells in the sample well group with the independent primer wells in each primer well group, solutions containing nucleic acids obtained from the two or more of the samples for quality evaluation (sample solutions), and a solution containing one or a plurality of primers specific for each of one or more of the neural retina-related cell-related genes or the non-neural retina-related cell-related genes (primer solution);
(2) adding the sample solutions at one sample solution/one sample well for each of the samples for quality evaluation to the sample well group;
(3) adding the primer solution to one or more primer wells in the one or more primer well groups so as to be different primer well groups;
(4) separately mixing the primers with the nucleic acids via the flow channels; and
(5) performing quantitative PCR using the mixture obtained in (4).

Solutions containing nucleic acids obtained from the two or more samples for quality evaluation can be prepared from the RNA extracted from the samples for quality evaluation through the reverse transcription reaction using reverse transcriptase and primers. The RNA extraction and the reverse transcription reaction can be appropriately carried out using approaches known to those skilled in the art. Also, those skilled in the art can provide primers that can amplify the genes according to the genes to be quantified.

In an embodiment, the solutions containing nucleic acids (sample solutions) may be solutions subjected to multiplex-PCR reaction (Pre-Run) using all the primers used in a PCR apparatus before being added to each well of the sample well group. By Pre-Run, it is possible to amplify the nucleic acids to some extent and to effectively carry out quantitative PCR. The conditions of Pre-Run involve, for example, subjecting the solutions containing nucleic acids to about 10 cycles to 15 cycles of PCR reaction using all the primers used.

In an embodiment, in the quantitative PCR, a flow channel plate **having** flow channels connecting the different well groups mentioned above may be used. On the flow channel plate, the flow channels (e.g., integrated fluidic circuit), a plurality of wells for adding the solutions containing nucleic acids, and a plurality of wells for adding the primers are present, and each well may be connected to one or more flow channels (integrated fluidic circuit). The solutions containing nucleic acids and primers are added at one solution per well. In an embodiment, air pressure is applied to the wells using a known apparatus (e.g., IFC Controller HX, IFC Controller MX, IFC Controller RX, all manufactured by Fluidigm Corp.) so that the solutions containing nucleic acids and the primers added to the individual wells can be injected into the flow channels (integrated fluidic circuit). The flow channels (integrated fluidic circuit) may have, for example, a structure where the sample solution and the primer solution are mixed at 1:1. By this, mixtures of sample solutions and primer solutions can be prepared at once depending on the number of combinations of the sample solutions and the primer solutions (e.g., in the case of using 96 each of sample solutions and primer solutions, the number of combinations is 96 × 96 = 9216). After preparation of the mixtures, the respective expression levels of the genes in the solutions containing individual nucleic acids can be simultaneously measured by subjecting the flow channel plate to quantitative PCR reaction in a quantitative PCR apparatus (e.g., Biomark HD).

Flow cytometry analysis using a flow cytometer capable of detecting the ratios of expressing cells is also useful. In recent years, improvement in detection rate has advanced, and a flow cytometer capable of evaluating multiple samples with high-throughput properties (FACS(R), etc.) is also available. Thus, for the detection of the expression of the neural retina-related cell-related gene and the non-neural retina-related non-target cell-related gene, use of a high-throughput flow cytometer is also useful. It is possible for such a high-throughput flow cytometer to use a commercially available product (e.g., MACSQuant(R) Analyzers: manufactured by Miltenyi Biotec).

### <Determination step>

The method according to the present invention comprises, when the expression of the neural retina-related cell-related gene is found and the expression of the non-neural retina-related cell-related gene is not found, determining that epithelial tissue (transplant neural retina) containing the neural retina in the cell aggregate containing the sample for quality evaluation being the part, and epithelial tissue (transplant neural retina)containing the neural retina in a cell aggregate of the same lot as the cell aggregate containing the sample for quality evaluation being the part, or the cell aggregate of the sample for quality evaluation being the whole, is applicable as the transplant neural retina (determination step). In this context, being applicable as the transplant neural retina means being a neural retina suitable for transplantation, and is also referred to as having an aptitude for the transplant neural retina, or being accepted as the transplant neural retina.

"The expression of the neural retina-related cell-related gene is found" means that, for a detection method for gene expression, the expression of the neural retina-related cell-related gene at a level substantially detectable by the detection method (e.g., detection lower limit value or more) is found. Also, "the expression of the non-neural retina-related cell-related gene is not found" means that, for a detection method for gene expression, the expression of the non-neural retina-related cell-related gene cannot be substantially detected by the detection method (e.g., less than detection lower limit value). The substantial detectability means that the gene is detected beyond an extent that cannot regard the gene as substantially functioning. Those skilled in the art are appropriately capable of setting it according to the genes and the detection method. For example, in the case of a detection method for gene expression with quantitativeness, it can be determined that the range of more than 0% to 10% or less or more than 0% to 5% or less with reference to the detection lower limit value of the gene expression is not the substantially detectable level (i.e., the expression of the gene cannot be detected).

In an embodiment, it is preferable to determine being applicable as the transplant neural retina when the following reference 1 and reference 2 are satisfied in the quantitative PCR:
reference 1: the difference between the threshold cycle (Ct) value of the neural retina-related cell-related gene and the Ct value of an internal standard gene (ΔCt value) is 10 or less, and
reference 2: the difference between the Ct value of the non-neural retina-related cell-related gene and the Ct value of the internal standard gene (ΔCt value) is 5 or more.

The threshold cycle (Ct) value means the number of cycles that reaches a predetermined amount of an amplification product in a region where the amplification of a gene by PCR occurs exponentially. The Ct value has an inverse correlation with the initial amount of the gene and as such, is used in the calculation of the initial copy number of the gene. In an embodiment, the "2^Ct value (Ct value power of 2)" is inversely proportional to the initial amount of the gene and as such, is used in the calculation of the initial copy number of the gene. Specifically, a sample containing a 2-fold initial amount of a gene has a Ct value more rapid by one cycle than that of a sample containing the gene at only half the copy number before amplification. The predetermined amount of an amplification product can fall within the region where the amplification of a gene by PCR occurs exponentially, and can be set by those skilled in the art.

The internal standard gene means a gene whose difference in expression level is small among samples. As the internal standard gene, the one known to those skilled in the art can be appropriately used, and examples thereof include 18S ribosomal RNA, β actin, HPRT, α tubulin, transferrin receptor, ubiquitin, and GAPDH, with GAPDH being preferable.

The Ct value is inversely correlated with the initial amount of a gene and therefore depends on the expression level of the gene within cells. Specifically, when the concentration of a nucleic acid-containing solution is constant, the Ct value differs depending on the internal standard gene used and the difference between the Ct value of a predetermined gene and the Ct value of the internal standard gene (ΔCt value) is influenced by the internal standard gene used. In the specification, the ΔCt value is described with reference to a value with GAPDH used as the internal standard gene, unless otherwise specified.

In the case of using an internal standard gene other than GAPDH as the internal standard gene, the ΔCt values of the reference 1 and the reference 2 can be corrected by comparing the expression levels of GAPDH and the internal standard gene other than GAPDH.

In an embodiment, in the case of using β actin as the internal standard gene, the Ct value of GAPDH is lower by about 1 than the Ct value of β actin, i.e., the absolute amount of GAPDH RNA is about twice the absolute amount of β actin RNA, as to GAPDH and β actin in the production method of the present application. Therefore,
reference 1: the difference between the threshold cycle (Ct) value of the neural retina-related cell-related gene and the Ct value of an internal standard gene (ΔCt value) is 9 or less, and
reference 2: the difference between the Ct value of the non-neural retina-related cell-related gene and the Ct value of the internal standard gene (ΔCt value) is 4 or more.
can hold.

In an embodiment, in the case of using HPRT as the internal standard gene, the Ct value of GAPDH is lower by about 7 than that of HPRT, i.e., the absolute amount of GAPDH RNA is about 2^7 (7th power of 2, 128 times) of the absolute amount of HPRT RNA, as to GAPDH and HPRT in the production method of the present application. Therefore,
reference 1: the difference between the threshold cycle (Ct) value of the neural retina-related cell-related gene and the Ct value of an internal standard gene (ΔCt value) is 3 or less, and
reference 2: the difference between the Ct value of the non-neural retina-related cell-related gene and the Ct value of the internal standard gene (ΔCt value) is -2 or more.
can hold.

The neural retina-related cell-related gene can be the gene mentioned above. As the neural retina-related cell-related gene, a plurality of genes are present. Specifically, even in the case of sampling it from the same neural retina-related cells, the Ct value of the reference 1 may differ depending on the type of the neural retina-related cell-related gene. Those skilled in the art can set a ΔCt value from which the expression of the neural retina-related cell-related gene can be determined on a gene basis, from known information such as the expression site or expression level of the neural retina-related cell-related gene.

For example, as for the Chx10 gene, when GAPDH is used as the internal standard, the ΔCt value may be 20 or less, preferably 15 or less, more preferably 10 or less.

For example, as for the recoverin gene, when GAPDH is used as the internal standard, the ΔCt value may be 16 or less, preferably 11 or less, more preferably 6 or less.

In general, the difference between the Ct value of the neural retina-related cell-related gene and the Ct value of the internal standard gene (e.g., GAPDH) (ΔCt value) may be, for example, 25 or less, 20 or less, 15 or less or 10 or less. The difference between the Ct value of the neural retina-related cell-related gene and the Ct value of the internal standard gene may be, for example, -10 or more, -5 or more, 0 or more or 5 or more.

The non-neural retina-related cell-related gene can be the gene mentioned above. As the non-neural retina-related cell-related gene, a plurality of genes are present. Specifically, even in the case of sampling it from the same non-retinal cells, the Ct value differs depending on the non-neural retina-related cell-related gene. Those skilled in the art can set a ΔCt value from which the expression of the non-neural retina-related cell-related gene can be determined on a gene basis, from known information such as the expression site or expression level of the non-neural retina-related cell-related gene. For example, as for the PAX2 gene, when GAPDH is used as the internal standard, the ΔCt value may be 5 or more. As for the HOXB2 gene, when GAPDH is used as the internal standard, the ΔCt value may be 5 or more. In general, the difference between the Ct value of the non-neural retina-related cell-related gene and the Ct value of the internal standard gene may be 30 or less, 25 or less or 20 or less. Also, the difference between the Ct value of the non-neural retina-related cell-related gene and the Ct value of the internal standard gene may be, for example, 0 or more, 3 or more or 5 or more.

The quality evaluation method mentioned above can be used as a quality control method for medicines (transplant neural retina) or a quality control approach in a production step of medicines (transplant neural retina).

### [Transplant neural retina sheet]

One aspect of the present invention is a transplant neural retina sheet
(1) being derived from a pluripotent stem cell,
(2) having a three-dimensional structure,
(3) comprising a neural retinal layer having a plurality of layer structures including a photoreceptor layer and an inner layer,
(4) the photoreceptor layer comprising one or more cells selected from the group consisting of a photoreceptor precursor cell and a photoreceptor cell,
(5) the inner layer comprising one or more cells selected from the group consisting of a retinal precursor cell, a ganglion cell, an amacrine cell and a bipolar cell,
(6) the surface of the neural retinal layer having an apical surface,
(7) the inner layer being present inside the photoreceptor layer present along the apical surface,
(8) the area of the neural retinal layer being 50% or more with respect to the total area of the surface of the transplant neural retina sheet,
(9) the area of a continuous epithelium structure being 80% or more with respect to the total area of the apical surface of the neural retinal layer, and
(10) the expression of neural retina-related cell-related gene being found and the expression of non-neural retina-related cell-related gene being not found in the transplant neural retina sheet, and the non-neural retina-related cell-related gene comprising one or more genes selected from the group consisting of brain and spinal cord tissue marker gene and eyeball-related tissue marker gene.

In an embodiment, the transplant neural retina sheet is a transplant neural retina dissected by the quality evaluation method mentioned above. Thus, features of the transplant neural retina sheet mentioned later also correspond to features of the transplant neural retina dissected by the quality evaluation method mentioned above.

The transplant neural retina sheet (3) comprises a neural retinal layer having a plurality of layer structures including a photoreceptor layer and an inner layer. As described in (6) and (7), although the photoreceptor layer is present outside (surface) the transplant neural retina sheet, an ectopic photoreceptor layer may be present in the inner layer.

In the transplant neural retina sheet, (5) the inner layer comprises one or more cells selected from the group consisting of a retinal precursor cell, a ganglion cell, an amacrine cell and a bipolar cell, but may comprise one or more cells selected from the group consisting of an ectopic photoreceptor precursor cell and photoreceptor cell. In an embodiment, a transplant neural retina sheet in which the content of a ganglion cell, an amacrine cell and a horizontal cell is 30% or less of the total number of cells, a transplant neural retina sheet in which the content of a ganglion cell, an amacrine cell, a horizontal cell and a bipolar cell is 30% or less of the total number of cells, and/or a transplant neural retina sheet in which the content of a bipolar cell is 10% or less of the total number of cells is also provided.

In the transplant neural retina sheet, (8) the area of the neural retinal layer is 40% or more, preferably 50% or more, more preferably 60% or more, with respect to the total area of the surface of the transplant neural retina sheet. In the transplant neural retina sheet, (9) the area of a continuous epithelium structure is 60% or more, preferably 70% or more, more preferably 80% or more, with respect to the total area of the apical surface of the neural retinal layer.

The neural retina-related cell-related gene and the non-neural retina-related cell-related gene (brain and spinal cord tissue marker gene and eyeball-related tissue marker gene) are the genes mentioned above.

(10) The expression of neural retina-related cell-related gene being found and the expression of non-neural retina-related cell-related gene being not found in the transplant neural retina sheet can be revealed by isolating a part of the transplant neural retina sheet, and detecting the expression of the genes. For a transplant neural retina sheet isolated from a cell aggregate in which the expression of neural retina-related cell-related gene is substantially found and the expression of non-neural retina-related cell-related gene is not substantially found in a sample for quality evaluation by the method for evaluating the quality of a transplant neural retina mentioned above, the detection of gene expression in the transplant neural retina sheet itself is unnecessary. The expression of the gene being substantially found or the expression being not found is determined, as mentioned above, by the detection method for gene expression, depending on whether or not to be a level substantially detectable by the detection method.

The neural retina-related cell-related gene in the transplant neural retina sheet may be, for example, one or more selected from the group consisting of Rx, Chx10, Pax6 and Crx. The ratio of cells expressing the neural retina-related cell-related gene (positive cell) to the total number of cells differs depending on the stage of differentiation into the neural retina.

In an embodiment, the ratio of a Rx-positive cell to the total number of cells in the transplant neural retina sheet may be 30% or more, 40% or more, 50% or more, or 60% or more. In an embodiment, the ratio of a Chx10-positive cell or a Pax6-positive cell to the total number of cells in the transplant neural retina sheet may be 10% or more, 20% or more, 30% or more, 40% or more, or 50% or more. In an embodiment, the ratio of a Crx-positive cell to the total number of cells in the transplant neural retina sheet may be 10% or more, 20% or more, 30% or more, 40% or more, or 50% or more.

In an embodiment, the ratio of the Rx-positive cell to the total number of cells in the transplant neural retina sheet may be 30% or more and 80% or less, 40% or more and 70% or less, 45% or more and 60% or less, or 50% or more and 60% or less. In an embodiment, the ratio of the Chx10-positive cell or the Pax6-positive cell to the total number of cells in the transplant neural retina sheet may be 10% or more and 80% or less, 20% or more and 70% or less, 30% or more and 60% or less, or 40% or more and 50% or less. In an embodiment, the ratio of the Crx-positive cell to the total number of cells in the transplant neural retina sheet is 10% or more and 70% or less, 10% or more and 60% or less, 20% or more and 60% or less, 30% or more and 60% or less, 40% or more and 60% or less, or 50% or more and 60% or less.

In an embodiment, (1) the ratio of a Chx10-positive and Pax6-positive cell (neural retinal precursor cell) may be 10% or more and 50% or less or 10% or more and 30% or less, (2) the ratio of a Chx10-positive and Pax6-negative cell (precursor cell biased toward a bipolar cell) may be 10% or more and 25% or less or 15% or more and 25% or less, and (3) the ratio of a Chx10-negative and Pax6-positive cell (ganglion cell and amacrine cell) may be 10% or more and 25% or less or 10% or more and 20% or less, to the total number of cells in the transplant neural retina sheet.

In another embodiment, (1) the ratio of the Chx10-positive and Pax6-positive cell (neural retinal precursor cell) may be 20% or more and 40% or less, (2) the ratio of the Chx10-positive and Pax6-negative cell (precursor cell biased toward a bipolar cell) may be 5% or more and 20% or less, and (3) the ratio of the Chx10-negative and Pax6-positive cell (ganglion cell and amacrine cell) may be 5% or more and 20% or less or 5% or more and 15% or less, to the total number of cells in the transplant neural retina sheet.

In an embodiment, the transplant neural retina sheet according to the present invention is a transplant neural retina determined as being applicable as the transplant neural retina by the method for evaluating the quality of a transplant neural retina, and may be an isolated sheet-shaped transplant neural retina.

In an embodiment, the transplant neural retina sheet according to the present invention has been isolated from a cell aggregate containing a neural retina, and may be a transplant neural retina sheet containing a region of the center and/or its neighborhood of continuous epithelial tissue in the cell aggregate.

In an embodiment, the transplant neural retina sheet according to the present invention has been isolated from a cell aggregate containing at least first epithelial tissue and second epithelial tissue. In the cell aggregate, the first epithelial tissue contains a human neural retina, and the second epithelial tissue has the continuity of the slope of a tangent line to a surface different from the continuity of the slope of a tangent line to the surface of the first epithelial tissue, and contains a non-neural retina-related cell. The transplant neural retina sheet may be a transplant neural retina sheet containing a region on the first epithelial tissue most distant from the second epithelial tissue. In this context, the second epithelial tissue may be a tissue selected from the group consisting of eyeball-related tissue, brain and spinal cord tissue and a tissue different from the neural retina of the first epithelial tissue.

In an embodiment, the transplant neural retina sheet according to the present invention may contain a region of the center and/or its neighborhood of continuous epithelial tissue in the cell aggregate.

The transplant neural retina sheet can be isolated, as mentioned above, from a cell aggregate containing a neural retina. Also, it can be obtained by a method for producing a transplant neural retina sheet mentioned later.

In an embodiment, the major axis of the transplant neural retina sheet according to the present invention may be, for example, from 300 µm to 3300 µm and is preferably from 600 µm to 2500 µm, more preferably from 1100 µm to 1700 µm.

In an embodiment, the minor axis of the transplant neural retina sheet according to the present invention may be, for example, from 100 µm to 2000 µm and is preferably from 200 µm to 1500 µm, more preferably from 400 µm to 1100 µm.

In an embodiment, the height of the transplant neural retina sheet according to the present invention may be, for example, from 50 µm to 1500 µm and is preferably from 100 µm to 1000 µm, more preferably from 200 µm to 700 µm.

In an embodiment, the volume of the transplant neural retina sheet according to the present invention may be, for example, from 0.001 mm³ to 4.0 mm³ and is preferably from 0.01 mm³ to 1.5 mm³, more preferably from 0.07 mm³ to 0.57 mm³.

Methods for measuring the major axis, minor axis and height of the transplant neural retina sheet are not particularly limited, and they can be measured, for example, from an image taken under a microscope. For example, a front image taken with a cut surface turned to an objective lens side, and a side image taken with the cut surface inclined so as to be perpendicular to an objective lens are taken under a stereo microscope as to the transplant neural retina sheet dissected from a cell aggregate, and they can be measured from the taken images. In this context, the major axis means the longest line segment among line segments connecting two end points on the sheet cross section in the front image, and the length thereof. The minor axis means the longest line segment among line segments connecting two end points on the sheet cross section in the front image and orthogonal to the major axis, and the length thereof. The height means the longest line segment among line segments orthogonal to the sheet cross section and having a point intersecting the sheet cross section and the apex of the retina sheet as end points, and the length thereof. The volume of the sheet means a volume calculated according to the following calculation expression by approximating a graft as being an ellipsoid halved such that the cross section passes through the major axis. 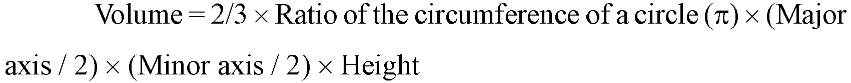 Volume = 2/3 × Ratio of the circumference of a circle (π) × (Major axis / 2) × (Minor axis / 2) × Height

### [Pharmaceutical composition, treatment method, therapeutic product and production method]

In an aspect of the present invention, a pharmaceutical composition comprising a transplant neural retina sheet is provided. The pharmaceutical composition comprises the transplant neural retina sheet of the present invention and preferably further comprises a pharmaceutically acceptable carrier. The pharmaceutical composition can be used in the treatment of a disease caused by the damage of a neural retina-related cell or a neural retina or the injury of a neural retina. Examples of the disease caused by the damage of a neural retina-related cell or a neural retina include ophthalmic diseases such as retinal degenerative diseases, macular degeneration, age-related macular degeneration, retinitis pigmentosa, glaucoma, corneal diseases, retinal detachment, central serous chorioretinopathy, cone dystrophy, and cone rod dystrophy. Examples of the injury state of a neural retina include a state in which photoreceptor cells die of degeneration.

As the pharmaceutically acceptable carrier, a physiological aqueous solvent (physiological saline, buffer, serum-free medium, etc.) can be used. If necessary, the pharmaceutical composition may be blended with a preservative, a stabilizer, a reducing agent, a tonicity agent, and the like which are usually used in a medicine containing tissues or cells to be transplanted in medical transplantation.

In an aspect of the present invention, a therapeutic product for a disease caused by the damage of a neural retina, comprising a transplant neural retina sheet obtainable in the present invention, is provided. In an aspect of the present invention, a method for treating a disease caused by the damage of a neural retina-related cell or a neural retina or the injury of a neural retina, comprising transplanting a transplant neural retina sheet obtainable in the present invention to a subject in need of transplantation (e.g., subretinally to an eye having the ophthalmic disease), is also provided. As the therapeutic product for a disease caused by the damage of a neural retina, or in order to make up for a corresponding injured site in the injury state of the neural retina, the transplant neural retina sheet of the present invention can be used. The disease caused by the damage of a neural retina-related cell or a neural retina, or the injury state of a neural retina can be treated by transplanting the transplant neural retina sheet of the present invention to a patient having the disease caused by the damage of a neural retina-related cell or a neural retina, or a patient with the injury state of a neural retina, in need of transplantation, and making up for the neural retina-related cell or the damaged neural retina. Examples of a transplantation method include a method of subretinally transplanting the transplant neural retina sheet to an injured site through an incision to an eyeball. Examples of a method for transplantation include a method of performing infusion using a thin tube, and a method of performing transplantation by sandwiching between tweezers, and examples of the thin tube include injection needles.

In an aspect of the present invention, a method for producing a transplant neural retina sheet obtainable in the present invention is provided. In one embodiment, the method for producing a transplant neural retina sheet comprises: evaluating a cell aggregate containing a neural retina having an epithelial structure derived from a pluripotent stem cell by use of the method for evaluating a transplant neural retina to determine that the neural retina is applicable as the transplant neural retina; and isolating the determined transplant neural retina.

In another embodiment, the method for producing a transplant neural retina sheet comprises: sampling a sample for quality evaluation from each of 2 or more and 800 or less cell aggregates containing a neural retina having an epithelial structure derived from a pluripotent stem cell, the sample for quality evaluation being a part of the cell aggregate; select a transplant neural retina determined as being applicable as the transplant neural retina by evaluating the sampled 2 or more and 800 or less samples for quality evaluation by use of the evaluation of a transplant neural retina; and isolating the selected transplant neural retina.

It is preferable that the cell aggregate should be a cell aggregate containing at least first epithelial tissue and second epithelial tissue, obtained by differentiating a pluripotent stem cell. It is preferable that the first epithelial tissue should contain a human neural retina, and the second epithelial tissue should have the continuity of the slope of a tangent line to a surface different from the continuity of the slope of a tangent line to the surface of the first epithelial tissue, and contain a non-neural retina-related cell. It is preferable that the isolation of the transplant neural retina should be the isolation of the transplant neural retina from the cell aggregate such that the transplant neural retina contains a region on the first epithelial tissue most distant from the second epithelial tissue. The isolation is performed through dissection by the approach mentioned above.

The quality evaluation method disclosed in the present invention using a cell aggregate containing epithelial tissue as a starting material, a part of the epithelial tissue as a sample for transplantation, and another part of the epithelial tissue as a sample for quality evaluation is particularly effective when individual cell aggregates differ in morphology/structure. It is also applicable to a cell aggregate containing various epithelial tissues.

### Examples

Hereinafter, the present invention will be described in detail with reference to Examples. However, the present invention is not limited by these by any means.

### <Example 1 Production of cell aggregate containing neural retina>

Human iPS cells (DSP-SQ strain, established by Sumitomo Dainippon Pharma Co., Ltd.) are those established by using commercially available Sendai virus vector (4 factors, i.e., Oct3/4, Sox2, KLF4, c-Myc, site tune kit manufactured by ID Pharma Co., Ltd.) based on the method described in the protocol open to public by Thermo Fisher Scientific Inc. (iPS 2.0 Sendai Reprogramming Kit, Publication Number MAN0009378, Revision 1.0) and protocol open to public (establishment/maintenance culture of feeder-free human iPS cells, CiRA_Ff-1PSC_protocol_JP_v140310, http://www.cira.kyoto-u.ac.jp/j/research/protocol.html) by Kyoto University, and using StemFit medium (AK03; manufactured by Ajinomoto Co., Inc.) and Laminin 511-E8 (manufactured by Nippi. Inc.).

The human iPS cells (DSP-SQ strain) were subjected to feeder-free culture in accordance with the method described in Scientific Reports, 4, 3594 (2014). As a feeder-free medium, StemFit medium (AK03N, manufactured by Ajinomoto Co., Inc.) was used, and as a feeder-free scaffold, Laminin511-E8 (manufactured by Nippi, Inc.) was used.

Specific operation of maintenance culture was as follows: First, human iPS cells (DSP-SQ strain) reached sub-confluency were washed with PBS and separated into single cells by use of TrypLE Select (manufactured by Life Technologies). Then, the separated human iPS single cells were seeded in plastic culture dishes coated with Laminin 511-E8 and cultured in feeder-free StemFit medium in the presence of Y27632 (ROCK inhibitor, 10 µM). When 6-well plates (for cell culture, culture area: 9.4 cm², manufactured by AGC TECHNO GLASS., LTD) were used as the plastic culture dishes, the number of separated human iPS single cells to be seeded was specified as 1.0×10⁴. One day after seeding, the medium was exchanged with StemFit medium not containing Y27632. Thereafter, the medium was exchanged with Y27632-free StemFit medium once every 1 to 2 days. Thereafter the cells were cultured until 5 days after seeding.

Operation of differentiation was carried out as follows: Human iPS cells (DSP-SQ strain) were cultured in feeder-free StemFit medium until 2 days before the cells reached sub-confluency (state where about 30% of the culture area is covered by cells). The human iPS cells the 2 days before the sub-confluency were subjected to feeder-free culture for 2 days (preconditioning treatment) in the presence of SAG (300 nM).

The preconditioned human iPS cells were treated for cell dispersions using TrypLE Select (manufactured by Life Technologies) and further separated into single cells by pipetting. Thereafter, the separated human iPS single cells were suspended in 100 µl of a serum-free medium such that the density of cells per well of a non-cell adhesive 96-well culture plate (PrimeSurface, 96 V-bottom plate, manufactured by Sumitomo Bakelite Co., Ltd.) was 1.3 × 10⁴ cells, and subjected to suspension culture in the conditions of 37°C and 5% CO₂. The serum-free medium (gfCDM + KSR) used herein is a serum-free medium prepared by adding 10% KSR and 450 µM 1-monothioglycerol and 1X Chemically defined lipid concentrate to a mixture of culture fluids containing F-12 medium and IMDM medium in a ratio of 1:1. At the initiation time of the suspension culture (Day 0 from initiation of the suspension culture), Y27632 (final concentration 20 µM) and SAG (final concentration 10 nM) were added to the serum-free medium. Day 2 from initiation of the suspension culture, 50 µl of a fresh serum-free medium (the same one as mentioned above), which did not contain Y27632 or SAG and contained human recombinant BMP4 (manufactured by R&D), was added such that the final concentration of exogenous human recombinant BMP4 became 1.5 nM (55 ng/ml).

Four days later (that is, Day 6 from initiation of the suspension culture), the medium was exchanged with the serum free medium, which did not contain Y27632, SAG or human recombinant BMP4. Operation of medium exchange was carried out as follows: 60 µl of the medium in the incubator was discarded, 90 µl of a fresh serum-free medium (the same one as mentioned above) was added. This operation was carried out to control the total medium volume to be 180 µl. Thereafter, a half of the medium was exchanged with serum-free medium, which did not contain Y27632, SAG or human recombinant BMP4, once every 2 to 4 days. The operation for exchanging a half volume of the medium was as follows. A half volume, i.e., 90 µl, of the medium in the incubator was discarded, 90 µl of a fresh serum-free medium (the same one as mentioned above) was added to control the total medium volume to be 180 µl.

The cell mass obtained on Day 13 from initiation of the suspension culture was cultured in a serum free medium (prepared by adding 1% N₂ supplement to DMEM/F12 medium) containing CHIR99021 (3 µM) and SU5402 (5 µM), for 3 days, i.e., up to Day 16 from initiation of the suspension culture.

The resultant cell aggregate on Day 16 from initiation of the suspension culture was cultured in each of the serum media shown in the following [1], [2] and [3] in the condition of 5% CO₂ up to Day 75 from initiation of the suspension culture.
[1] Day 16 to day 40 from initiation of the suspension culture:
   DMEM/F12 medium containing 10% fetal bovine serum, 1% N₂ supplement and 100 µM taurine (hereinafter referred to as medium A).
[2] Day 40 to day 60 from initiation of the suspension culture:
   Mixture of culture fluids containing medium A and a medium, which was Neurobasal medium containing 10% fetal bovine serum, 2% B27 supplement, 2 mM glutamine, 60 nM T3 and 100 µM taurine (hereinafter referred to as medium B) in a ratio of 1:3.
[3] On and after Day 60 from initiation of the suspension culture: medium B.

The cell mass on Day 75 from initiation of the suspension culture was observed under an inverted microscope to confirm morphology. It was found here that a neuroepithelial structure was formed.

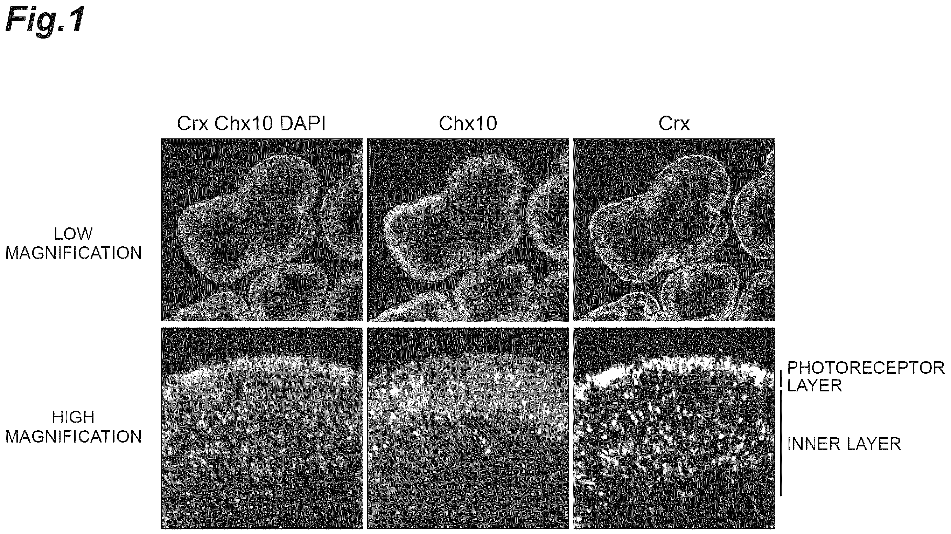

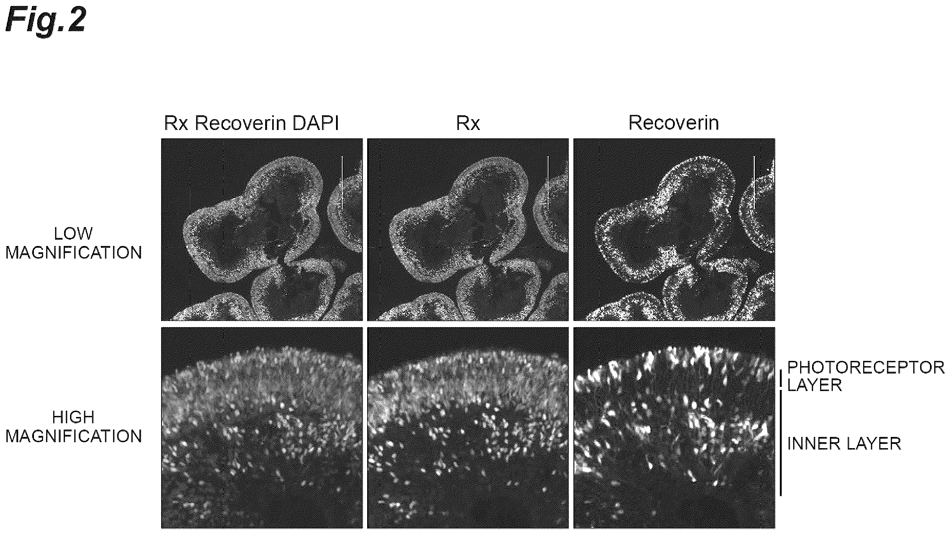

The cell mass on Day 75 from initiation of the suspension culture was fixed with 4% paraformaldehyde, frozen and sectioned. The frozen sections were subjected to immunostaining to stain a neural retina marker, Chx10 (anti-Chx10 antibody, Exalpha Biologicals, sheep) and a photoreceptor precursor cell marker Crx (anti-Crx antibody, Takara Bio Inc., rabbit) (Figure 1). Other frozen sections were subjected to immunostaining to stain a neural retina marker Rx (anti-Rx antibody, Takara Bio Inc., guinea pig) and a photoreceptor cell marker recoverin (anti-recoverin antibody, Proteintech Group, rabbit) (Figure 2). The nuclei of the cells were stained with DAPI.

These sections stained were observed using a fluorescence microscope (manufactured by Keyence Corp.) to obtain immunostained images. The photographs in which the produced cells were observed under a fluorescence microscope are shown in Figure 1 and Figure 2. The upper boxes of Figure 1 and Figure 2 are images taken with a low-magnification lens, and the lower boxes are images taken with a high-magnification lens.

From the DAPI-stained images of Figure 1 and Figure 2, it was found that neural tissue densely packed with cells was formed on the surface of the cell mass and this neural tissue formed a continuous epithelium structure. As a result of analyzing the image of Figure 1, it was found that in this neural tissue, a Crx-positive layer (photoreceptor layer) with a thickness on the order of 2 to 5 cells was formed on the surface of the cell mass, a Chx10-positive layer with a thickness on the order of 5 to 20 cells was formed inside the Crx-positive layer, and a layer in which Crx-positive cells were sparsely present was further formed inside it (Figure 1). It was found that the surface of this cell mass was morphologically an apical surface. Furthermore, as a result of analyzing the image of Figure 2, it was found that in this neural tissue, a recoverin-positive layer (photoreceptor layer) was formed and a Rx-positive layer was also formed. From these results, it was found that in this neural tissue, a photoreceptor layer containing Crx-positive cells and recoverin-positive cells was formed on the surface, a retinal precursor cell layer containing Chx10-positive cells was formed inside the photoreceptor layer, and a cell layer was also formed inside the retinal precursor cell. In short, it was found that by this production method, a neural retina containing a photoreceptor layer and a retinal precursor cell layer can be prepared from human iPS cells and this neural retina has a continuous epithelium structure.

### <Example 2 Identification of non-target cell (non-neural retina-related cell) and search for marker gene>

Human iPS cells (QHJI-01-s04 strain, obtained from Center for iPS Cell Research and Application, Kyoto University) were differentiated into a retina under various culture conditions. Specifically, human iPS cells subjected to maintenance culture by the method described in Example 1 were subjected to feeder-free culture until 2 days before the cells reached sub-confluency (state where about 30% of the culture area is covered by cells) or the day before the cells reached sub-confluency (state where about 50% of the culture area is covered by cells). The human iPS cells 2 days before the sub-confluency and the human iPS cells the day before the sub-confluency were subjected to feeder-free culture for 2 days in the presence of SAG (300 nM) and for one day in the presence of SAG(300 nM) and LDN(LDN193189, 100 nM), respectively (preconditioning treatment).

The preconditioned human iPS cells were subjected to suspension culture by the method described in Example 1. The serum-free medium (gfCDM + KSR) is a serum-free medium prepared by adding 10% or 5% KSR and 450 µM 1-monothioglycerol and 1X Chemically defined lipid concentrate to a mixture of culture fluids containing F-12 medium and IMDM medium in a ratio of 1:1. At the initiation time of the suspension culture (Day 0 from initiation of the suspension culture), Y27632 (final concentration 20 µM) and IWR-1e (final concentration 3 µM), or Y27632 (final concentration 20 µM), IWR-1e (final concentration 3 µM) and SAG (30 nM) were added to the serum-free medium. Day 3 from initiation of the suspension culture, 50 µl of a fresh serum-free medium (the same one as mentioned above), which did not contain Y27632 or SAG and contained human recombinant BMP4 (manufactured by R&D) and IWR-1e was added such that the final concentration of exogenous human recombinant BMP4 became 1.5 nM (55 ng/ml), and that the final concentration of IWR-1e became 3 µM.

Three days later (that is, Day 6 from initiation of the suspension culture), the medium was exchanged with the serum free medium, which did not contain Y27632, SAG or human recombinant BMP4 and contained IWR-1e. Operation of medium exchange was carried out as follows: 60 µl of the medium in the incubator was discarded, 90 µl of a fresh serum-free medium (the same one as mentioned above) was added. This operation was carried out to control the total medium volume to be 180 µl. Ten to twelve days after initiation of the suspension culture, 67% of the medium was exchanged with the serum-free medium, which did not contain IWR-1e, Y27632, SAG or human recombinant BMP4. This medium exchange operation was repeated twice, such that the concentration of exogenous IWR-1e became about 10% compared to that before medium exchange. Thereafter, a half of the medium was exchanged with serum-free medium, which did not contain IWR-1e, Y27632 or human recombinant BMP4, once every 2 to 4 days. The operation for exchanging a half volume of the medium was as follows. A half volume, i.e., 90 µl, of the medium in the incubator was discarded, 90 µl of a fresh serum-free medium (the same one as mentioned above) was added to control the total medium volume to be 180 µl.

The cell mass obtained on Days 19 to 20 from initiation of the suspension culture was cultured in a serum free medium (prepared by adding 1% N₂ supplement to DMEM/F12 medium) containing Wnt signaling pathway agonist, CHIR99021 (3 µM) and FGF signaling pathway inhibitor SU5402 (5 µM), for 3 days, i.e., up to Days 22 to 23 from initiation of the suspension culture.

Thereafter, the resultant was cultured using each of the serum media shown in the following [1], [2] and [3] described in Example 1, or a medium (hereinafter referred to as Retina medium) prepared by adding 10% fetal bovine serum, 1% N₂ supplement, 0.5 µM retinoic acid and 100 µM taurine to DMEM/F12 medium, in the condition of 5% CO₂ up to Days 89 to 97 from initiation of the suspension culture.

The cell aggregate obtained on Days 89 to 97 from initiation of the suspension culture was subjected to observation by an inverted microscope (ECLIPSE Ti, manufactured by Nikon Corp.) as a bright-field image (phase contrast image). The observation was performed, particularly, focusing on features of the morphology of individual cells and the mutual adhesion state between the cells. In the cell aggregate, a site having a continuous epithelium structure where an outer neuroblastic layer (containing photoreceptor layer and neural retinal precursor cell layer) and an inner neuroblastic layer appeared to be divided as two layers was determined as the neural retina. Also, a tissue in which a continuous epithelium structure was not found, and a site having a continuous epithelium structure where, however, an outer neuroblastic layer and an inner neuroblastic layer were not able to be distinguished from each other and appeared to be one layer, were determined as by-products (A, B, C, D, E and F). Thereafter, while observed under a stereo microscope, tissue pieces were prepared by dissecting the neural retina or the by-products from the cell aggregate under the stereo microscope using tapered tweezers and scissors. The dissected tissue pieces were 33 samples in total of "neural retinas #1 to 9", "by-products A, #10 to 16", "by-products B, #17 to 20", "by-products C, #21, #22", "by-products D, #23, #24", "by-products E, #25 to 29", and "by-products F, #30 to 33".

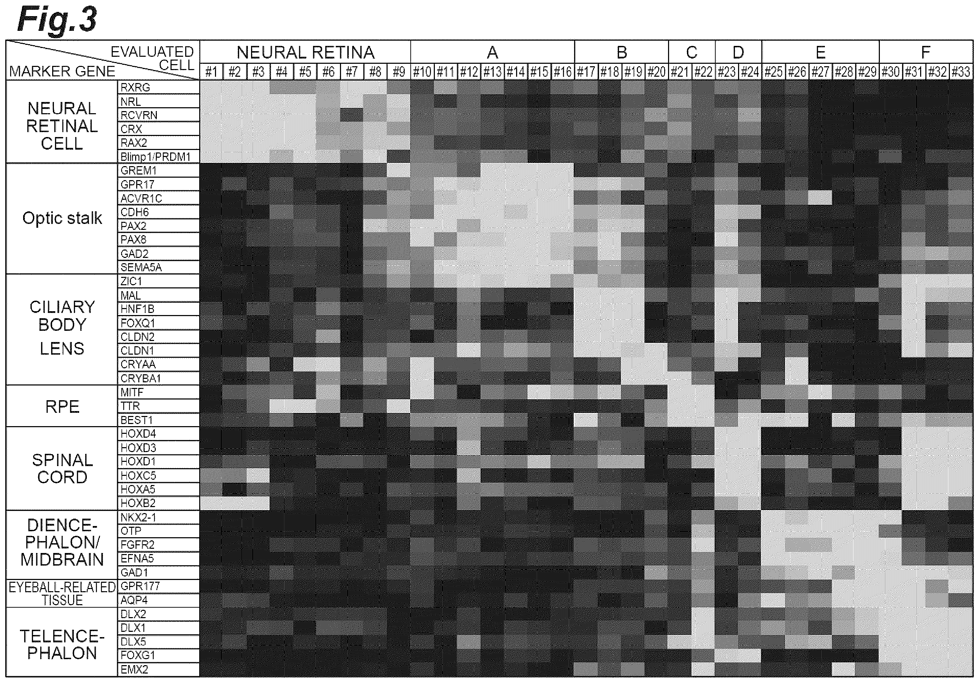

Thereafter, the tissue pieces were subjected to total RNA extraction with a spin column (manufactured by QIAGEN N.V., RNeasy Micro kit) by the method described in the kit, and analyzed in a microarray (manufactured by Affymetrix, Human Genome U133 Plus2.0) (Figure 3). Figure 3 is a heatmap view. The gray color corresponds to a high level of gene expression, and the black color corresponds to a low level of gene expression (a lighter color corresponds to a higher level of gene expression).

As a result, first, it was found that in 9 tissue pieces of the "neural retinas #1 to 9", the expression levels of the cone photoreceptor precursor cell marker RXRG, the rod photoreceptor precursor cell marker NRL, the photoreceptor cell marker recoverin (also called RCVRN), the photoreceptor precursor cell marker Crx, the neural retina marker Rax2 and the photoreceptor precursor cell marker Blimp1 (also called PRDM1) were generally high. In short, it was able to be confirmed that the "neural retinas #1 to 9" were retinal tissue containing neural retina-related cells.

The expression of the HOX gene (HOXC5, HOXA5 and HOXB2) was found in the "neural retinas #1 to 3". For the "neural retinas #1 to 3", retinoic acid was added in the production process, and the expression of the HOX gene is considered to be regulated by the retinoic acid. However, the "neural retinas #1 to 3" are good products of retinal tissue containing neural retina-related cells, as mentioned above. Thus, in the case of adding retinoic acid in a production step, the expression of the HOX gene (HOXC5, HOXA5 and HOXB2) is acceptable.

The "by-products A, #10 to 16" were analyzed. As a result, it was found that in the by-products A, the expression levels of the neural retina-related cell markers were generally low. On the other hand, as a result of searching for marker gene highly expressed in the by-products A, it was found that GREM1, GPR17, ACVR1C, CDH6, Pax2, Pax8, GAD2 and SEMA5A were expressed. As a result of scrutinizing literature information regarding the markers (Baumer N, et al., Development. 2003 Jul; 130 (13): 2903-15, Pfeffer PL, et al., Development. 1998 Aug; 125 (16): 3063-74), it was found that the by-products A were the optic stalk. In short, it was found that in the case of producing a neural retina from pluripotent stem cells *in vitro,* the optic stalk might be produced as by-products. It was further found that GREM1, GPR17, ACVR1C, CDH6, Pax2, Pax8, GAD2 and SEMA5A are useful as marker gene for discriminating this optic stalk.

The "by-products B, #17 to 20" were analyzed. As a result, it was found that in the by-products B, the expression levels of the neural retina-related cell markers were generally low. On the other hand, as a result of searching for marker gene highly expressed in the by-products B, it was found that Zic1, MAL, HNF1beta, FoxQ1, CLDN2, CLDN1, CRYAA and CRYBA1 were expressed. As a result of scrutinizing literature information regarding the markers, it was found that the by-products B were the ciliary body/lens/ciliary marginal zone (hereinafter, referred to as the ciliary body/lens). In short, it was found that in the case of producing a neural retina from pluripotent stem cells *in vitro,* the ciliary body/lens might be produced as by-products. It was further found that Zic1, MAL, HNF1beta, FoxQ1, CLDN2, CLDN1, CRYAA and CRYBA1 are useful as marker gene for discriminating this ciliary body/lens.

The "by-products C, #21, #22" were analyzed. As a result, it was found that in the by-products C, the expression levels of the neural retina-related cell markers were generally low. On the other hand, as a result of searching for marker gene highly expressed in the by-products C, it was found that MITF, TTR and BEST 1 were expressed. As a result of scrutinizing literature information regarding the markers, it was found that the by-products C were the retinal pigment epithelium (RPE). In short, it was found that in the case of producing a neural retina from pluripotent stem cells *in vitro,* RPE might be produced as by-products. It was further found that MITF, TTR and BEST1 are useful as marker gene for discriminating this RPE.

The "by-products D, #23, #24" were analyzed. As a result, it was found that in the by-products D, the expression levels of the neural retina-related cell markers were generally low. On the other hand, as a result of searching for marker gene highly expressed in the by-products D, it was found that HOXD4, HOXD3, HOXD1, HOXC5, HOXA5 and HOXB2 were expressed. As a result of scrutinizing literature information regarding the markers, it was found that the by-products D were spinal cord tissue. In short, it was found that in the case of producing a neural retina from pluripotent stem cells *in vitro,* spinal cord tissue might be produced as by-products. It was further found that HOXD4, HOXD3, HOXD1, HOXC5, HOXA5 and HOXB2 are useful as marker gene for discriminating this spinal cord tissue.

The "by-products E, #25 to 29" were analyzed. As a result, it was found that in the by-products E, the expression levels of the neural retina-related cell markers were generally low. On the other hand, as a result of searching for marker gene highly expressed in the by-products E, it was found that Nkx2.1, OTP, FGFR2, EFNA5 and GAD1 were expressed. As a result of scrutinizing literature information regarding the markers, it was found that the by-products E were the diencephalon/midbrain/hypothalamus (hereinafter, referred to as the diencephalon/midbrain). In short, it was found that in the case of producing a neural retina from pluripotent stem cells *in vitro,* the diencephalon/midbrain might be produced as by-products. It was further found that Nkx2.1, OTP, FGFR2, EFNA5 and GAD1 are useful as marker gene for discriminating this diencephalon/midbrain.

The "by-products F, #30 to 33" were analyzed. As a result, it was found that in the by-products F, the expression levels of the neural retina-related cell markers were generally low. On the other hand, as a result of searching for marker gene highly expressed in the by-products F, it was found that DLX2, DLX1, DLX5, FOXG1, EMX2, GPR177 (Wls), and AQP4 were expressed. As a result of scrutinizing literature information regarding the markers, it was found that the by-products F were the telencephalon because DLX2, DLX1, DLX5, FOXG1 and EMX2 were expressed. It was found that GPR177 and AQP4 are also useful as by-product markers. In short, it was found that in the case of producing a neural retina from pluripotent stem cells *in vitro,* the telencephalon might be produced as by-products. It was further found that DLX2, DLX1, DLX5, FOXG1 and EMX2 are useful as marker gene for discriminating this telencephalon.

From the results described above, it was found that in the by-products, the expression level of the neural retina-related cell marker gene was low and the expression levels of genes known to be expressed in the tissues of the optic stalk, the ciliary body/lens, RPE, the spinal cord, the diencephalon/midbrain and the telencephalon were high. Thus, it was found that the optic stalk, the ciliary body/lens, RPE, the spinal cord, the diencephalon/midbrain and the telencephalon may be produced as by-products in the process of differentiation into a three-dimensional retina. It was also found that markers that permit detection of each tissue are the genes shown in Table 11.

**[Table 11]**

| Tissue | Gene name |
|---|---|
| Retina | RXRG, NRL, Recoverin, Crx, Rax2, Blimp1 |
| Optic Stalk | GREM1, GPR17, ACVR1C, CDH6, Pax2, Pax8, GAD2, SEMA5A |
| Ciliary body, lens | Zic1, MAL, HNF1beta, FoxQ1, CLDN2, CLDN1, CRYAA, CRYBA1, GPR177, AQP4 |
| RPE | MITF, TTR, BEST1 |
| Spinal cord | HOXD4, HOXD3, HOXD1, HOXC5, HOXA5, HOXB2 |
| Hypothalamus | Nkx2.1, OTP, FGFR2, EFNA5, GAD1 |
| Telencephalon | DLX2, DLX1, DLX5, FOXG1, EMX2 |

### <Example 3 Design of method for evaluating graft>

A three-dimensional retina prepared from human iPS cells consists of a neural retina having a neuroepithelial structure with the continuity of the composition or distribution of cells. This neural retina having the neuroepithelial structure has a layer structure constituted by a photoreceptor layer and an inner layer and has a characteristic appearance and morphology (Figure 1).

Individual human three-dimensional retinas differ in shape with a size on the order of 1 to 2 mm. Although a neural retina that is used in transplantation is a main product owing to the characteristics of a production method using self-organization culture, it was found that eyeball-related tissue (RPE, ciliary body, etc.) and brain and spinal cord tissue (telencephalon, spinal cord, etc.) which are non-neural retinas are produced as by-products (Example 2). Accordingly, the central part of a neural retina that did not contain a non-neural retina was dissected to obtain a retina piece (graft, cap) (Figure 4 and Figure 5). Although it is considered desirable for quality evaluation regarding the composition or purity of retina pieces to carry out a total test, the destructive test of the total number of retina pieces cannot be conducted. Accordingly, a neighboring part of the retina piece (cap) was used as a sample for quality evaluation (ring). The total number of rings was analyzed (preferably by quantitative PCR), and study was made to use only a Cap corresponding to a Ring adapted for references as a transplant neural retina.

Figure 4 and Figure 5 are conceptual views of typical cell aggregates. A site at which the neuroepithelial structure (preferably continuous epithelium structure) intrinsic to the neural retina where a photoreceptor layer and an inner layer appeared to be divided as two layers was found, was regarded as a graft (cap). A site that is a neighboring site of the Cap and exhibits a neuroepithelial structure (preferably continuous epithelium structure) similar to that of the Cap was regarded as a sample for quality evaluation (ring). A site other than the Cap and the Ring was referred to as a root.

Hereinbelow, the usefulness of an approach of isolating a Cap and a Ring from a neuroepithelial structure contained in one cell aggregate was studied.

### <Example 4 Shape of graft (cap)>

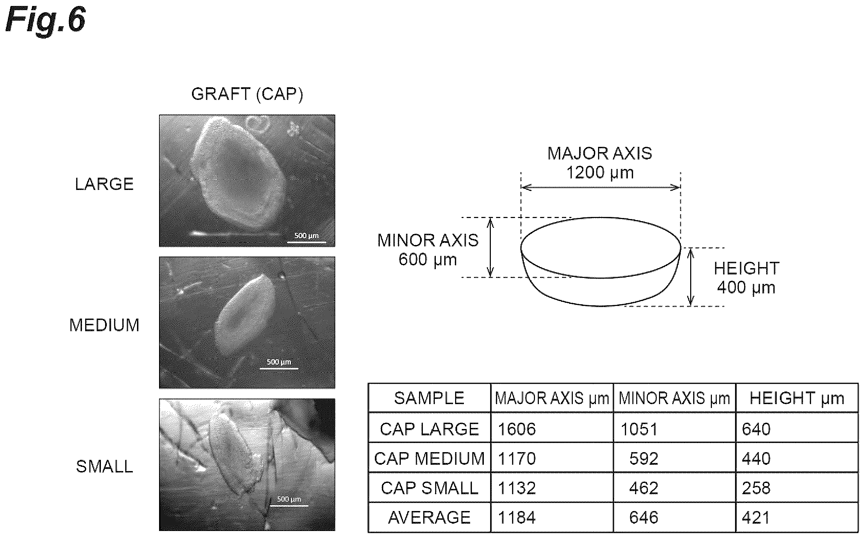

Grafts (caps) were prepared by the following method (Figure 6). First, a bright-field image (phase contrast image) of a cell aggregate on Day 99 from initiation of suspension culture prepared from human iPS cells (DSP-SQ strain) in accordance with the method described in Example 1 was taken under an inverted microscope (manufactured by Olympus Corp.). After confirming that a neural retina was present on the cell aggregate, the cell aggregate was transferred to under a stereo microscope, and various sizes of the neural retina were dissected as grafts by use of the method described in Example 2. Also, study was made on the influence of a graft size on the operation of transplantation with a device for transplantation.

A front image taken with a cut surface turned to an objective lens side, and a side image taken with the cut surface inclined so as to be perpendicular to an objective lens were taken under a stereo microscope as to the dissected grafts. Thereafter, the major axes, minor axes, and heights of the grafts were measured from the taken images.
For the measurement, the major axis was defined as the longest line segment among line segments connecting two end points on the retina sheet cross section in the front image, and the length thereof. The minor axis was defined as the longest line segment among line segments connecting two end points on the retina sheet cross section in the front image and orthogonal to the major axis, and the length thereof. The height was defined as the longest line segment among line segments orthogonal to the retina sheet cross section in the side image and having a point intersecting the retina sheet cross section and the surface of the retina sheet as end points, and the length thereof. The volume of the graft was calculated according to the following calculation expression by approximating the graft as being an ellipsoid halved such that the cross section passed through the major axis. 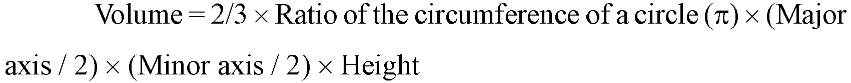 Volume = 2/3 × Ratio of the circumference of a circle (π) × (Major axis / 2) × (Minor axis / 2) × Height

As a result, it was found that the loading of a graft in a device for transplantation, the stability of the graft in the device for transplantation and the discharge of the graft from the device for transplantation were influenced by the size of the graft. It was also suggested that, particularly, the minor axis was a useful parameter. The major axis, the minor axis, the height and the volume were calculated as to each of 11 grafts for which the operation of transplantation with the device for transplantation was favorable. Results of determining an average value, the maximum value and the minimum value as to each parameter were summarized in Table 12. From this result, it was found that the graft (cap) was at least from 0.8 to 1.7 mm in major axis, from 0.4 to 1.1 mm in minor axis, from 0.2 to 0.7 mm in height, from about 0.07 to about 0.57 mm³ in apparent volume.

**[Table 12]**

| | Major axis [mm] | Minor axis [mm] | Height [mm] | Volume [mm³] |
|---|---|---|---|---|
| Average value | 1.184 | 0.646 | 0.421 | 0.184 |
| Maximum value | 1.606 | 1.051 | 0.640 | 0.565 |
| Minimum value | 0.870 | 0.462 | 0.258 | 0.070 |

### <Example 5 Composition of cell in graft (cap)>

Grafts (caps) were prepared by the following method (Nos: 18001MF, d89, H5). First, grafts (caps) were isolated by the methods described in Examples 2 and 3 from a cell aggregate on Day 89 from initiation of suspension culture prepared from human iPS cells (DSP-SQ strain) in accordance with the method described in Example 1.

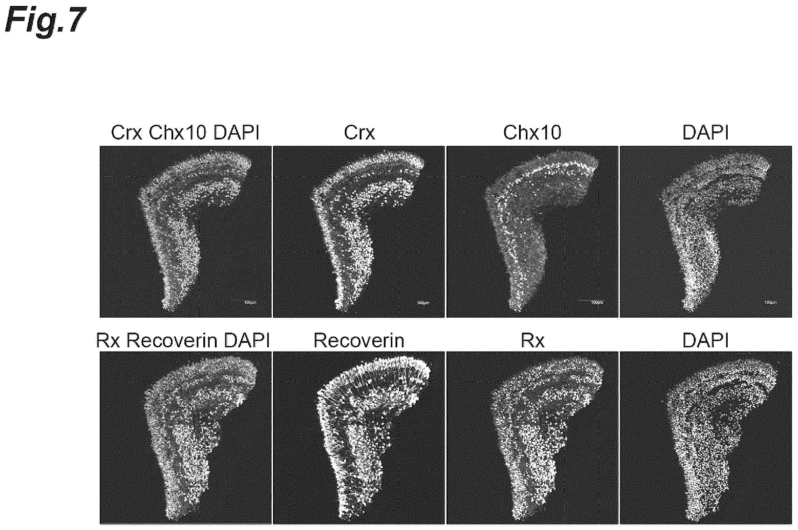

The graft was fixed with 4% paraformaldehyde, frozen and sectioned. The frozen sections were subjected to immunostaining to stain a neural retina marker, Chx10 (anti-Chx10 antibody, Exalpha Biologicals, sheep) and a photoreceptor precursor cell marker Crx (anti-Crx antibody, Takara Bio Inc., rabbit) (Figure 7). Other frozen sections were subjected to immunostaining to stain a neural retina marker Rx (anti-Rx antibody, Takara Bio Inc., guinea pig) and a photoreceptor cell marker recoverin (anti-recoverin antibody, Proteintech Group, rabbit) (Figure 7). The nuclei of the cells were stained with DAPI. These sections stained were observed using a confocal laser microscope (manufactured by Olympus Corp.) to obtain immunostained images.

From the stained images, it was found that neural tissue densely packed with cells was formed on the surface (left side in the drawing) of the graft (cap) and this neural tissue formed a neuroepithelial structure (particularly, continuous epithelium structure) (Figure 7). It was further found that in this neural tissue, a Crx-positive layer (photoreceptor layer, Figure 7) with a thickness on the order of 2 to 10 cells was formed on the surface of the cell mass, a Chx10-positive layer with a thickness on the order of 5 to 20 cells was formed inside the Crx-positive layer, and a layer in which Crx-positive cells were present was further formed inside it (Figure 7). It was found that the surface of this graft (cap) was morphologically an apical surface. Furthermore, it was found that in this neural tissue, a recoverin-positive layer (photoreceptor layer, Figure 7, arrow) was formed and a Rx-positive layer was also formed. From these results, it was found that in this neural tissue, a photoreceptor layer containing Crx-positive cells and recoverin-positive cells was formed on the surface, a retinal precursor cell layer containing Chx10-positive cells was formed inside the photoreceptor layer, and a cell layer was also formed inside the retinal precursor cell. In short, it was found that the graft (cap) can prepare a neural retina containing a photoreceptor layer and a retinal precursor cell layer and this neural retina has a continuous epithelium structure.

### <Example 6 Verification of equivalent state between Cap and ring>

In order to compare gene expression between a Cap and a ring, a test was conducted by the following method. First, a cell aggregate on Day 99 from initiation of suspension culture was prepared from human iPS cells (DSP-SQ strain) in accordance with the method described in Example 1, and used as lot 1. Further, a cell aggregate on Day 82 from initiation of suspension culture was prepared from human iPS cells (DSP-SQ strain) in accordance with the method described in Example 1, and used as lot 2. The main product neural retina and by-products were determined as to these two lots using a microscope by the methods described in Example 2 and Example 3 to isolate a Cap of the neural retina and caps of the by-products. Rings were isolated by dissection under a stereo microscope using tapered tweezers and scissors, as in the grafts. From the caps and the rings isolated from the neural retina and the by-products, total RNA was extracted by the method described in Example 2. The concentration of the total RNA was measured in measurement equipment (Nanodrop, manufactured by Thermo Fisher Scientific Inc.), and then, it was reversely transcribed into cDNA using reverse transcriptase and primers (Reverse Transcription Master Mix Kit, manufactured by Fluidigm Corp.). The cDNA was subjected to multiplex-PCR reaction (Pre-Run) using all the probes used in the test and using a PCR apparatus (Veriti 96 well thermal cycler, manufactured by Applied Biosystems). Thereafter, the Pre-Run reaction solution was injected to multi-wells with flow channels (96.96 Dynamic Array IFC, manufactured by Fluidigm Corp.) using IFC Controller HX (manufactured by Fluidigm Corp.), and the expression level of marker gene in the neural retina and the by-products other than the neural retina was measured by real-time PCR using a multi-sample real-time PCR system (Biomark HD, manufactured by Fluidigm Corp.). The probes for PCR used in the test are shown in Table 13.

**[Table 13]**

| Classification | Gene name | Probe ID | GenBank ID |
|---|---|---|---|
| Internal standard | GAPDH | Hs02758991_g1 | NM_001256799.2, NM_001289745.2 |
| | | | NM_001289746.1, NM_001357943.1 |
| | | | NM_002046.7 |
| | ActinB | Hs01060665_g1 | NM_001101.5 |
| Neural retina | RAX | Hs00429459_m1 | NM_013435.2 |
| | Chx10 | Hs01584047_m1 | NM_182894.2 |
| | SIX3 | Hs00193667_m1 | NM_005413.4 |
| | SIX6 | Hs00201310_m1 | NM_007374.2 |
| | RCVRN | Hs00610056_m1 | NM_002903.2 |
| | CRX | Hs00230899_m1 | NM_000554.6 |
| | NRL | Hs00172997_m1 | NM_006177.4 |
| | NESTIN | Hs04187831_g1 | NM_006617.2 |
| Cerebrospinal | FOXG1 | Hs01850784_s1 | NM_005249.4 |
| | Emx2 | Hs00244574_m1 | NM_004098.4, NM_001165924.1 |
| | Nkx2.1 | Hs00968940_m1 | NM_003317.3, NM_001079668.2 |
| | Dmbx1 | Hs00542612_m1 | NM_172225.1, NM_147192.2 |
| | | | XM_011540668.2, XM_017000289.1 |
| | HOXB2 | Hs01911167_s1 | NM_002145.3, XM_005257275.4 |
| | HoxA5 | Hs00430330_m1 | NM_019102.4 |
| | MITF | Hs01117294_m1 | NM_000248.3, NM_006722.2 |
| | | | NM_198158.2, NM_198159.2 |
| | | | NM_198177.2, NM_198178.2 |
| | | | NM_001184967.1, NM_001184968.1 |
| | | | NM_001354604.1, NM_001354605.1 |
| Eyeball | | | NM_001354606.1, NM_001354607.1 |
| | | | NM_001354608.1 |
| | aqp1 | Hs01028916_m1 | NM_198098.3, NM_001329872.1 |
| | ZIC1 | Hs00602749_m1 | NM_003412.4 |
| | PAX2 | Hs01057416_m1 | NM_000278.4, NM_003987.4 |
| | | | NM_003988.4, NM_003989.4 |
| | | | NM_003990.4, NM_001304569.1 |
| Undifferentiated iPSC | POU5F1 | Hs00999632_g1 | NM_002701.6, NM_203289.5 |
| | | | NM_001173531.2, NM_001285986.1 |
| | | | NM_001285987.1 |
| | Nanog | Hs04260366_g1 | NM_024865.4, NM_001297698.1 |

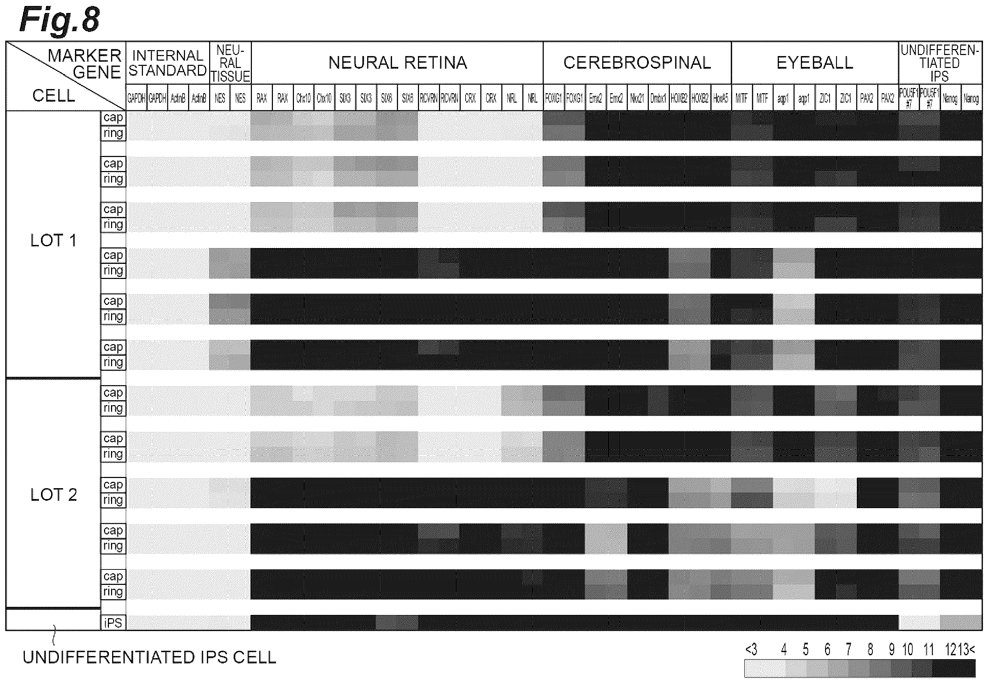

The results are shown in a heatmap in Figure 8. The gene expression levels were evaluated from ΔCt values calculated from the difference between the Ct value of the target gene and the Ct value of the GAPDH gene used as an internal standard. A lower ΔCt value represents a higher gene expression level, and a higher ΔCt value represents a lower gene expression level. The gray color corresponds to a high gene expression level, and the black color corresponds to a low gene expression level (a lighter color corresponds to a higher level of gene expression). As a result of examining gene expression in each Cap and ring, the neural retina marker gene group was expressed in the Cap and the Ring isolated from the neural retina in both the lot 1 and the lot 2. On the other hand, as a result of examining gene expression in the caps and the rings isolated from the by-products, the expression level of the neural retina marker gene group was low and the expression levels of the by-product marker gene groups were high, on the contrary to the neural retina, in both the lots. From this, it was able to be confirmed that the marker gene groups found in Example 2 were able to respectively detect the neural retina and the by-products by a distinction. Moreover, as a result of comparing gene expression between the Cap and the Ring isolated from the same cell aggregate, it was found that the expression level of the neural retina marker gene or the expression level of the by-product marker gene was equivalent between the Cap and the Ring isolated from any of the neural retina and the by-products.

From these results, it was able to be demonstrated that, provided that the Ring is the neural retina, the Cap is also the neural retina. It was also able to be demonstrated that gene expression is equivalent between the Cap and the ring.

### <Example 7 Verification of equivalent state between Cap and Ring for various pluripotent stem cell strains>

In order to examine whether gene expression would also be equivalent between a Cap and a Ring for cell aggregates differentiated from various pluripotent stem cells, the following was studied.

First, Crx:: Venus knock-in human ES cells (derived from KhES-1; Nakano, T. et al., Cell Stem Cell 2012, 10 (6), 771-785; obtained from Kyoto University, established in RIKEN CENTER FOR DEVELOPMENTAL BIOLOGY and put in use), human iPS cells (QHJI-01-s04 strain), and human iPS cells (DSP-SQ strain) were differentiated into retinas by the production method described in Example 1. Thereafter, caps and rings of the neural retina and by-products were dissected by the method described in Example 6 from cell aggregates on Day 70 or more from initiation of suspension culture. Thereafter, total RNA was extracted by the method described in Example 6. The concentration of the total RNA was measured in measurement equipment (Nanodrop, manufactured by Thermo Fisher Scientific Inc.), and then, it was reversely transcribed into cDNA using reverse transcriptase and primers (Reverse Transcription Master Mix Kit, manufactured by Fluidigm Corp.). The cDNA was subjected to multiplex-PCR reaction (Pre-Run) using all the probes used in the test and using a PCR apparatus (Veriti 96 well thermal cycler, manufactured by Applied Biosystems). Thereafter, the Pre-Run reaction solution was injected to multi-wells with flow channels (96.96 Dynamic Array IFC, manufactured by Fluidigm Corp.) using IFC Controller HX (manufactured by Fluidigm Corp.), and the expression level of marker gene in the neural retina and the by-products other than the neural retina was measured by real-time PCR using a multi-sample real-time PCR system (Biomark HD, manufactured by Fluidigm Corp.). The probes for PCR shown in Table 13 of Example 6 were used in the test.

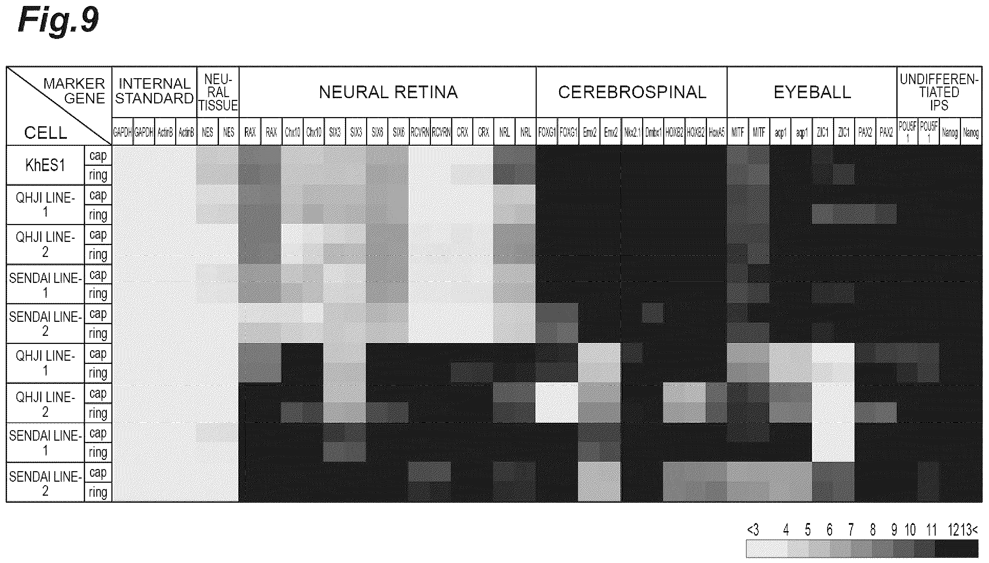

The results are shown in a heatmap in Figure 9. The heatmap was prepared in accordance with the method described in Example 6. As a result of examining gene expression in the caps and the rings, the expression level of the neural retina marker gene was high and the expression level of the by-product marker gene was low in the Cap and the Ring derived from any of the cell strains as long as they were isolated from the neural retina. On the other hand, the expression level of the by-product marker gene was high and the expression level of the neural retina marker gene was low in the caps and the rings isolated from the by-products. Moreover, as a result of comparing gene expression between the Cap and the Ring isolated from the same cell aggregate, it was found that the neural retina or by-product marker gene was equivalently expressed between the Cap and the Ring isolated from any of the cell strains.

From these results, it was also able to be demonstrated for the cell aggregate derived from any pluripotent stem cell strain that, provided that the Ring is the neural retina, the Cap is also the neural retina. It was also able to be demonstrated that gene expression is equivalent between the Cap and the ring. In short, it was found that even if a Cap of the size in Table 12 is dissected and if a Ring is dissected from the remaining portion in the cell aggregate, the Ring can be dissected as a portion having gene expression equivalent to that of the cap.

### <Example 8 Transplantation results of graft>

In Example 6 and Example 7, it was found that gene expression is equivalent between a Cap and a ring. It was also able to be confirmed that, provided that the Ring is the neural retina, the Cap is also the neural retina. Accordingly, a method of analyzing the gene expression of a Ring before transplantation to confirm that the Ring is the neural retina, and transplanting a Cap corresponding to the ring, was designed. For the purpose of demonstrating the usefulness of this method, the gene expression of a Ring was analyzed, and then, the corresponding Cap was transplanted to a retinal degenerative nude rat to evaluate images of post-transplant engraftment.

First, a cell aggregate was prepared from human iPS cells (DSP-SQ strain) in accordance with the method described in Example 1. Thereafter, a Cap and a Ring were isolated by the method described in Example 6 from the cell aggregate on Day 75 or later from initiation of suspension culture. The isolated Cap was preserved using a commercially available preservation solution while the gene analysis of the Ring was carried out. The isolated Ring was subjected to gene expression analysis by real-time PCR using Biomark HD (manufactured by Fluidigm Corp.) in accordance with the method described in Example 6. From the results of the gene expression analysis, a Ring that expressed the neural retina marker gene and did not express the by-product marker gene was selected, and a Cap corresponding to this Ring was selected as a graft. The graft was washed with a buffer (manufactured by Thermo Fisher Scientific Inc.) and then subretinally transplanted to a retinal degenerative nude rat (photoreceptor cell degenerative model, SD-Foxn1 Tg(S334ter)3LavRrrc nude rat) using the injector described in the known literature (Shirai et al., PNAS 113, E81-E90).

The eye tissue obtained on Days 230 to 240 from initiation of the suspension culture was fixed with paraformaldehyde (PFA fixed) and subjected to sucrose replacement. The eye tissue fixed was frozen and sectioned by use of a cryostat. These frozen sections were subjected to immunostaining to stain a human nucleus (anti-HuNu antibody, Merck Millipore, mouse, or anti-HNA antibody), a photoreceptor cell marker recoverin (anti-recoverin antibody, Proteintech Group, rabbit) and a bipolar cell marker PKCα (anti-PKCα antibody, R&D systems, Inc., goat).

Results of summarizing quality evaluation results by the gene expression analysis of rings and transplantation results of grafts are shown in Table 14. A method for calculating ΔCt values employed the method described in Example 6. The gene expression analysis of rings passed them on the quality evaluation test (ring-PCR test) when the ΔCt value of a neural retina marker gene, recoverin, was 10 or less and each of the ΔCt values of by-product marker genes FOXG1, HOXB2, ZIC1 and OCT3/4 was 5 or more. As for the transplantation results, engraftment was evaluated as being favorable when human nucleus-positive and recoverin-positive photoreceptor cells were able to be subretinally detected. It was determined that swelling was not detected unless the transplantation site was much thicker than the proper size of engraftment.

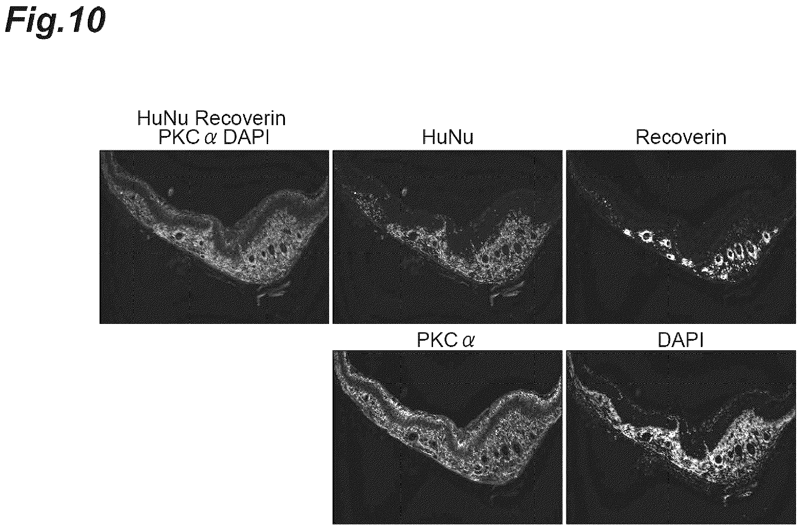

A typical image of engraftment is shown in Figure 10. As a result of evaluating images of post-transplant engraftment as to 14 eyes passed on the quality evaluation test before transplantation, recoverin-positive photoreceptor cells were detected in all the 14 eyes. Thus, favorable engraftment was found. Since these cells were HuNu-positive, it was found that the recoverin-positive photoreceptor cells were derived from the transplanted caps. Swelling was not detected in any of the 14 eyes.

From the results described above, it was able to be demonstrated that a graft that is subretinally favorably engrafted, i.e., in which photoreceptor cells are engrafted without causing swelling, can be selected by examining the expression levels of the neural retina and by-products marker genes before transplantation by the gene expression analysis of the ring.

**[Table 14]**

| Graft | | Transplantation results | |
|---|---|---|---|
| Strain | Quality evaluation test | Engraftment of photoreceptor cell | Swelling |
| DSP-SQ | Pass on ring-PCR test | All cases of 14 eyes | All cases of 14 eyes |
| | | Favorable engraftment | Not observed |

### <Example 9 Transplantation results of graft>

In Example 8, Biomark HD (manufactured by Fluidigm Corp.) was used in the gene expression analysis of rings. Biomark HD is originally a machine designed for single-cell analysis, and the way of use in Example 8 is not general. Accordingly, the gene expression of a Ring was analyzed in a real-time PCR apparatus generally used, to test whether a graft to be subretinally favorably engrafted could be selected, as in the case of using Biomark HD.

Human iPS cells (DSP-L strain, established by Sumitomo Dainippon Pharma Co., Ltd.) are those established by using commercially available Sendai virus vector (4 factors, i.e., Oct3/4, Sox2, KLF4, c-Myc, site tune kit manufactured by ID Pharma Co., Ltd.) based on the method described in the protocol open to public by Thermo Fisher Scientific Inc. (iPS 2.0 Sendai Reprogramming Kit, Publication Number MAN0009378, Revision 1.0) and protocol open to public (establishment/maintenance culture of feeder-free human iPS cells, CiRA_Ff-1PSC_protocol_JP_v140310, http://www.cira.kyoto-u.ac.jp/j/research/protocol.html) by Kyoto University, and using StemFit medium (AK03; manufactured by Ajinomoto Co., Inc.) and Laminin 511-E8 (manufactured by Nippi. Inc.).

First, a cell aggregate was prepared from human iPS cells (DSP-L strain) in accordance with the method described in Example 1. Thereafter, a Cap and a Ring were isolated by the method described in Example 6 from the cell aggregate on Day 86 from initiation of suspension culture. The isolated Cap was preserved using a commercially available preservation solution while the gene analysis of the Ring was carried out. The gene expression analysis of the isolated Ring was carried out as follows.

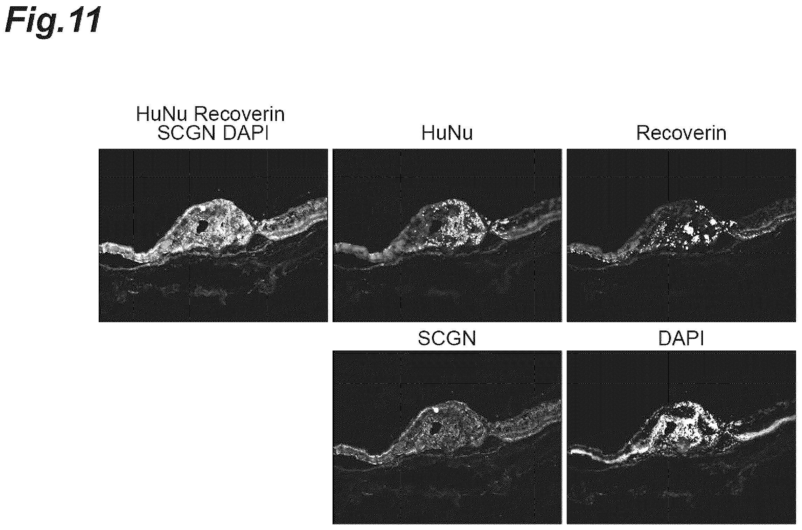

First, total RNA was extracted by the method described in Example 2. The concentration of the total RNA was measured in measurement equipment (Nanodrop, manufactured by Thermo Fisher Scientific Inc.), and then, it was reversely transcribed into cDNA using reverse transcriptase (QuantiTect Revese Transcription Kit (manufactured by QIAGEN N.V.) and primers (RT primer mix, manufactured by QIAGEN N.V.)). Thereafter, the cDNA was subjected to real-time PCR using real-time PCR enzymes (TaqMan Fast Advanced Master Mix, manufactured by Applied Biosystems) and using StepOnePlus real-time PCR system (manufactured by Applied Biosystems) to measure the expression level of marker gene in the neural retina and the by-products other than the neural retina. The results are shown in Figure 11. A sample in which cDNA of undifferentiated iPS cells and cDNA of a three-dimensional retina were mixed was used as a positive control sample. The positive control sample was serially diluted to prepare calibration curve samples, and a calibration curve was drawn. Gene expression was normalized using gapdh as an internal standard.

As a result of the PCR analysis, it was found that Sample 1 and Sample 2 had high expression levels of the neural retina markers Chx10, Rx, Crx and recoverin and had low expression levels of the by-product marker genes Oct3/4, FoxG1, Aqpl, Nkx2.1, Dlx6, CDH6, Emx2, Zic1, HoxA5, Pax2 and Pax8. A Ring that expressed the neural retina marker gene and did not express the by-product marker gene was selected, and a Cap corresponding to this Ring was subretinally transplanted to a retinal degenerative nude rat in accordance with the method described in Example 8.

The eye tissue obtained on Days 230 to 240 from initiation of the suspension culture was fixed with paraformaldehyde (PFA fixed) and subjected to sucrose replacement. The eye tissue fixed was frozen and sectioned by use of a cryostat. These frozen sections were subjected to immunostaining to stain a human nucleus (anti-HuNu antibody, Merck Millipore, mouse), a photoreceptor cell marker recoverin (anti-recoverin antibody, Proteintech Group, rabbit) and a bipolar cell marker SCGN (anti-SCGN antibody, BioVendor Group, sheep).

Images of engraftment in the rat eye to which the Cap corresponding to the Ring was transplanted are shown in Figure 11. As a result of evaluating the images of post-transplant engraftment, recoverin-positive photoreceptor cells were found and exhibited favorable engraftment. Since these cells were HuNu-positive, it was found that the recoverin-positive photoreceptor cells were derived from the transplanted cap. Swelling was not detected.

From the results described above, it was found that a graft that is subretinally favorably engrafted can be selected by examining the expression levels of the neural retina and by-products marker genes before transplantation even when the gene expression of a Ring is analyzed using a real-time PCR system generally used. Thus, it was found that the analysis of a plurality of genes is possible by a multi-sample real-time PCR system.

### <Example 10 Transplantation results of ring>

In Examples 8 and 9, it was found that, when a Cap prepared by the approach described above is subretinally transplanted to a retinal degenerative nude rat, photoreceptor cells are engrafted. For the purpose of verifying the equivalent state between a Ring and a cap, study was conducted to subretinally transplant the Ring in the same manner.

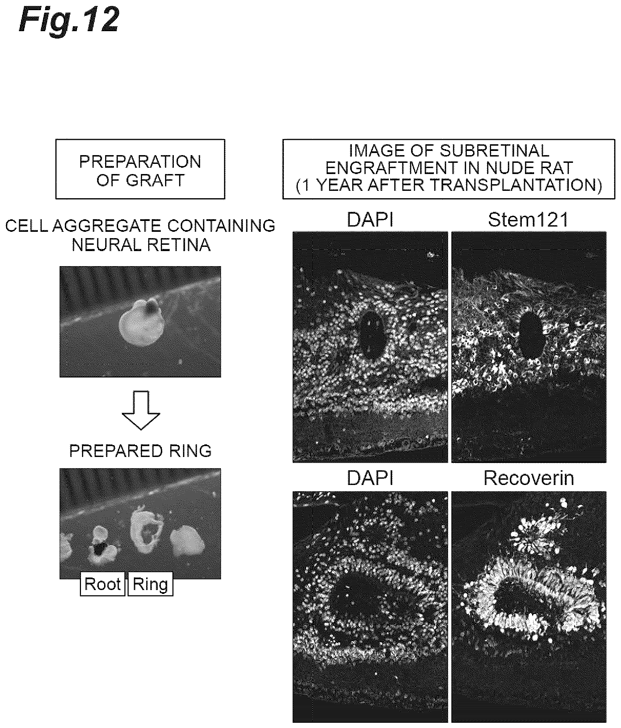

Crx::Venus knock-in human ES cells (derived from KhES-1; Nakano, T. et al., Cell Stem Cell 2012, 10 (6), 771-785; obtained from Kyoto University, established in RIKEN CENTER FOR DEVELOPMENTAL BIOLOGY and put in use) were differentiated into retinas by the production method described in Example 1. Thereafter, caps and rings of the neural retina and by-products were dissected by the method described in Example 6 from a cell aggregate on Day 74 from initiation of suspension culture (Figure 12). Thereafter, the Ring was subretinally transplanted to a photoreceptor cell degenerative model, a retinal degenerative nude rat (SD-Foxn1 Tg(S334ter)3LavRrrc nude rat) using the injector of the known literature (Shirai et al., PNAS 113, E81-E90).

The retinal degenerative nude rat was raised for 1 year after the transplantation. Thereafter, eye tissue to which the Ring was transplanted was harvested, fixed with paraformaldehyde (PFA fixed) and subjected to sucrose replacement. The eye tissue fixed was frozen and sectioned by use of a cryostat. These frozen sections were subjected to immunostaining to stain a human cytoplasm marker Stem121 (anti-Stem121 antibody, Cellartis, mouse) or a photoreceptor cell marker recoverin (anti-recoverin antibody, Proteintech Group, rabbit).

Bright-field images taken immediately after dissecting the Ring used in the transplantation, and the results of immunostaining the sections prepared from the eye tissue harvested from the rat raised for 1 year after the Ring transplantation are each shown in Figure 12. As a result of subretinally transplanting the Ring shown on the left side of Figure 12 to the retinal degenerative nude rat, the immunostaining results shown on the right side of Figure 12 were obtained. From the immunostaining results, it was found that Stem121-positive human cells were engrafted. It was also found that recoverin-positive photoreceptor cells were engrafted.

From the results described above, it was found that when a Ring is transplanted, photoreceptor cells are engrafted, as in a cap. This result further suggested that similar transplantation results are obtained by transplanting either a Cap or a Ring as long as it is the neural retina.

### <Example 11 Verification of equivalent state between Cap and ring>

In Examples 6 and 7, it was demonstrated by use of PCR that gene expression was equivalent between a Cap and a Ring at the RNA level. It was further demonstrated that whether the Cap is a neural retina or a by-product can be tested by analyzing the gene expression of the Ring by PCR. Next, study was made to be able to test whether the Cap would be a neural retina or a by-product by analyzing the gene expression of the Ring by immunostaining.

First, human iPS cells (DSP-SQ strain) were differentiated into retinas by the production method described in Example 1. Thereafter, a Cap and a Ring of the neural retina were isolated by the method described in Example 6 from a cell aggregate on Day 120 from initiation of suspension culture. Thereafter, the Cap and the Ring were washed, then fixed with 4% paraformaldehyde (PFA fixed) and subjected to sucrose replacement. The Cap and the Ring fixed were frozen and sectioned by use of a cryostat. These frozen sections were subjected to immunostaining to stain a nucleus with DAPI, a photoreceptor precursor cell marker Crx (anti-Crx antibody, Takara Bio Inc., rabbit), a neural retina marker Chx10 (anti-Chx10 antibody, Exalpha Biologicals, sheep), a rod photoreceptor precursor cell marker NRL (anti-NRL antibody, Bio-Techne Corp., goat), a telencephalon marker FOXG1 (anti-FOXG1 antibody, Takara Bio Inc., rabbit), an optic stalk marker PAX2 (anti-PAX2 antibody, Thermo Fisher Scientific Inc., rabbit), and an undifferentiated pluripotent stem cell marker NANOG (anti-NANOG antibody, Merck, mouse).

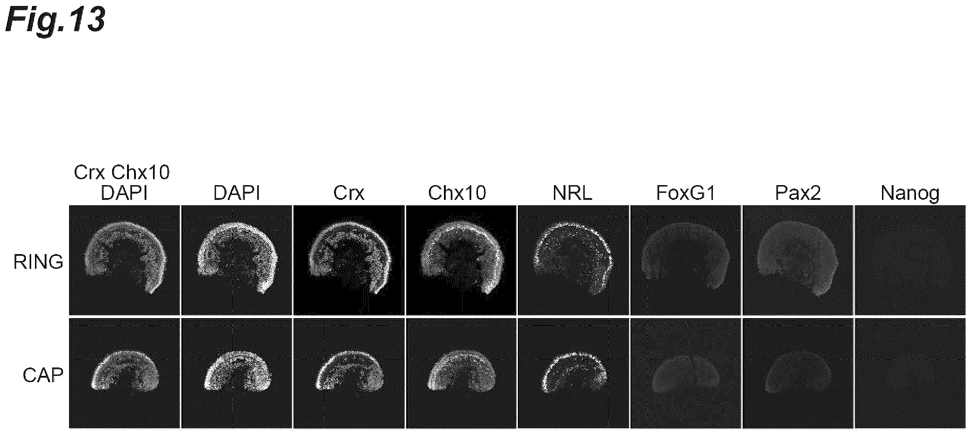

The results of the immunostaining are shown in Figure 13. As for the Cap and the Ring dissected from the same cell aggregate, the results of immunostaining the Ring are shown in the upper boxes, and the results of immunostaining the Cap are shown in the lower boxes. From these results, it was found that Crx-positive photoreceptor precursor cells, Chx10-positive neural retina, and NRL-positive rod photoreceptor precursor cells were expressed in a continuous layer pattern in both the Cap and the ring. FOXG1-positive telencephalon, PAX2-positive optic stalk, or NANOG-positive pluripotent stem cells were not detected in any of the Cap and the ring. When Crx-, Chx10- and NRL-stained images were compared, it was found that these neural retina markers exhibited almost equivalent distribution between the Cap and the ring.

From the results described above, it was able to be demonstrated not only by gene expression analysis but by immunostaining that a Cap and a Ring are equivalent. From this result, it was able to be demonstrated that whether the Cap is a neural retina or a by-product can be tested by analyzing the gene expression of the Ring by immunostaining.

### <Example 12 Verification of equivalent state between Cap and Ring for non-neural retina>

In Examples 6 and 7, it was found that gene expression is equivalent between caps and rings of the neural retina and by-products. Accordingly, whether gene expression would be equivalent between a Cap and a Ring isolated from a tissue other than the retina was analyzed in detail.

First, human iPS cells (QHJI-01-s04 strain) and human iPS cells (DSP-SQ strain) were differentiated into retinas by the production method described in Example 1. As a result of then microscopically observing cell aggregates on Day 80 or more from initiation of suspension culture, the presence of an epithelial structure was observed. Caps and rings of the neural retina and by-products were dissected by the method described in Example 6 from the cell aggregates on Day 80 or more from initiation of suspension culture. Thereafter, RNA was extracted by the method described in Example 6, and the expression level of marker gene in the neural retina and the by-products other than the neural retina was measured by real-time PCR using Biomark HD (manufactured by Fluidigm Corp.).

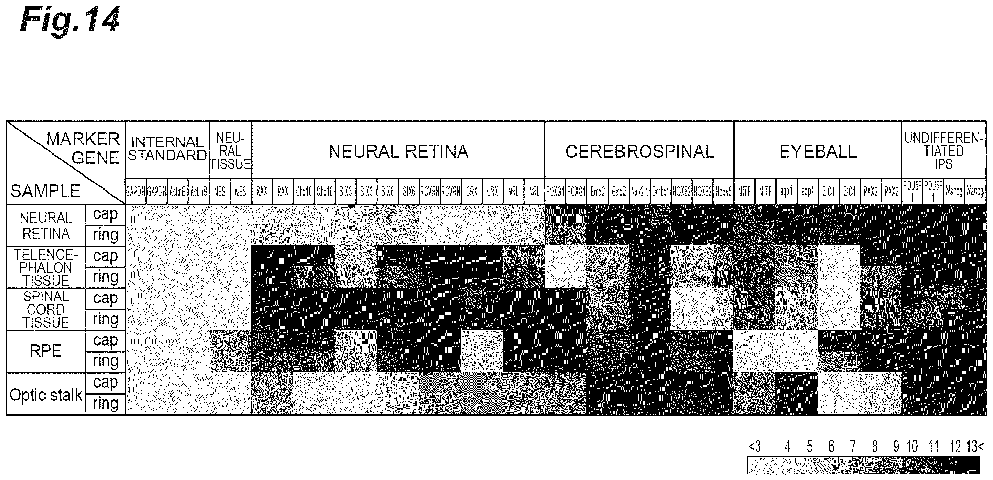

The results are shown in a heatmap in Figure 14. The heatmap was prepared in accordance with the method described in Example 6. As a result of examining gene expression in the caps and the rings, it was confirmed that the neural retina marker gene was expressed and the by-product marker gene was not expressed, in the Cap and the Ring dissected from the neural retina. On the other hand, as a result of examining gene expression in the caps and the rings dissected from the by-products, a Cap and a Ring highly expressing a telencephalon marker FOXG1, a Cap and a Ring highly expressing spinal cord markers HOXB2 and HOXA5, a Cap and a Ring highly expressing a RPE marker MITF, and a Cap and a Ring highly expressing an optic stalk marker PAX2, were found. Accordingly, from the highly expressed marker genes, these by-products were each classified into telencephalon tissue, spinal cord tissue, RPE and optic stalk. As a result of examining gene expression in the caps and the rings isolated from these by-products, all the by-products exhibited equivalent gene expression between the Cap and the ring.

From the results described above, it was found that gene expression was equivalent between caps and rings isolated not only from the neural retina but from telencephalon tissue, spinal cord tissue, RPE and optic stalk. From this result, it was found that whether the Cap is the same tissue can be determined by examining gene expression in the Ring isolated from a non-neural retina such as telencephalon tissue, spinal cord tissue, RPE, and optic stalk.

### <Example 13 Ratios of photoreceptor precursor cell and neural retinal precursor cell constituting transplant neural retina sheet>

The ratios of photoreceptor precursor cells and neural retinal precursor cells to cells constituting a transplant neural retina sheet prepared from a cell aggregate differentiated from pluripotent stem cells were analyzed and quantified by an immunostaining method, immunohistochemistry (IHC).

Human iPS cells (DSP-SQ strain) were differentiated into retinas by the production method described in Example 1. Thereafter, caps and rings were isolated by the method described in Example 6 from the cell aggregates on Days 84, 92 and 93 from initiation of suspension culture. The gene expression analysis of the isolated rings was carried out by the method described in Example 8. A Ring that expressed the neural retina marker gene and did not express the by-product marker gene was selected by the method described in Example 8, and a Cap corresponding to this Ring was used as a transplant neural retina sheet. In this way, one transplant neural retina sheet from the cell aggregate on Day 84 from initiation of suspension culture, two transplant neural retina sheets from the cell aggregate on Day 92 from initiation of suspension culture, and one transplant neural retina sheet from the cell aggregate on Day 93 from initiation of suspension culture, were prepared. In other words, a total of four transplant neural retina sheets were prepared.

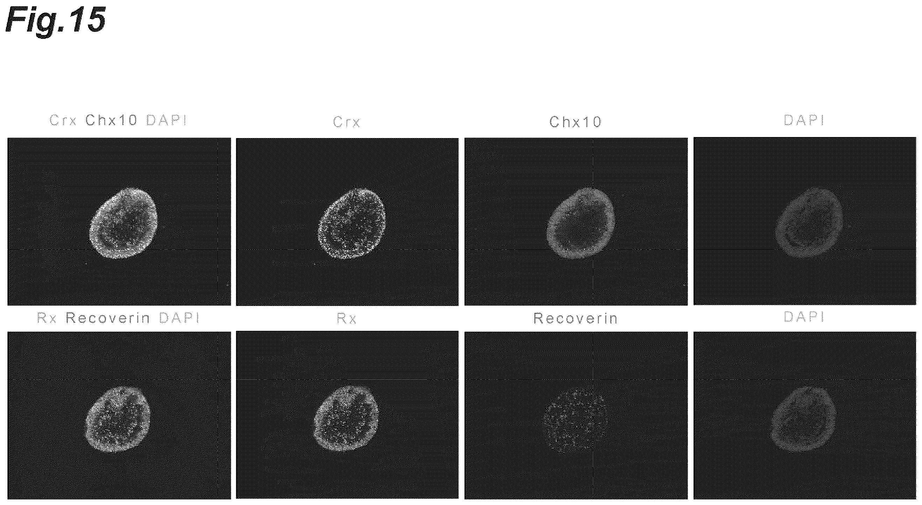

The obtained transplant neural retina sheets were cultured for 7 days in B medium for analysis. The cultured transplant neural retina sheets were fixed with 4% paraformaldehyde, frozen and sectioned. The frozen sections were subjected to immunostaining to stain a neural retinal precursor cell marker, Chx10 (anti-Chx10 antibody, Exalpha Biologicals, sheep) and a photoreceptor precursor cell marker Crx (anti-Crx antibody, Takara Bio Inc., rabbit). Other frozen sections were subjected to immunostaining to stain a neural retina marker Rx (anti-Rx antibody, Takara Bio Inc., guinea pig) and a photoreceptor cell marker recoverin (anti-recoverin antibody, Proteintech Group, rabbit). The nuclei of the cells were stained with DAPI. These sections stained were observed using a fluorescence microscope (manufactured by Keyence Corp.) to obtain immunostained images. One example thereof (D3) is shown in Figure 15.

The immunostained images were analyzed in ImageJ (version 1.52a, manufactured by National Institutes of Health (NIH)) to analyze the number of DAPI-positive cells, the number of DAPI-positive and Chx10-positive cells, and the number of DAPI-positive and Crx-positive cells as to each of the four transplant neural retina sheets. The immunostained images were analyzed in the same manner to analyze the number of DAPI-positive cells and the number of DAPI-positive and Rx-positive cells. From these numerical values, the ratio of Chx10-positive cells, the ratio of Crx-positive cells, and the ratio of Rx-positive cells were calculated. The obtained results are shown in Table 15.

**[Table 15]**

| Cap ID No | The number of days from initiation of suspension culture (d) | Ratio of positive cell (%) | | |
|---|---|---|---|---|
| | | Chx10 | Crx | Rx |
| B3 | 84+7 | 44.6 | 29.7 | 39.5 |
| D2 | 92+7 | 38.9 | 41.5 | 44.3 |
| D3 | 92+7 | 22.7 | 39.0 | 53.5 |
| G3 | 93+7 | 36.6 | 55.5 | 53.5 |

From the results described above, it was found that the ratio of Chxl0-positive cells contained in the transplant neural retina sheet was on the order of 23 to 45%, the ratio of Crx-positive cells was on the order of 30 to 56%, and the ratio of Rx-positive cells was on the order of 40 to 54%.

In short, it was suggested that about 34% (about 23 to 45%) Chx10-positive neural retinal precursor cells, about 40% (30 to 56%) Crx-positive photoreceptor precursor cells, and about 47% (40 to 54%) Rx-positive cells were contained in the transplant neural retina sheet.

### <Example 14 Ratios of photoreceptor precursor cell and neural retinal precursor cell constituting transplant neural retina sheet>

The composition of cells constituting transplant neural retina sheets prepared from cell aggregates differentiated from various pluripotent stem cells was examined by an immunostaining method, flow cytometry (also referred to as FACS).

Human iPS cells (QHJI-01-s04 strain) were differentiated into retinas by the production method described in Example 1. Thereafter, a Cap and a Ring were isolated by the method described in Example 6 from the cell aggregate on Day 88 from initiation of suspension culture. The Cap was used as a transplant neural retina sheet. The transplant neural retina sheet was preserved at a low temperature of 17°C for 2 days. Five transplant neural retina sheets obtained were combined as one sample, washed with PBS, enzymatically treated at 37°C for 30 minutes using a neuronal cell dispersion solution (manufactured by FUJIFILM Wako Pure Chemical Corp, containing papain), and dispersed into single cells by pipetting to obtain a single-cell suspension. The obtained single-cell suspension was fixed using a fixative solution (manufactured by Becton, Dickinson and Company, CytoFix) to obtain a sample for FACS. The sample for FACS was subjected to blocking and permeation treatment (cell membrane perforation) using Perm/Wash buffer (manufactured by Becton, Dickinson and Company) containing serum. Then, immunostaining was performed with the following antibodies fluorescently labeled: anti-Chx10 antibody (manufactured by Santa Cruz Biotechnology, Inc.), anti-Pax6 antibody (manufactured by Becton, Dickinson and Company), and anti-Crx antibody (manufactured by Santa Cruz Biotechnology, Inc.). Then, analysis was conducted by flow cytometry using an analyzer (manufactured by Becton, Dickinson and Company).

As a result, it was found that a Chx10-positive and Pax6-positive fraction (neural retinal precursor cell fraction) occupied 11.5%, a Chx10-positive and Pax6-negative fraction (precursor cell fraction biased toward bipolar cells) occupied 23.4%, a Chx10-negative and Pax6-positive fraction (ganglion cell and amacrine cell fraction) occupied 10.7%, and a Crx-positive cell fraction (photoreceptor precursor cell fraction) occupied 17.4%.

Further, human iPS cells (DSP-SQ strain) were differentiated into retinas by the production method described in Example 1. Thereafter, **11** cell aggregates on Day 88 from initiation of suspension culture were prepared, and **11** each of caps and rings were isolated by the method described in Example 6 from each of the cell aggregates. The **11** caps were combined as one sample. Likewise, the **11** rings were combined as one sample. The Cap sample and the Ring sample were each washed with PBS and enzymatically treated at 37°C for 30 minutes using a neuronal cell dispersion solution (manufactured by FUJIFILM Wako Pure Chemical Corp, containing papain) to obtain respective single-cell suspensions of the Cap and the ring. The obtained respective single-cell suspensions of the Cap and the Ring were fixed using a fixative solution (manufactured by Becton, Dickinson and Company, CytoFix) to obtain samples for FACS. The samples for FACS were subjected to blocking and perforation using Perm/Wash buffer (manufactured by Becton, Dickinson and Company) containing serum and subjected to immunostaining with the following antibodies fluorescently labeled: anti-Chx10 antibody (manufactured by Santa Cruz Biotechnology, Inc.), anti-Crx antibody (manufactured by Santa Cruz Biotechnology, Inc.), and anti-SSEA-4 antibody. Isotype controls were used as negative controls for immunostaining. Then, analysis was conducted by flow cytometry using an analyzer (manufactured by Becton, Dickinson and Company). The ratio of Chx10-positive cells, the ratio of Crx-positive cells, and the ratio of SSEA-4-positive cells were calculated from delta from their respective isotype controls. The results are described in Table 16.

**[Table 16]**

| Sample | The number of days from initiation of suspension culture (d) | Ratio of positive cell (%) | | |
|---|---|---|---|---|
| | | Chx10 | Crx | SSEA-4 |
| Cap | 88 | 29.4 | 15.8 | <1 |
| Ring | 88 | 28.1 | 21.7 | <1 |

In the Cap sample, the ratio of neural retinal precursor cell marker Chx10-positive cells was 29.4%, the ratio of photoreceptor precursor cell marker Crx-positive cells was 21.7%, and the ratio of pluripotent stem cell marker SSEA-4-positive cells (non-target cells) was less than 1%. In the Ring sample, the ratio of neural retinal precursor cell marker Chx10-positive cells was 28.1%, the ratio of photoreceptor precursor cell marker Crx-positive cells was 15.8%, and the ratio of pluripotent stem cell marker SSEA-4-positive cells (non-target cells) was less than 1%.

From these results, first, it was found that the Cap sample and the Ring sample were neural retinas containing Chx10-positive cells and Crx-positive cells and did not substantially contain undifferentiated iPS cells. Furthermore, it was able to be demonstrated that the ratios of Chxl0-positive cells and Crx-positive cells contained in the Cap sample were equivalent to the ratios of Chx10-positive cells and Crx-positive cells contained in the Ring sample. Moreover, it was able to be demonstrated that, provided that the Ring sample was the neural retina, the Cap was also the neural retina.

Besides, it was found that, in the case of using such a Cap or a Ring (preferably cap) as a transplant neural retina sheet, the Chx10-positive cell fraction (neural retinal precursor cell fraction) contained in this transplant neural retina sheet occupied about 30% (about 20 to 40%), and the Crx-positive cell fraction (photoreceptor precursor cell fraction) occupied about 17% (about 10 to 30%).

For the approach according to the present application, the usefulness of quantitative PCR for evaluating multiple samples or multiple genes as an approach of quality evaluation was demonstrated, whereas it was found that the quality evaluation can also be carried out by an immunostaining method, flow cytometry analysis.

From the results described above, it was found that for a cell aggregate containing epithelial tissue (preferably neural epithelium, more preferably neural retina) having an epithelial structure, the quality of a Cap can be insured by isolating the Cap and a Ring from one epithelial structure, and examining the gene expression of the ring. It was found that both quantitative PCR and immunostaining are useful as approaches of examining the gene expression. It was further found that the methodology of this quality examination can be applied both when the epithelial tissue having the epithelial structure is the neural retina and when it is the non-neural retina.

### Industrial Applicability

According to the present invention, it becomes possible to provide a method for evaluating the quality of a transplant neural retina and a transplant neural retina sheet selected by the method.

## Claims

1. A method for evaluating the quality of a transplant neural retina,
the method comprising:
sampling a part or the whole of a cell aggregate containing a neural retina having an epithelial structure derived from a pluripotent stem cell as a sample for quality evaluation;
detecting the expression of a neural retina-related cell-related gene and a non-neural retina-related cell-related gene in the sample for quality evaluation; and
when the expression of the neural retina-related cell-related gene is found and the expression of the non-neural retina-related cell-related gene is not found, determining that
(1) the neural retina (transplant neural retina) in the same cell aggregate as the cell aggregate containing the sample for quality evaluation being the part,
(2) the neural retina (transplant neural retina) in a cell aggregate of the same lot as the cell aggregate containing the sample for quality evaluation being the part, or
(3) the neural retina (transplant neural retina) in a cell aggregate of the same lot as the cell aggregate of the sample for quality evaluation being the whole,
is applicable as the transplant neural retina, wherein
the non-neural retina-related cell-related gene comprises one or more genes selected from the group consisting of a brain and spinal cord tissue marker gene and an eyeball-related tissue marker gene, wherein
the brain and spinal cord tissue marker gene is one or more genes selected from the group consisting of a telencephalon marker gene, a diencephalon/midbrain marker gene, and a spinal cord marker gene, and
the eyeball-related tissue marker gene is one or more genes selected from the group consisting of an optic stalk marker gene, a ciliary body marker gene, a lens marker gene and a retinal pigment epithelium marker gene;
wherein
the telencephalon marker gene comprises one or more genes selected from the group consisting of FoxG1**,** Emx2, Dlx2, Dlx1 and Dlx5,
the diencephalon/midbrain marker gene comprises one or more genes selected from the group consisting of DMBX1, Nkx2.1, OTP, FGFR2, EFNA5 and GAD1,
the spinal cord marker gene comprises one or more genes selected from the group consisting of HOXD4, HOXD3, HOXD1, HOXC5, HOXA5 and HOXB2,
the optic stalk marker gene comprises one or more genes selected from the group consisting of GREM1, GPR17, ACVR1C, CDH6, Pax2, Pax8, GAD2 and SEMA5A,
the ciliary body marker gene comprises one or more genes selected from the group consisting of Zic1, MAL, HNF1beta, FoxQ1, CLDN2, CLDN1, GPR177, AQP1 and AQP4,
the lens marker gene comprises one or more genes selected from the group consisting of CRYAA and CRYBA1, and
the retinal pigment epithelium marker gene comprises one or more genes selected from the group consisting of MITF, TTR and BEST1; and,
wherein the neural retina-related cell-related gene is one or more genes selected from the group consisting of RAX, Chx10, SIX3, SIX6, RCVRN, CRX, NRL and NESTIN.

2. The method according to claim 1, wherein the non-neural retina-related cell-related gene further comprises an undifferentiated pluripotent stem cell marker gene;
optionally wherein the undifferentiated pluripotent stem cell marker gene comprises one or more genes selected from the group consisting of Oct3/4, Nanog and Lin28.

3. The method according to claim 1 or 2, wherein the cell aggregate of the same lot as the cell aggregate of the sample for quality evaluation is a cell aggregate produced in the same cell culture container as that of the transplant neural retina.

4. The method according to any one of claims 1 to 3, wherein
the sample for quality evaluation is a part of the cell aggregate, and
when the expression of the neural retina-related cell-related gene is found and the expression of the non-neural retina-related cell-related gene is not found, it is determined that a neural retina continuous or adjacent at least partially to the part in the same cell aggregate as the cell aggregate containing the sample for quality evaluation being the part is applicable as the transplant neural retina;
optionally wherein the transplant neural retina is contained in the same epithelial tissue as that of the sample for quality evaluation;
optionally wherein the transplant neural retina contains the center and/or its neighborhood of the same epithelial tissue;
optionally wherein the transplant neural retina is continuous epithelial tissue.

5. The method according to claim 4, wherein
the cell aggregate containing a neural retina contains first epithelial tissue containing the transplant neural retina, and second epithelial tissue having the continuity of the slope of a tangent line to a surface different from the continuity of the slope of a tangent line to the surface of the first epithelial tissue, and containing a non-neural retina-related cell,
the transplant neural retina contains a region on the first epithelial tissue most distant from the second epithelial tissue, and
the sample for quality evaluation is a part present between the second epithelial tissue and the transplant neural retina.

6. The method according to claim 5, wherein the second epithelial tissue is eyeball-related tissue and/or brain and spinal cord tissue;
optionally wherein the eyeball-related tissue contains a retinal pigment epithelial cell and ciliary body.

7. The method according to any one of claims 1 to 6, comprising performing the detection of the expression of the neural retina-related cell-related gene and the non-neural retina-related cell-related gene by quantitative PCR;
optionally comprising determining as being applicable as the transplant neural retina when the following reference 1 and reference 2 are satisfied:
reference 1: the difference between the threshold cycle (Ct) value of the neural retina-related cell-related gene and the Ct value of an internal standard gene (ΔCt value) is 10 or less, and
reference 2: the difference between the Ct value of the non-neural retina-related cell-related gene and the Ct value of the internal standard gene (ΔCt value) is 5 or more; and/or
optionally wherein the quantitative PCR is performed by a method comprising the following steps (1) to (4), thereby simultaneously detecting the respective expression levels of neural retina-related cell-related gene and non-neural retina-related cell-related gene in two or more of the samples for quality evaluation:
(1) providing a flow channel plate having one sample well group consisting of 8 or more and 800 or less independent sample wells, one or more primer well groups consisting of 8 or more and 800 or less independent primer wells, and flow channels connecting the independent sample wells in the sample well group with the independent primer wells in each primer well group, solutions containing nucleic acids obtained from the two or more of the samples for quality evaluation (sample solutions), and a solution containing one or a plurality of primers specific for each of one or more of the neural retina-related cell-related genes or the non-neural retina-related cell-related genes (primer solution);
(2) adding the sample solutions at one sample solution/one sample well for each of the samples for quality evaluation to the sample well group;
(3) adding the primer solution to one or more primer wells in the one or more primer well groups so as to be different primer well groups;
(4) separately mixing the primers with the nucleic acids via the flow channels; and
(5) performing quantitative PCR using the mixture obtained in (4).

8. A neural retina sheet,
(1) being derived from a pluripotent stem cell,
(2) having a three-dimensional structure,
(3) comprising a neural retinal layer having a plurality of layer structures including a photoreceptor layer and an inner layer,
(4) the photoreceptor layer comprising one or more cells selected from the group consisting of a photoreceptor precursor cell and a photoreceptor cell,
(5) the inner layer comprising one or more cells selected from the group consisting of a retinal precursor cell, a ganglion cell, an amacrine cell and a bipolar cell,
(6) the surface of the neural retinal layer having an apical surface,
(7) the inner layer being present inside the photoreceptor layer present along the apical surface,
(8) the area of the neural retinal layer being 50% or more with respect to the total area of the surface of the neural retina sheet,
(9) the area of a continuous epithelium structure being 80% or more with respect to the total area of the apical surface of the neural retinal layer, and
(10) the expression of neural retina-related cell-related gene being found and the expression of non-neural retina-related cell-related gene being not found in the neural retina sheet, wherein the non-neural retina-related cell-related gene comprising one or more genes selected from the group consisting of a brain and spinal cord tissue marker gene and an eyeball-related tissue marker gene, wherein
the brain and spinal cord tissue marker gene is one or more genes selected from the group consisting of a telencephalon marker gene, a diencephalon/midbrain marker gene, and a spinal cord marker gene, and
the eyeball-related tissue marker gene is one or more genes selected from the group consisting of an optic stalk marker gene, a ciliary body marker gene, a lens marker gene and a retinal pigment epithelium marker gene;
wherein
the telencephalon marker gene comprises one or more genes selected from the group consisting of FoxG1, Emx2, Dlx2, Dlx1 and Dlx5,
the diencephalon/midbrain marker gene comprises one or more genes selected from the group consisting of DMBX1, Nkx2.1, OTP, FGFR2, EFNA5 and GAD1,
the spinal cord marker gene comprises one or more genes selected from the group consisting of HOXD4, HOXD3, HOXD1, HOXC5, HOXA5 and HOXB2,
the optic stalk marker gene comprises one or more genes selected from the group consisting of GREM1, GPR17, ACVR1C, CDH6, Pax2, Pax8, GAD2 and SEMA5A,
the ciliary body marker gene comprises one or more genes selected from the group consisting of Zic1, MAL, HNF1beta, FoxQ1, CLDN2, CLDN1, GPR177, AQP1 and AQP4,
the lens marker gene comprises one or more genes selected from the group consisting of CRYAA and CRYBA1, and
the retinal pigment epithelium marker gene comprises one or more genes selected from the group consisting of MITF, TTR and BEST1, and,
wherein in (10) the neural retina-related cell-related gene is one or more genes selected from the group consisting of RAX, Chx10, SIX3, SIX6, RCVRN, CRX, NRL and NES TIN.

9. The neural retina sheet according to claim 8, wherein the major axis is from 600 µm to 2500 µm; and/or
wherein the minor axis is from 200 µm to 1500 µm; and/or
wherein the height is from 100 µm to 1000 µm.

10. The neural retina sheet according to claim 8 or 9, wherein the neural retina sheet
(1) has been isolated from a cell aggregate containing a neural retina,
(2) contains a region of the center and/or its neighborhood of continuous epithelial tissue in the cell aggregate, and
(3) is from 600 µm to 2500 µm in major axis, from 200 µm to 1500 µm in minor axis, and from 100 µm to 1000 µm in height; and/or
wherein the neural retina sheet
(1) has been isolated from a cell aggregate containing at least first epithelial tissue and second epithelial tissue, wherein
in the cell aggregate, the first epithelial tissue contains a human neural retina, and the second epithelial tissue has the continuity of the slope of a tangent line to a surface different from the continuity of the slope of a tangent line to the surface of the first epithelial tissue, and contains a non-neural retina-related cell,
(2) contains a region on the first epithelial tissue most distant from the second epithelial tissue, and
(3) is from 600 µm to 2500 µm in major axis, from 200 µm to 1500 µm in minor axis, and from 100 µm to 1000 µm in height, wherein
the second epithelial tissue is a tissue selected from the group consisting of eyeball-related tissue, brain and spinal cord tissue and other tissues different from the neural retina of the first epithelial tissue.

11. A pharmaceutical composition comprising the neural retina sheet according to any one of claims 8 to 10.

12. A neural retina sheet according to any one of claims 8 to 10 for use in a method for treating a disease caused by the damage of a neural retina-related cell or a neural retina or the injury of a neural retina, comprising transplanting the neural retina sheet to a subject in need of transplantation.

13. A method for producing the neural retina sheet according to any one of claims 8 to 10, comprising:
selecting a transplant neural retina determined as being applicable as the transplant neural retina by evaluating a cell aggregate containing a neural retina having an epithelial structure derived from a pluripotent stem cell by use of the method according to any one of claims 1 to 7; and
isolating the selected transplant neural retina;
wherein the cell aggregate containing a neural retina is produced by differentiating pluripotent stem cells using a differentiation factor; and
optionally wherein the cell aggregate is a cell aggregate containing at least first epithelial tissue and second epithelial tissue, obtained by differentiating a pluripotent stem cell, wherein the first epithelial tissue contains a human neural retina, and the second epithelial tissue has the continuity of the slope of a tangent line to a surface different from the continuity of the slope of a tangent line to the surface of the first epithelial tissue, and contains a non-neural retina-related cell, and
the isolation of the transplant neural retina is isolation from the cell aggregate such that the transplant neural retina contains a region on the first epithelial tissue most distant from the second epithelial tissue.

14. A method for producing a neural retina sheet, comprising:
sampling a sample for quality evaluation from each of 2 or more and 800 or less cell aggregates containing a neural retina having an epithelial structure derived from a pluripotent stem cell, the sample for quality evaluation being a part of the cell aggregate;
selecting a transplant neural retina determined as being applicable as the transplant neural retina by evaluating the sampled 2 or more and 800 or less samples for quality evaluation by use of the method according to any one of claims 1 to 7; and
isolating the selected transplant neural retina;
wherein the cell aggregate containing a neural retina is produced by differentiating pluripotent stem cells using a differentiation factor; and
optionally wherein the cell aggregate is a cell aggregate containing at least first epithelial tissue and second epithelial tissue, obtained by differentiating a pluripotent stem cell, wherein the first epithelial tissue contains a human neural retina, and the second epithelial tissue has the continuity of the slope of a tangent line to a surface different from the continuity of the slope of a tangent line to the surface of the first epithelial tissue, and contains a non-neural retina-related cell, and
the isolation of the transplant neural retina is isolation from the cell aggregate such that the transplant neural retina contains a region on the first epithelial tissue most distant from the second epithelial tissue.

15. The method according to claim 13 or 14, wherein the cell aggregate containing a neural retina is prepared by a method comprising the following steps (A) to (C):
(A) culturing pluripotent stem cells in a culture medium containing a factor for maintaining undifferentiated state in the absence of feeder cells;
(B) forming a cell aggregate by suspension-culturing the cells obtained in the step (A); and
(C) further suspension-culturing the cell aggregate obtained in the step (B) in a culture medium containing the differentiation factor, wherein the differentiation factor is a BMP signaling pathway agonist.

16. The method according to claim 13 or 14, wherein the cell aggregate containing a neural retina is prepared by a method comprising the following steps (A) to (E):
(A) culturing pluripotent stem cells in a culture medium containing a factor for maintaining undifferentiated state and optionally containing a TGFβ family signaling pathway inhibitor and/or a sonic hedgehog signaling pathway agonist in the absence of feeder cells;
(B) forming a cell aggregate by suspension-culturing the cells obtained in the step (A) in a culture medium optionally containing a Wnt signaling pathway inhibitor and/or a sonic hedgehog signaling pathway agonist;
(C) further suspension-culturing the cell aggregate obtained in the step (B) in a culture medium containing the differentiation factor, wherein the differentiation factor is a BMP signaling pathway agonist;
(D) culturing the cell aggregate obtained in the step (C) in a serum-free medium or a serum medium containing a Wnt signaling pathway agonist and/or a FGF signaling pathway inhibitor for a period on the order of 2 days to 4 days; and
(E) culturing the cell aggregate obtained in the step (D) in a serum-free medium or a serum medium containing neither a Wnt signaling pathway agonist nor a FGF signaling pathway inhibitor and optionally containing a thyroid hormone signaling pathway agonist for about 30 days to about 200 days.

## Patentansprüche

1. Verfahren zur Bewertung der Qualität eines neuronalen Netzhauttransplantats,
wobei das Verfahren umfasst:
Probenahme eines Teils oder des gesamten Zellaggregats, das eine neuronale Netzhaut enthält, die eine aus einer pluripotenten Stammzelle abgeleitete Epithelstruktur aufweist, als Probe zur Qualitätsbewertung;
Erkennen der Expression eines neuronalen netzhautbezogenen zellbezogenen Gens und eines nicht-neuronalen netzhautbezogenen zellbezogenen Gens in der Probe zur Qualitätsbewertung; und
wenn die Expression des neuronalen netzhautbezogenen zellbezogenen Gens gefunden wird und die Expression des nicht-neuronalen netzhautbezogenen zellbezogenen Gens nicht gefunden wird, Bestimmen, dass
(1) die neuronale Netzhaut (neuronales Netzhauttransplantat) in demselben Zellaggregat wie das Zellaggregat, das die Probe zur Qualitätsbewertung enthält, das Teil ist,
(2) die neuronale Netzhaut (neuronales Netzhauttransplantat) in einem Zellaggregat derselben Charge wie das Zellaggregat, das die Probe zur Qualitätsbewertung enthält, das Teil ist, oder
(3) die neuronale Netzhaut (neuronales Netzhauttransplantat) in einem Zellaggregat derselben Charge wie das Zellaggregat der Probe zur Qualitätsbewertung das Ganze ist, als neuronales Netzhauttransplantat anwendbar ist, wobei
das nicht-neuronale netzhautbezogene zellbezogene Gen ein oder mehrere Gene, ausgewählt aus der Gruppe bestehend aus einem Marker-Gen für Gehirn- und Rückenmarkgewebe und einem augapfelbezogenen Gewebemarker-Gen umfasst, wobei
das Marker-Gen für Gehirn- und Rückenmarkgewebe ein oder mehrere Gene, ausgewählt aus der Gruppe bestehend aus einem Telencephalon-Marker-Gen, einem Diencephalon/Midbrain-Marker-Gen und einem Rückenmark-Marker-Gen ist, und
das augapfelbezogene Gewebemarker-Gen ein oder mehrere Gene, ausgewählt aus der Gruppe bestehend aus einem Augenstiel-Marker-Gen, einem Ziliarkörper-Marker-Gen, einem Linsenmarker-Gen und einem Netzhaut-Pigmentepithel-Marker-Gen ist;
wobei
das Telencephalon-Marker-Gen ein oder mehrere Gene, ausgewählt aus der Gruppe bestehend aus FoxG1, Emx2, Dlx2, Dlx1 und Dlx5 umfasst,
das Dienzephalon-/Midbrain-Marker-Gen ein oder mehrere Gene ausgewählt aus der Gruppe bestehend aus DMBX1, Nkx2.1, OTP, FGFR2, EFNA5 und GAD1 umfasst,
das Marker-Gen für das Rückenmark ein oder mehrere Gene, ausgewählt aus der Gruppe bestehend aus HOXD4, HOXD3, HOXD1, HOXC5, HOXA5 und HOXB2 umfasst,
das Augenstiel-Marker-Gen ein oder mehrere Gene, ausgewählt aus der Gruppe bestehend aus GREM1, GPR17, ACVR1C, CDH6, Pax2, Pax8, GAD2 und SEMA5A umfasst,
das Ziliarkörper-Marker-Gen ein oder mehrere Gene, ausgewählt aus der Gruppe bestehend aus Zic1, MAL, HNF1beta, FoxQ1, CLDN2, CLDN1, GPR177, AQP1 und AQP4 umfasst,
das Linsenmarker-Gen ein oder mehrere Gene, ausgewählt aus der Gruppe bestehend aus CRYAA und CRYBA1 umfasst, und
das Netzhautepithel-Marker-Gen ein oder mehrere Gene, ausgewählt aus der Gruppe bestehend aus MITF, TTR und BEST1 umfasst; und,
wobei das neuronale netzhautbezogene zellbezogene Gen eines oder mehrere Gene, ausgewählt aus der Gruppe bestehend aus RAX, Chx10, SIX3, SIX6, RCVRN, CRX, NRL und NESTIN ist.

2. Verfahren nach Anspruch 1, wobei das nicht-neuronale netzhautbezogene zellbezogene Gen weiter ein undifferenziertes pluripotentes Stammzell-Marker-Gen umfasst;
wobei das undifferenzierte pluripotente Stammzell-Marker-Gen wahlweise ein oder mehrere Gene, ausgewählt aus der Gruppe bestehend aus Oct3/4, Nanog und Lin28 umfasst.

3. Verfahren nach Anspruch 1 oder 2, wobei das Zellaggregat derselben Charge wie das Zellaggregat der Probe zur Qualitätsbewertung ein Zellaggregat ist, das in demselben Zellkulturbehälter hergestellt wird wie das der transplantierten neuronalen Netzhaut.

4. Verfahren entsprechend einem der PATENTANSPRÜCHE 1 bis 3, wobei
die Probe zur Qualitätsbewertung Teil des Zellaggregats ist und
wenn die Expression des neuronalen netzhautbezogenen zellbezogenen Gens gefunden wird und die Expression des nicht-neuronalen retinabezogenen zellbezogenen Gens nicht gefunden wird, bestimmt wird, dass eine neuronale Netzhaut mindestens teilweise fortlaufend oder angrenzend an das Teil in demselben Zellaggregat wie das Zellaggregat, das die Probe zur Qualitätsbewertung enthält, das Teil ist, das als das neuronale Netzhauttransplantat anwendbar ist;
wobei das neuronale Netzhauttransplantat wahlweise in demselben Epithelgewebe wie das der Probe zur Qualitätsbewertung enthalten ist;
wobei das neuronale Netzhauttransplantat wahlweise das Zentrum und/oder dessen Nachbarschaft desselben Epithelgewebes enthält;
wobei das neuronale Netzhauttransplantat wahlweise fortlaufendes Epithelgewebe ist.

5. Verfahren nach Anspruch 4, wobei
das Zellaggregat, das eine neuronale Netzhaut enthält, erstes Epithelgewebe enthält, welches das neuronale Netzhauttransplantat enthält, und zweites Epithelgewebe, das die Kontinuität der Neigung einer Berührungslinie zu einer Oberfläche aufweist, die sich von der Kontinuität der Neigung einer Berührungslinie zur Oberfläche des ersten Epithelgewebes unterscheidet, und eine nicht-neuronale netzhautbezogene Zelle enthält,
das neuronale Netzhauttransplantat einen vom zweiten Epithelgewebe am weitesten entfernten Bereich auf dem ersten Epithelgewebe enthält, und
die Probe zur Qualitätsbewertung ein Teil ist, das zwischen dem zweiten Epithelgewebe und dem neuronalen Netzhauttransplantat vorhanden ist.

6. Verfahren nach Anspruch 5, wobei das zweite Epithelgewebe augapfelbezogenes Gewebe und/oder Gehirn- und Rückenmarkgewebe ist;
wobei das augapfelbezogene Gewebe wahlweise eine Netzhautpigmentepithelzelle und einen Ziliarkörper enthält.

7. Verfahren entsprechend einem der PATENTANSPRÜCHE 1 bis 6, umfassend Durchführen des Nachweises der Expression des neuronalen netzhautbezogenen zellbezogenen Gens und des nicht-neuronalen netzhautbezogenen zellbezogenen Gens durch quantitative PCR;
wahlweise umfassend Bestimmen als anwendbar als das neuronale Netzhauttransplantat, wenn die folgende Referenz 1 und Referenz 2 erfüllt sind:
Referenz 1: der Unterschied zwischen dem Schwellenzykluswert (Ct) des neuronalen netzhautbezogenen zellbezogenen Gens und dem Ct-Wert eines internen Standardgens (Ct-Wert) ist 10 oder weniger, und
Referenz 2: der Unterschied zwischen dem Ct-Wert des nicht-neuronalen netzhautbezogenen zellbezogenen Gens und dem Ct-Wert des internen Standardgens (Ct-Wert) ist 5 oder mehr; und/oder
wobei die quantitative PCR wahlweise durch ein Verfahren durchgeführt wird, umfassend die folgenden Schritte (1) bis (4), wodurch gleichzeitig die jeweiligen Expressionsstufen des neuronalen netzhautbezogenen zellbezogenen Gens und des nicht-neuronalen netzhautbezogenen zellbezogenen Gens in zwei oder mehr der Proben zur Qualitätsbewertung nachgewiesen werden:
(1) Bereitstellen einer Strömungskanalplatte, die eine Probenmuldengruppe aufweist, die aus 8 oder mehr und 800 oder weniger unabhängigen Probenmulden besteht, eine oder mehrere Primermuldengruppen, die aus 8 oder mehr und 800 oder weniger unabhängigen Primermulden bestehen, und Strömungskanälen, welche die unabhängigen Probenmulden in der Probenmuldengruppe mit den unabhängigen Primermulden in jeder Primermuldengruppe verbinden, Lösungen, die Nukleinsäuren enthalten, die aus den zwei oder mehr der Proben zur Qualitätsbewertung erhalten werden, und eine Lösung, die einen oder eine Vielzahl von Primern enthält, die spezifisch für jedes einer oder mehrerer der neuronalen netzhautbezogenen zellbezogenen Gene oder der nicht-neuronalen netzhautbezogenen zellbezogenen Gene (Primerlösung) sind;
(2) Hinzufügen der Probenlösungen an einer Probenlösung/einer Probenmulde für jede der Proben zur Qualitätsbewertung zur Probenmuldengruppe;
(3) Hinzufügen der Primerlösung zu einer oder mehreren Primermulden in den einen oder mehreren Primermulden-Gruppen, um unterschiedliche Primermulden-Gruppen zu sein;
(4) getrenntes Mischen der Primer mit den Nukleinsäuren über die Strömungskanäle; und
(5) Durchführen einer quantitativen PCR unter Verwendung des in (4) erhaltenen Gemisches.

8. Neuronale Netzhaut-Folie,
(1) die von einer pluripotenten Stammzelle abgeleitet ist,
(2) die eine dreidimensionale Struktur aufweist,
(3) umfassend eine neuronale Netzhautschicht, die eine Vielzahl von Schichtstrukturen aufweist, die eine Photorezeptorschicht und eine innere Schicht einschließt,
(4) wobei die Photorezeptorschicht eine oder mehrere Zellen, ausgewählt aus der Gruppe bestehend aus einer Photorezeptor-Vorläuferzelle und einer Photorezeptorzelle umfasst,
(5) die innere Schicht eine oder mehrere Zellen aus der Gruppe bestehend aus einer Netzhaut-Vorläuferzelle, einer Ganglienzelle, einer amakrinen Zelle und einer bipolaren Zelle umfasst,
(6) die Oberfläche der neuronalen Netzhautschicht eine apikale Oberfläche aufweist,
(7) die innere Schicht innerhalb der Photorezeptorschicht vorhanden ist, die entlang der apikalen Oberfläche vorhanden ist,
(8) die Fläche der neuronalen Netzhautschicht 50% oder mehr in Bezug auf die Gesamtfläche der Oberfläche der neuronalen Netzhaut-Folie beträgt,
(9) die Fläche einer fortlaufenden Epithelstruktur 80% oder mehr in Bezug auf die Gesamtfläche der apikalen Oberfläche der neuronalen Netzhautschicht beträgt, und
(10) die Expression eines neuronalen netzhautbezogenen zellbezogenen Gens, das gefunden wird, und die Expression eines nicht-neuronalen netzhautbezogenen zellbezogenen Gens, das nicht in der neuronalen Netzhautfolie gefunden wird, wobei das nicht-neuronale netzhautbezogene zellbezogene Gen ein oder mehrere Gene, ausgewählt aus der Gruppe bestehend aus einem Marker-Gen für Gehirn- und Rückenmarkgewebe und einem Marker-Gen für augapfelbezogenes Gewebe umfasst, wobei
das Marker-Gen für Gehirn- und Rückenmarkgewebe ein oder mehrere Gene, ausgewählt aus der Gruppe bestehend aus einem Telencephalon-Marker-Gen, einem Diencephalon/Midbrain-Marker-Gen und einem Rückenmark-Marker-Gen ist, und
das augapfelbezogene Gewebemarker-Gen ein oder mehrere Gene, ausgewählt aus der Gruppe bestehend aus einem Augenstiel-Marker-Gen, einem Ziliarkörper-Marker-Gen, einem Linsenmarker-Gen und einem Netzhaut-Pigmentepithel-Marker-Gen ist;
wobei
das Telencephalon-Marker-Gen ein oder mehrere Gene, ausgewählt aus der Gruppe bestehend aus FoxG1, Emx2, Dlx2, Dlx1 und Dlx5 umfasst,
das Dienzephalon-/Midbrain-Marker-Gen ein oder mehrere Gene ausgewählt aus der Gruppe bestehend aus DMBX1, Nkx2.1, OTP, FGFR2, EFNA5 und GAD1 umfasst,
das Marker-Gen für das Rückenmark ein oder mehrere Gene, ausgewählt aus der Gruppe bestehend aus HOXD4, HOXD3, HOXD1, HOXC5, HOXA5 und HOXB2 umfasst,
das Augenstiel-Marker-Gen ein oder mehrere Gene, ausgewählt aus der Gruppe bestehend aus GREM1, GPR17, ACVR1C, CDH6, Pax2, Pax8, GAD2 und SEMA5A umfasst,
das Ziliarkörper-Marker-Gen ein oder mehrere Gene, ausgewählt aus der Gruppe bestehend aus Zic1, MAL, HNF1beta, FoxQ1, CLDN2, CLDN1, GPR177, AQP1 und AQP4 umfasst,
das Linsenmarker-Gen ein oder mehrere Gene, ausgewählt aus der Gruppe bestehend aus CRYAA und CRYBA1 umfasst, und
das Netzhautepithel-Marker-Gen ein oder mehrere Gene, ausgewählt aus der Gruppe bestehend aus MITF, TTR und BEST1 umfasst, und,
wobei in (10) das neuronale netzhautbezogene zellbezogene Gen eines oder mehrere Gene ausgewählt aus der Gruppe bestehend aus RAX, Chx10, SIX3, SIX6, RCVRN, CRX, NRL und NESTIN ist.

9. Neuronale Netzhautfolie nach Anspruch 8, wobei die Hauptachse von 600 µm bis 2500 µm beträgt; und/oder
wobei die Nebenachse von 200 µm bis 1500 µm beträgt; und/oder
wobei die Höhe von 100 µm bis 1000 µm beträgt.

10. Neuronale Netzhautfolie nach Anspruch 8 oder 9, wobei die neuronale Netzhautfolie
(1) aus einem Zellaggregat isoliert wurde, das eine neuronale Netzhaut enthält,
(2) einen Bereich des Zentrums und/oder seiner Nachbarschaft von kontinuierlichem Epithelgewebe im Zellaggregat enthält, und
(3) von 600 µm bis 2500 µm in der Hauptachse, von 200 µm bis 1500 µm in der Nebenachse und von 100 µm bis 1000 µm in der Höhe beträgt; und/oder
wobei die neuronale Netzhautfolie
(1) aus einem Zellaggregat isoliert wurde, das mindestens erstes Epithelgewebe und zweites Epithelgewebe enthält, wobei
das erste Epithelgewebe im Zellaggregat eine menschliche neuronale Netzhaut enthält,
und das zweite Epithelgewebe die Kontinuität der Neigung einer Berührungslinie zu einer Oberfläche aufweist, die sich von der Kontinuität der Neigung einer Berührungslinie zur Oberfläche des ersten Epithelgewebes unterscheidet, und eine nicht-neuronale netzhautbezogene Zelle enthält,
(2) einen vom zweiten Epithelgewebe am weitesten entfernten Bereich auf dem ersten Epithelgewebe enthält, und
(3) von 600 µm bis 2500 µm in der Hauptachse, von 200 µm bis 1500 µm in der Nebenachse und von 100 µm bis 1000 µm in der Höhe, wobei
das zweite Epithelgewebe ein Gewebe, ausgewählt aus der Gruppe bestehend aus augapfelbezogenem Gewebe, Gehirn- und Rückenmarkgewebe und anderen Geweben ist, die sich von der neuronalen Netzhaut des ersten Epithelgewebes unterscheiden.

11. Pharmazeutische Zusammensetzung, umfassend die neuronale Netzhautfolie entsprechend einem der PATENTANSPRÜCHE 8 bis 10.

12. Neuronale Netzhautfolie nach einem der PATENTANSPRÜCHE 8 bis 10 zur Verwendung in einem Verfahren zur Behandlung einer Erkrankung, die durch die Schädigung einer neuronalen netzhautbezogenen Zelle oder einer neuronalen Netzhaut oder die Schädigung einer neuronalen Netzhaut veranlasst wird, umfassend die Transplantation der neuronalen Netzhautfolie an ein Subjekt, das eine Transplantation benötigt.

13. Erzeugungsverfahren der neuronalen Netzhautfolie entsprechend einem der PATENTANSPRÜCHE 8 bis 10, umfassend:
Auswählen eines neuronalen Netzhauttransplantats, das als anwendbar als neuronales Netzhauttransplantat bestimmt worden ist, durch Bewerten eines Zellaggregats, das eine neuronale Netzhaut enthält, die eine Epithelstruktur aufweist, die von einer pluripotenten Stammzelle abgeleitet ist, unter Verwendung des Verfahrens entsprechend einem der PATENTANSPRÜCHE 1 bis 7; und
Isolieren des ausgewählten neuronalen Netzhauttransplantats;
wobei das Zellaggregat, das eine neuronale Netzhaut enthält, durch Differenzieren pluripotenter Stammzellen unter Verwendung eines Differenzierungsfaktors erzeugt wird; und
wobei das Zellaggregat wahlweise ein Zellaggregat ist, das mindestens erstes Epithelgewebe und zweites Epithelgewebe enthält, das durch Differenzieren einer pluripotenten Stammzelle erhalten wird, wobei das erste Epithelgewebe eine menschliche neuronale Netzhaut enthält, und das zweite Epithelgewebe die Kontinuität der Neigung einer Berührungslinie zu einer Oberfläche aufweist, die sich von der Kontinuität der Neigung einer Berührungslinie zur Oberfläche des ersten Epithelgewebes unterscheidet, und eine nicht-neuronale netzhautbezogene Zelle enthält, und
das Isolieren des neuronalen Netzhauttransplantats Isolieren vom Zellaggregat ist, sodass das neuronale Netzhauttransplantat einen Bereich auf dem ersten Epithelgewebe enthält, der am weitesten vom zweiten Epithelgewebe entfernt ist.

14. Erzeugungsverfahren einer neuronalen Netzhautfolie, umfassend:
Probenahme zur Qualitätsbewertung aus jedem von 2 oder mehr und 800 oder weniger Zellaggregaten, die eine neuronale Netzhaut enthalten, die eine Epithelstruktur aufweist, die von einer pluripotenten Stammzelle abgeleitet ist, wobei die Probe zur Qualitätsbewertung Teil des Zellaggregats ist;
Auswählen eines neuronalen Netzhauttransplantats, das als neuronales Netzhauttransplantat anwendbar bestimmt wird, durch Bewerten der entnommenen 2 oder mehr und 800 oder weniger Proben zur Qualitätsbewertung unter Verwendung des Verfahrens entsprechend einem der PATENTANSPRÜCHE 1 bis 7; und
Isolieren des ausgewählten neuronalen Netzhauttransplantats;
wobei das Zellaggregat, das eine neuronale Netzhaut enthält, durch Differenzieren pluripotenter Stammzellen unter Verwendung eines Differenzierungsfaktors erzeugt wird; und
wobei das Zellaggregat wahlweise ein Zellaggregat ist, das mindestens das erste Epithelgewebe und das zweite Epithelgewebe enthält, das durch Differenzieren einer pluripotenten Stammzelle erhalten wird, wobei das erste Epithelgewebe eine menschliche neuronale Netzhaut enthält, und das zweite Epithelgewebe die Kontinuität der Neigung einer Berührungslinie zu einer Oberfläche aufweist, die sich von der Kontinuität der Neigung einer Berührungslinie zur Oberfläche des ersten Epithelgewebes unterscheidet, und eine nicht-neuronale netzhautbezogene Zelle enthält, und
das Isolieren des neuronalen Netzhauttransplantats Isolieren vom Zellaggregat ist, sodass das neuronale Netzhauttransplantat einen Bereich auf dem ersten Epithelgewebe enthält, der am weitesten vom zweiten Epithelgewebe entfernt ist.

15. Verfahren nach Anspruch 13 oder 14, wobei das Zellaggregat, das eine neuronale Netzhaut enthält, durch ein Verfahren vorbereitet wird, das die folgenden Schritte (A) bis (C) umfasst:
(A) Kultivieren pluripotenter Stammzellen in einem Kulturmedium, enthaltend einen Faktor zum Beibehalten des undifferenzierten Zustands in Abwesenheit von Speisezellen;
(B) Bilden eines Zellaggregats durch Suspensionskultur der in Schritt (A) erhaltenen Zellen; und
(C) weiteres Suspensionskultivieren des im Schritt (B) erhaltenen Zellaggregats in einem Kulturmedium, das den Differenzierungsfaktor enthält, wobei der Differenzierungsfaktor ein BMP-Signalweg-Agonist ist.

16. Verfahren nach Anspruch 13 oder 14, wobei das Zellaggregat, das eine neuronale Netzhaut enthält, durch ein Verfahren vorbereitet wird, das die folgenden Schritte (A) bis (E) umfasst:
(A) Kultivieren pluripotenter Stammzellen in einem Kulturmedium, enthaltend einen Faktor zum
Beibehalten eines undifferenzierten Zustands und wahlweise Enthalten eines Signalweginhibitors der TGFß-Familie und/oder einen Hedgehoge-Signalweg-Agonisten bei Abwesenheit von Speisezellen;
(B) Bilden eines Zellaggregats durch Suspensionskultivierung der im Schritt (A) erhaltenen Zellen in einem Kulturmedium, das wahlweise einen Wnt-Signalweginhibitor enthält
und/oder eines Hedgehoge-Signalweg-Agonisten;
(C) weiteres Suspensionskultivieren des in Schritt (B) erhaltenen Zellaggregats in einem Kulturmedium, das den Differenzierungsfaktor enthält, wobei der Differenzierungsfaktor
ein BMP-Signalweg-Agonist ist;
(D) Kultivieren des in Schritt (C) erhaltenen Zellaggregats in einem serumfreien Medium oder einem Serummedium, das einen Wnt-Signalweg-Agonisten und/oder einen FGF-Signalweginhibitor für einen Zeitraum in der Größenordnung von 2 bis 4 Tagen enthält; und
(E) Kultivieren des im Schritt (D) erhaltenen Zellaggregats in einem serumfreien Medium oder einem Serummedium, das weder einen Wnt-Signalweg-Agonisten noch einen FGF-Signalweginhibitor enthält und wahlweise für etwa 30 Tage bis etwa 200 Tage einen Schilddrüsenhormon-Signalweg-Agonisten enthält.

## Revendications

1. Procédé d'évaluation de la qualité d'une rétine neurale de transplantation,
le procédé comprenant :
l'échantillonnage d'une partie ou de la totalité d'un agrégat cellulaire contenant une rétine neurale présentant une structure épithéliale dérivée d'une cellule souche pluripotente en tant qu'échantillon pour une évaluation de la qualité ;
la détection de l'expression d'un gène lié à une cellule liée à la rétine neurale et d'un gène lié à une cellule liée à la rétine non neurale dans l'échantillon pour une évaluation de la qualité ; et
lorsque l'expression du gène lié à une cellule liée à la rétine neurale est trouvée et l'expression du gène lié à une cellule liée à la rétine non neurale n'est pas trouvée, déterminant que
(1) la rétine neurale (rétine neurale de transplantation) dans le même agrégat cellulaire que l'agrégat cellulaire contenant l'échantillon pour une évaluation de la qualité étant la partie,
(2) la rétine neurale (rétine neurale de transplantation) dans un agrégat cellulaire du même lot que l'agrégat cellulaire contenant l'échantillon pour une évaluation de la qualité étant la partie, ou
(3) la rétine neurale (rétine neurale de transplantation) dans un agrégat cellulaire du même lot que l'agrégat cellulaire de l'échantillon pour une évaluation de la qualité est la totalité, étant applicable en tant que la rétine neurale de transplantation, dans lequel
le gène lié à une cellule liée à la rétine non neurale comprend un ou plusieurs gènes sélectionnés dans le groupe consistant en un gène marqueur de tissu cérébral et médullaire et un gène marqueur de tissu lié au globe oculaire, dans lequel
le gène marqueur de tissu cérébral et médullaire est un ou plusieurs gènes sélectionnés dans le groupe consistant en un gène marqueur de télencéphale, un gène marqueur de diencéphale/mésencéphale et un gène marqueur de moelle épinière, et
le gène marqueur de tissu lié au globe oculaire est un ou plusieurs gènes sélectionnés dans le groupe consistant en un gène marqueur de pédoncule optique, un gène marqueur de corps ciliaire, un gène marqueur de cristallin et un gène marqueur d'épithélium pigmentaire rétinien ;
dans lequel
le gène marqueur de télencephale comprend un ou plusieurs gènes sélectionnés dans le groupe consistant en FoxG1, Emx2, Dlx2, Dlx1 et Dlx5,
le gène marqueur de diencéphale/mésencéphale comprend un ou plusieurs gènes sélectionnés dans le groupe consistant en DMBX1, Nkx2.1, OTP, FGFR2, EFNA5 et GAD1,
le gène marqueur de moelle épinière comprend un ou plusieurs gènes sélectionnés dans le groupe consistant en HOXD4, HOXD3, HOXD1, HOXC5, HOXA5 et HOXB2,
le gène marqueur de pédoncule optique comprend un ou plusieurs gènes sélectionnés dans le groupe consistant en GREM1, GPR17, ACVR1C, CDH6, Pax2, Pax8, GAD2 et SEMA5A,
le gène marqueur de corps ciliaire comprend un ou plusieurs gènes sélectionnés dans le groupe consistant en Zic1, MAL, HNF1beta, FoxQ1, CLDN2, CLDN1, GPR177, AQP1 et AQP4,
le gène marqueur de cristallin comprend un ou plusieurs gènes sélectionnés dans le groupe consistant en CRYAA et CRYBA1, et
le gène marqueur d'épithélium pigmentaire rétinien comprend un ou plusieurs gènes sélectionnés dans le groupe consistant en MITF, TTR et BEST1 ; et,
dans lequel le gène lié à une cellule liée à la rétine neurale est un ou plusieurs gènes sélectionnés dans le groupe consistant en RAX, Chx10, SIX3, SIX6, RCVRN, CRX, NRL et NESTIN.

2. Procédé selon la revendication 1, dans lequel le gène lié à une cellule liée à la rétine non neurale comprend en outre un gène marqueur de cellules souches pluripotentes indifférenciées ;
facultativement dans lequel le gène marqueur de cellules souches pluripotentes indifférenciées comprend un ou plusieurs gènes sélectionnés dans le groupe consistant en Oct3/4, Nanog et Lin28.

3. Procédé selon la revendication 1 ou la revendication 2, dans lequel l'agrégat cellulaire du même lot que l'agrégat cellulaire de l'échantillon pour une évaluation de la qualité est un agrégat cellulaire produit dans le même récipient de culture cellulaire que celui de la rétine neurale de transplantation.

4. Procédé selon l'une quelconque des revendications 1 à 3, dans lequel
l'échantillon pour une évaluation de la qualité fait partie de l'agrégat cellulaire, et
lorsque l'expression du gène lié à une cellule liée à la rétine neurale est trouvée et l'expression du gène lié à une cellule liée à la rétine non neurale n'est pas trouvée, il est déterminé qu'une rétine neurale continue ou adjacente au moins partiellement à la partie dans le même agrégat cellulaire que l'agrégat cellulaire contenant l'échantillon pour une évaluation de la qualité étant la partie est applicable en tant que rétine neurale de transplantation ;
facultativement dans lequel la rétine neurale de transplantation est contenue dans le même tissu épithélial que celui de l'échantillon pour une évaluation de la qualité ;
facultativement dans lequel la rétine neurale de transplantation contient le centre et/ou son voisinage du même tissu épithélial ;
facultativement dans lequel la rétine neurale de transplantation est un tissu épithélial continu.

5. Procédé selon la revendication 4, dans lequel
l'agrégat cellulaire contenant une rétine neurale contient un premier tissu épithélial contenant la rétine neurale de transplantation, et un deuxième tissu épithélial présentant la continuité de la pente d'une ligne tangente à une surface différente de la continuité de la pente d'une ligne tangente à la surface du premier tissu épithélial, et contenant une cellule liée à la rétine non neurale,
la rétine neurale de transplantation contient une région sur le premier tissu épithélial la plus éloignée du deuxième tissu épithélial, et
l'échantillon pour une évaluation de la qualité est une partie présente entre le deuxième tissu épithélial et la rétine neurale de transplantation.

6. Procédé selon la revendication 5, dans lequel le deuxième tissu épithélial est un tissu lié au globe oculaire et/ou un tissu cérébral et médullaire ;
facultativement dans lequel le tissu lié au globe oculaire contient une cellule épithéliale pigmentaire rétinienne et un corps ciliaire.

7. Procédé selon l'une quelconque des revendications 1 à 6, comprenant la réalisation de la détection de l'expression du gène lié aux cellules liées à la rétine neurale et du gène lié aux cellules liées à la rétine non neurale par PCR quantitative ;
comprenant facultativement la détermination comme étant applicable en tant que rétine neurale de transplantation lorsque les références 1 et 2 suivantes sont satisfaites :
référence 1 : la différence entre la valeur de cycle seuil (Ct) du gène lié à une cellule liée à la rétine neurale et la valeur Ct d'un gène standard interne (valeur ΔCt) est de 10 ou moins, et
référence 2 : la différence entre la valeur Ct du gène lié à une cellule liée à la rétine non neurale et la valeur Ct du gène standard interne (valeur ΔCt) est de 5 ou plus ; et/ou
facultativement dans lequel la PCR quantitative est réalisée par un procédé comprenant
les étapes (1) à (4) suivantes, détectant ainsi simultanément les niveaux d'expression respectifs du gène lié à une cellule liée à la rétine neurale et du gène lié à une cellule non neurale liée à la rétine dans deux des échantillons ou plus pour une évaluation de la qualité :
(1) la fourniture d'une plaque de canal d'écoulement présentant un groupe de puits d'échantillonnage constitué de 8 puits d'échantillonnage indépendants ou plus et de 800 puits d'échantillonnage indépendants ou moins, d'un ou plusieurs groupes de puits d'amorçage constitués de 8 puits d'amorçage indépendants ou plus et de 800 puits d'amorçage indépendants ou moins, et de canaux d'écoulement reliant les puits d'échantillonnage indépendants dans le groupe de puits d'échantillonnage avec les puits d'amorçage indépendants dans chaque groupe de puits d'amorçage, de solutions contenant des acides nucléiques obtenues à partir des deux ou plus des échantillons pour une évaluation de la qualité (solutions d'échantillonnage), et d'une solution contenant un ou plusieurs primaires spécifiques à chacun d'un ou plusieurs des gènes liés à des cellules liées à la rétine neurale ou des gènes liés à des cellules liées à la rétine non neurale (solution d'amorce) ;
(2) l'ajout des solutions d'échantillonnage à une solution d'échantillonnage/un puits d'échantillonnage pour chacun des échantillons pour une évaluation de la qualité au groupe de puits d'échantillonnage ;
(3) l'ajout de la solution d'amorce à un ou plusieurs puits d'amorçage dans les un ou plusieurs groupes de puits d'amorçage de manière à être différents groupes de puits d'amorçage ;
(4) le mélange séparé des amorces avec les acides nucléiques via les canaux d'écoulement ; et
(5) la réalisation d'une PCR quantitative en utilisant le mélange obtenu en (4).

8. Feuille de rétine neurale,
(1) étant dérivée d'une cellule souche pluripotente,
(2) présentant une structure tridimensionnelle,
(3) comprenant une couche rétinienne neurale présentant une pluralité de structures de couche incluant une couche photoréceptrice et une couche interne,
(4) la couche photoréceptrice comprenant une ou plusieurs cellules sélectionnées dans le groupe consistant en une cellule précurseur photoréceptrice et une cellule photoréceptrice,
(5) la couche interne comprenant une ou plusieurs cellules sélectionnées dans le groupe consistant en une cellule précurseur rétinienne, une cellule ganglionnaire, une cellule amacrine et une cellule bipolaire,
(6) la surface de la couche rétinienne neurale présentant une surface apicale,
(7) la couche interne étant présente à l'intérieur de la couche photoréceptrice présente le long de la surface apicale,
(8) la surface de la couche rétinienne neurale étant de 50 % ou plus par rapport à la surface totale de la feuille de rétine neurale,
(9) la surface d'une structure épithéliale continue étant de 80 % ou plus par rapport à la surface totale de la surface apicale de la couche rétinienne neurale, et
(10) l'expression d'un gène lié à une cellule liée à la rétine neurale étant trouvée et l'expression d'un gène lié à une cellule liée à la rétine non neurale n'étant pas trouvée dans la feuille de rétine neurale, dans laquelle le gène lié à une cellule liée à la rétine non neurale comprenant un ou plusieurs gènes sélectionnés dans le groupe consistant en un gène marqueur de tissu cérébral et médullaire et un gène marqueur de tissu lié au globe oculaire, dans laquelle
le gène marqueur de tissu cérébral et médullaire est un ou plusieurs gènes sélectionnés dans le groupe consistant en un gène marqueur de télencéphale, un gène marqueur de diencéphale/mésencéphale et un gène marqueur de moelle épinière, et
le gène marqueur de tissu lié au globe oculaire est un ou plusieurs gènes sélectionnés dans le groupe consistant en un gène marqueur de pédoncule optique, un gène marqueur de corps ciliaire, un gène marqueur de cristallin et un gène marqueur d'épithélium pigmentaire rétinien ;
dans laquelle
le gène marqueur de télencephale comprend un ou plusieurs gènes sélectionnés dans le groupe consistant en FoxG1, Emx2, Dlx2, Dlx1 et Dlx5,
le gène marqueur de diencéphale/mésencéphale comprend un ou plusieurs gènes sélectionnés dans le groupe consistant en DMBX1, Nkx2.1, OTP, FGFR2, EFNA5 et GAD1,
le gène marqueur de moelle épinière comprend un ou plusieurs gènes sélectionnés dans le groupe consistant en HOXD4, HOXD3, HOXD1, HOXC5, HOXA5 et HOXB2,
le gène marqueur de pédoncule optique comprend un ou plusieurs gènes sélectionnés dans le groupe consistant en GREM1, GPR17, ACVR1C, CDH6, Pax2, Pax8, GAD2 et SEMA5A,
le gène marqueur de corps ciliaire comprend un ou plusieurs gènes sélectionnés dans le groupe consistant en Zic1, MAL, HNF1beta, FoxQ1, CLDN2, CLDN1, GPR177, AQP1 et AQP4,
le gène marqueur de cristallin comprend un ou plusieurs gènes sélectionnés dans le groupe consistant en CRYAA et CRYBA1, et
le gène marqueur d'épithélium pigmentaire rétinien comprend un ou plusieurs gènes sélectionnés dans le groupe consistant en MITF, TTR et BEST1, et,
dans laquelle en (10) le gène lié à une cellule liée à la rétine neurale est un ou plusieurs gènes sélectionnés dans le groupe consistant en RAX, Chx10, SIX3, SIX6, RCVRN, CRX, NRL et NESTIN.

9. Feuille de rétine neurale selon la revendication 8, dans laquelle l'axe principal est de 600 µm à 2500 µm ; et/ou
dans laquelle l'axe secondaire est de 200 µm à 1500 µm ; et/ou
dans laquelle la hauteur est de 100 µm à 1000 µm.

10. Feuille de rétine neurale selon la revendication 8 ou la revendication 9, dans laquelle la feuille de rétine neurale
(1) a été isolée d'un agrégat cellulaire contenant une rétine neurale,
(2) contient une région du centre et/ou son voisinage de tissu épithélial continu dans l'agrégat cellulaire, et
(3) est de 600 µm à 2500 µm dans l'axe principal, de 200 µm à 1500 µm dans l'axe secondaire, et de 100 µm à 1000 µm en hauteur ; et/ou
dans laquelle la feuille de rétine neurale
(1) a été isolée d'un agrégat cellulaire contenant au moins un premier tissu épithélial et un deuxième tissu épithélial, dans laquelle
dans l'agrégat cellulaire, le premier tissu épithélial contient une rétine neurale humaine, et le deuxième tissu épithélial présente la continuité de la pente d'une ligne tangente à une surface différente de la continuité de la pente d'une ligne tangente à la surface du premier tissu épithélial, et contient une cellule liée à la rétine non neurale,
(2) contient une région sur le premier tissu épithélial la plus éloignée du deuxième tissu épithélial, et
(3) est de 600 µm à 2500 µm dans l'axe principal, de 200 µm à 1500 µm dans l'axe secondaire, et de 100 µm à 1000 µm en hauteur, dans laquelle
le deuxième tissu épithélial est un tissu sélectionné dans le groupe consistant en un tissu lié au globe oculaire, un tissu cérébral et médullaire et d'autres tissus différents de la rétine neurale du premier tissu épithélial.

11. Composition pharmaceutique comprenant la feuille de rétine neurale selon l'une quelconque des revendications 8 à 10.

12. Feuille de rétine neurale selon l'une quelconque des revendications 8 à 10 pour être utilisée dans un procédé de traitement d'une maladie causée par un endommagement d'une cellule liée à la rétine neurale ou d'une rétine neurale ou la lésion d'une rétine neurale, comprenant la greffe de la feuille de rétine neurale à un sujet ayant besoin d'une greffe.

13. Procédé de production de la feuille de rétine neurale selon l'une quelconque des revendications 8 à 10, comprenant :
la sélection d'une rétine neurale de transplantation déterminée comme étant applicable en tant que rétine neurale de transplantation en évaluant un agrégat cellulaire contenant une rétine neurale présentant une structure épithéliale dérivée d'une cellule souche pluripotente en utilisant le procédé selon l'une quelconque des revendications 1 à 7 ; et
l'isolement de la rétine neurale de transplantation sélectionnée ;
dans lequel l'agrégat cellulaire contenant une rétine neurale est produit par différenciation de cellules souches pluripotentes en utilisant un facteur de différenciation ; et
facultativement dans lequel l'agrégat cellulaire est un agrégat cellulaire contenant au moins un premier tissu épithélial et un deuxième tissu épithélial, obtenu par différenciation d'une cellule souche pluripotente, dans lequel le premier tissu épithélial contient une rétine neurale humaine, et le deuxième tissu épithélial présente la continuité de la pente d'une ligne tangente à une surface différente de la continuité de la pente d'une ligne tangente à la surface du premier tissu épithélial, et contient une cellule liée à la rétine non neurale, et
l'isolement de la rétine neurale de transplantation est l'isolement de l'agrégat cellulaire de sorte que la rétine neurale de transplantation contient une région sur le premier tissu épithélial la plus éloignée du deuxième tissu épithélial.

14. Procédé de production d'une feuille de rétine neurale, comprenant :
l'échantillonnage d'un échantillon pour une évaluation de la qualité à partir de chacun de 2 agrégats cellulaires ou plus et de 800 agrégats cellulaires ou moins contenant une rétine neurale présentant une structure épithéliale dérivée d'une cellule souche pluripotente, l'échantillon pour une évaluation de la qualité étant une partie de l'agrégat cellulaire ;
la sélection d'une rétine neurale de transplantation déterminée comme étant applicable en tant que rétine neurale de transplantation en évaluant les 2 échantillons prélevés pour une évaluation de la qualité ou plus et 800 échantillons prélevés pour une évaluation de la qualité ou moins en utilisant le procédé selon l'une quelconque des revendications 1 à 7 ; et
l'isolement de la rétine neurale de transplantation sélectionnée ;
dans lequel l'agrégat cellulaire contenant une rétine neurale est produit par différenciation de cellules souches pluripotentes en utilisant un facteur de différenciation ; et
facultativement dans lequel l'agrégat cellulaire est un agrégat cellulaire contenant au moins un premier tissu épithélial et un deuxième tissu épithélial, obtenu par différenciation d'une cellule souche pluripotente, dans lequel le premier tissu épithélial contient une rétine neurale humaine, et le deuxième tissu épithélial présente la continuité de la pente d'une ligne tangente à une surface différente de la continuité de la pente d'une ligne tangente à la surface du premier tissu épithélial, et contient une cellule liée à la rétine non neurale, et
l'isolement de la rétine neurale de transplantation est l'isolement de l'agrégat cellulaire de sorte que la rétine neurale de transplantation contient une région sur le premier tissu épithélial la plus éloignée du deuxième tissu épithélial.

15. Procédé selon la revendication 13 ou la revendication 14, dans lequel l'agrégat cellulaire contenant une rétine neurale est préparé par un procédé comprenant les étapes (A) à (C) suivantes :
(A) la culture de cellules souches pluripotentes dans un milieu de culture contenant un facteur pour
le maintien de l'état indifférencié en l'absence de cellules nourricières ;
(B) la formation d'un agrégat cellulaire par culture en suspension des cellules obtenues à l'étape (A) ; et
(C) une culture en suspension supplémentaire de l'agrégat cellulaire obtenu à l'étape (B) dans un milieu de culture contenant le facteur de différenciation, dans lequel le facteur de différenciation est un agoniste de la voie de signalisation BMP.

16. Procédé selon la revendication 13 ou la revendication 14, dans lequel l'agrégat cellulaire contenant une rétine neurale est préparé par un procédé comprenant les étapes (A) à (E) suivantes :
(A) la culture de cellules souches pluripotentes dans un milieu de culture contenant un facteur pour
le maintien à l'état indifférencié et contenant facultativement un inhibiteur de la voie de signalisation de la famille TGFβ et/ou un agoniste de la voie de signalisation de Sonic Hedgehog en l'absence de cellules nourricières ;
(B) la formation d'un agrégat cellulaire par culture en suspension des cellules obtenues à l'étape (A) dans un milieu de culture contenant facultativement un inhibiteur de voie de signalisation Wnt
et/ou un agoniste de voie de signalisation de Sonic Hedgehog ;
(C) une culture en suspension supplémentaire de l'agrégat cellulaire obtenu à l'étape (B) dans un milieu de culture contenant le facteur de différenciation, dans lequel le facteur de différenciation est un agoniste de la voie de signalisation BMP ;
(D) la culture de l'agrégat cellulaire obtenu à l'étape (C) dans un milieu sans sérum ou un milieu sérique contenant un agoniste de la voie de signalisation Wnt et/ou un inhibiteur de la voie de signalisation FGF pendant une période de l'ordre de 2 jours à 4 jours ; et
(E) la culture de l'agrégat cellulaire obtenu à l'étape (D) dans un milieu sans sérum ou un milieu sérique ne contenant ni un agoniste de la voie de signalisation Wnt ni un inhibiteur de la voie de signalisation FGF et contenant facultativement un agoniste de la voie de signalisation de l'hormone thyroïdienne pendant environ 30 jours à environ 200 jours.
